# EUROPEAN PATENT APPLICATION

(11) **EP 4 624 486 A1**
(43) Date of publication of application: **01.10.2025**
(21) Application number: 24744266.8
(22) Date of filing: 16.01.2024
(51) Int. Cl.: C07K 14/00, C07K 1/107, A61K 38/26, A61P 3/04, A61P 3/10

(54) **POLYPEPTIDE HAVING MODIFIED STRUCTURE AND USE THEREOF**

(30) Priority: 17.01.2023 CN 202310077454; 20.03.2023 CN 202310280348; 28.04.2023 CN 202310484812; 13.05.2023 CN 202310542293; 13.07.2023 CN 202310865180; 20.07.2023 CN 202310901959; 16.08.2023 CN 202311035566; 24.08.2023 CN 202311076512; 04.01.2024 CN 202410018856
(71) Applicant: Guangdong Raynovent Biotech Co., Ltd., Huangpu District Guangzhou 510700 (CN)
(72) Inventor: CHEN, Xiaoxin, Guangzhou, Guangdong 510700 (CN); HUANG, Jianzhou, Guangzhou, Guangdong 510700 (CN); LIU, Ming, Guangzhou, Guangdong 510700 (CN); LIU, Weishun, Guangzhou, Guangdong 510700 (CN); HUANG, Shihai, Guangzhou, Guangdong 510700 (CN); WEN, Kang, Guangzhou, Guangdong 510700 (CN); LI, Zhenmei, Guangzhou, Guangdong 510700 (CN); CHEN, Minglu, Guangzhou, Guangdong 510700 (CN); ZHANG, Qianru, Guangzhou, Guangdong 510700 (CN); LIU, Miao, Guangzhou, Guangdong 510700 (CN); LIU, Zhuowei, Guangzhou, Guangdong 510700 (CN); LONG, Chaofeng, Guangzhou, Guangdong 510700 (CN)
(74) Representative: Clark, Claudia
(86) International application number: PCT/CN2024/072611
(87) International publication number: WO 2024/153091

(57) **Abstract**

The present invention provides a series of polypeptides containing a modified structure. The series of polypeptides act on three targets, i.e., GLP-1, GIP and GCG, and has prospects of being developed into drugs for the prevention or treatment of metabolic diseases, wherein the sequence of the polypeptides is as shown in formula Z-3.

X₅X₀X₆GT FTSDY SIX₁X₇X₈ KX₉X₁₀X₁₁X₀ X₄FX₁₂X₁₃X₁₄ LLX₁₅GG PSSGA PPPS₀ Z-3

## Description

### TECHNICAL FIELD

The present disclosure relates to a series of polypeptides having a modified structure and use thereof. Specifically, the present disclosure relates to a series of polypeptides with a staple structure, a pharmaceutical composition containing the series of polypeptides and use thereof in the treatment of metabolic diseases.

### BACKGROUND

Glucagon-like peptide-1 (GLP-1) and glucose-dependent insulinotropic polypeptide (GIP) are incretins, and glucagon (GCG) is secreted from pancreatic islet α-cells. The aforementioned substances can directly or indirectly affect glucose metabolism and/or lipid metabolism in human body. Therefore, compounds that act on GLP-1, GIP and GCG may possess the prospect of being developed into drugs for the treatment of metabolic diseases. For example, the compounds (polypeptides) that act on GLP-1, such as Exenatide, Liraglutide, and Semaglutide, which have been marketed, are drugs for treating diabetes mellitus type 2 (T2DM) and/or obesity.

However, the compounds that act on a single one of GLP-1, GIP or GCG generally have deficiencies in terms of treatment field, therapeutic effects, and metabolic behavior, thereby affecting the druggability of the compounds and their clinical application after marketing. Therefore, efforts have been made to develop compounds that act on multiple targets in order to intervene through multiple mechanisms at the same time and achieve better clinical effects. Tirzepatide, a GLP-1/GIP dual agonist developed by Eli Lilly, has shown superior effects to Semaglutide in head-to-head clinical trials and has been approved for marketing at present. Further for example, LY3437943, a GLP-1/GIP/GCG triple agonist developed by Eli Lilly, has also shown good clinical effects in clinical studies and good development prospect.

GLP-1R/GIPR/GCGR multiple agonists can activate the molecular mechanisms of blood glucose regulating receptors, thus affecting food intake and satiety through different mechanisms and playing a role in the maintenance of body weight homeostasis. Further, it was also found in the studies that GLP-1 receptor agonists can promote the effect of incretins by agonizing the GLP-1 receptor to produce good hypoglycemic and weight loss effects, and can also treat non-alcoholic steatohepatitis (NASH) through multi-pathway synergistic effects, suggesting that the compounds that act on the target or multiple targets have the prospect of being developed into drugs for the prevention and/or treatment of a series of specific diseases associated with abnormal glucose metabolism and/or lipid metabolism.

### SUMMARY OF THE DISCLOSURE

In view of the deficiencies of the prior art, the present disclosure provides a series of polypeptides having a modified structure and a pharmaceutically acceptable salt thereof. The series of polypeptides have shown good effects on GLP-1 receptor (GLP-1R), GIP receptor (GIPR) and GCG receptor (GCGR) in *in-vitro* trials and thus have a good prospect for drug development.

The present disclosure provides a polypeptide having a modified structure with a polypeptide sequence as shown in Formula Z, or a pharmaceutically acceptable salt thereof:

X₅X₀X₆GT XₐTSDY SX_{b}X₁X₇X₈ KX₉X₁₀X₁₁X₀ X₄FX₁₂X₁₃X₁₄ X_{c}X_{d}X₁₅GG PSSGA PPPS₀ Formula Z

wherein:
X₀ is independently selected from 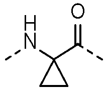 or Aib or alanine (Ala, A), with the structure of Aib being 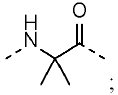
X₁ is independently selected from α-methyl-substituted leucine (α-MeLeu, α-MeL), tyrosine (Tyr, Y) or Aib, with the structure of α-MeL being 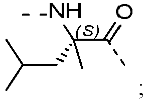
X₄ is selected from α-methyl-substituted lysine (α-MeLys, α-MeK), D-lysine (d-K), L-omithine (L-Orn), alanine (Ala, A), lysine (Lys, K), glutamic acid (Glu, E) or leucine (Leu, L); with the structure of α-methyl-substituted lysine being 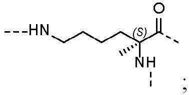 the structure of D-lysine (d-K) being 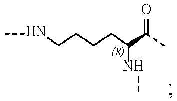 and the structure of L-ornithine (L-Orn) being 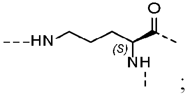
X₅ is selected from tyrosine (Tyr, Y) or histidine (His, H);
X₆ is selected from glutamine (Gln, Q) or histidine (His, H);
X₇ is selected from leucine (Leu, L) or lysine (Lys, K);
X₈ is selected from aspartic acid (Asp, D) or glutamic acid (Glu, E);
X₉ is selected from lysine (Lys, K), glutamine (Gln, Q) or glutamic acid (Glu, E);
X₁₀ is selected from alanine (Ala, A) or tyrosine (Tyr, Y);
X₁₁ is selected from lysine (Lys, K), glutamine (Gln, Q) or alanine (Ala, A);
X₁₂ is selected from valine (Val, V) or isoleucine (Ile, I);
X₁₃ is selected from lysine (Lys, K), glutamine (Gln, Q) or glutamic acid (Glu, E);
X₁₄ is selected from lysine (Lys, K), tryptophan (Trp, W), tyrosine (Tyr, Y), glutamic acid (Glu, E) or phenylalanine (Phe, F);
X₁₃ is selected from lysine (Lys, K) or glutamic acid (Glu, E);
Xₐ is selected from phenylalanine (Phe, F) or α-methyl-substituted phenylalanine (α-MePhe, α-MeF), with the structure of α-methyl-substituted phenylalanine being 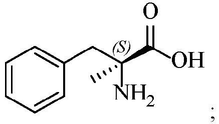
X_{b} is selected from lysine (Lys, K) or isoleucine (Ile, I);
X_{c} is selected from leucine (Leu, L) or phenylalanine (Phe, F);
X_{d} is selected from leucine (Leu, L) or isoleucine (Ile, I);
S₀ is selected from 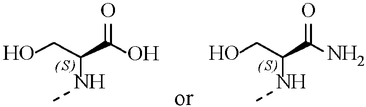 (i.e., the C-terminal amino acid is optionally amidated to a C-terminal primary amide);
and the compound (polypeptide) has the following modifications:
   1) side chains of amino acids at positions i and i+j in the sequence can be connected by a modification to form a modified structure similar to a "staple" structure (peptide-staple) (stapled body), where i is independently selected from 12, 14, 16, 17, 19, 21, 24 or 25, and j is independently selected from 2, 3, 4, 5 or 7,
   2) the modification is formed by the condensation connection of amino or carboxyl groups on the side chains of two amino acid unit structures to 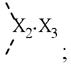
X₂ is selected from 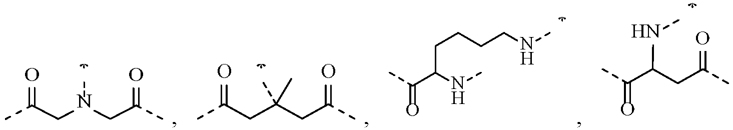 or 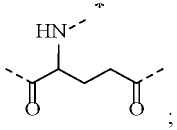 where, "*" indicates a position connected to X₃, and two ends are positions connected to the corresponding amino or carboxyl groups in the modified amino acids;
X₃ is selected from 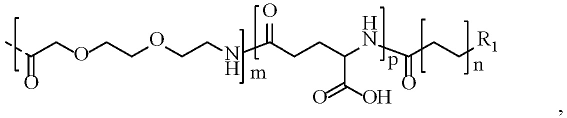 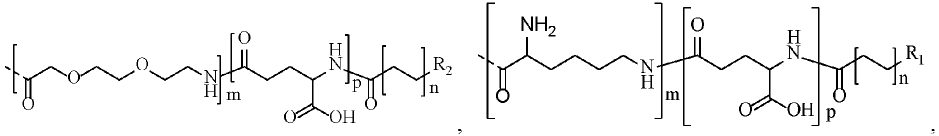 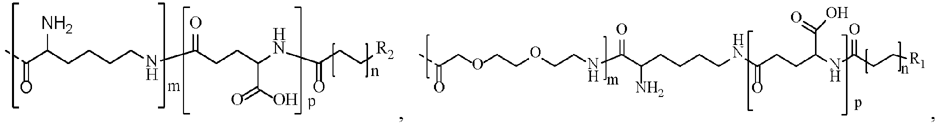 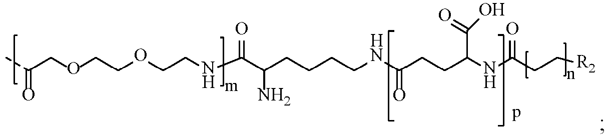
R₁ and R₂ are independently selected from 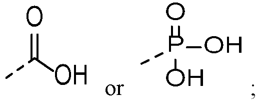
m is selected from 1, 2 or 3;
p is selected from 1 or 2;
n is selected from 8, 9 or 10.

In some embodiments of the present disclosure, the polypeptide having a modified structure or the pharmaceutically acceptable salt thereof has a polypeptide sequence as shown in Formula Z-1:

X₅X₀X₆GT FTSDY SX_{b}X₁X₇X₈ KX₉X₁₀X₁₁X₀ X₄FX₁₂X₁₃X₁₄ X_{c}X_{d}X₁₅GG PSSGA PPPSo Formula Z-1

the compound (polypeptide) has the following modifications:
1) side chains of amino acids at positions i and i+j in the sequence can be connected by a modification to form a modified structure similar to a "staple" (peptide-staple) structure (stapled body), where i is independently selected from 12, 14, 16, 17, 19, 21, 24 or 25, and j is independently selected from 2, 3, 4, 5 or 7,
2) the modification is formed by the condensation connection of amino or carboxyl groups on the side chains of two amino acid unit structures to 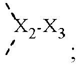 and other variables are as defined in the present disclosure.

In some embodiments of the present disclosure, the polypeptide having a modified structure or the pharmaceutically acceptable salt thereof has a polypeptide sequence as shown in Formula Z-2:

X₅X₀X₆GT FTSDY SIX₁X₇X₈ KX₉X₁₀X₁₁X₀ X₄FX₁₂X₁₃X₁₄ LLX₁₅GG PSSGA PPPSo Formula Z-2

the compound (polypeptide) has the following modifications:
1) side chains of amino acids at positions i and i+j in the sequence can be connected by a modification to form a modified structure similar to a "staple" structure (stapled body), where i is independently selected from 12, 14, 16, 17, 19, 21, 24, 25, and j is independently selected from 2, 3, 4, 5, 7,
2) the modification is formed by the condensation connection of amino or carboxyl groups on the side chains of two amino acid unit structures to 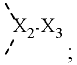 and other variables are as defined in the present disclosure.

In some embodiments of the present disclosure, the polypeptide having a modified structure or the pharmaceutically acceptable salt thereof has a polypeptide sequence as shown in Formula Z-3:

X₅X₀X₆GT FTSDY SIX₁X₇X₈ KX₉X₁₀X₁₁X₀ X₄FX₁₂X₁₃X₁₄ LLX₁₅GG PSSGA PPPSo Formula Z-3

and the polypeptide compound has the following modifications:
1) side chains of amino acids at positions i and i+j in the sequence can be connected by a modification to form a modified structure similar to a "staple" structure (stapled body), where i is independently selected from 14, 17, 19, 21, 24 or 25, and j is independently selected from 3, 4, 5 or 7,
2) the modification is formed by the condensation connection of amino or carboxyl groups on the side chains of two amino acid unit structures to

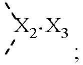

X₂ is selected from 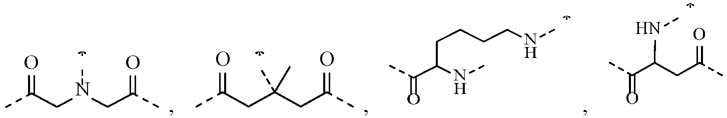 or 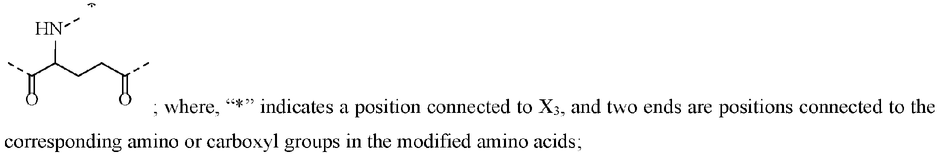 X₃ is selected from: 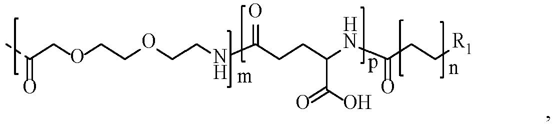 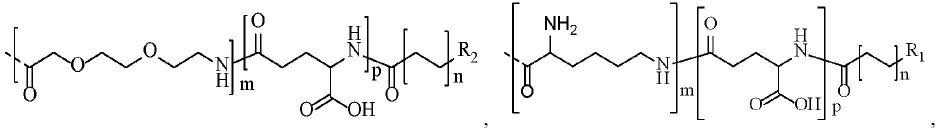 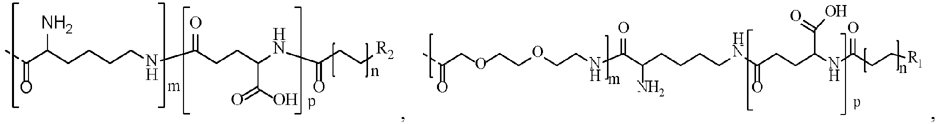 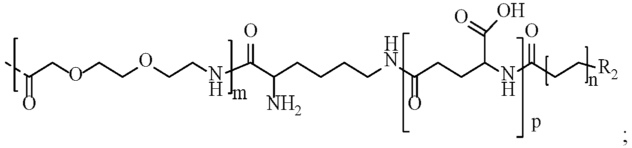
R₁ and R₂ are independently selected from 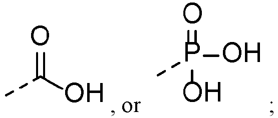
m is selected from 1, 2 or 3;
p is selected from 1 or 2;
n is selected from 8, 9 or 10; and other variables are as defined in the present disclosure.

In some embodiments of the present disclosure, the polypeptide having a modified structure or the pharmaceutically acceptable salt thereof has a polypeptide sequence as shown in Formula Z-4:

YX₀QGT FTSDY SX_{b}X₁LD KKAQX₀ X₄FX₁₂X₁₃X₁₄ X_{c}X_{d}X₁₅GG PSSGA PPPSo Formula Z-4

S₀ is selected from 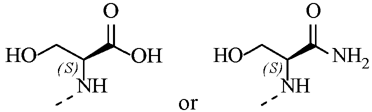 (i.e., the C-terminal amino acid is optionally amidated to a C-terminal primary amide);
and the polypeptide compound has the following modifications:
   1) side chains of amino acids at positions i and i+j in the sequence can be connected by a modification to form a modified structure similar to a "staple" structure (stapled body), where i is independently selected from 12, 17, 21 or 25; and j is independently selected from 3, 4 or 7,
   2) the modification is formed by the condensation connection of amino or carboxyl groups on the side chains of two amino acid unit structures to 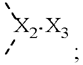
X₂ is selected from 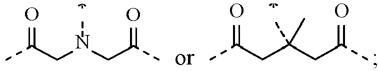 and where, "*" indicates a position connected to X₃, and two ends are positions connected to the corresponding amino or carboxyl groups in the modified amino acids;
X₃ is selected from: 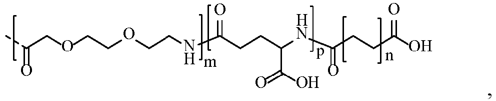 or 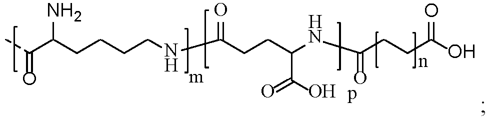
m is selected from 1, 2 or 3;
p is selected from 1 or 2;
n is selected from 8, 9 or 10; and other variables are as defined in the present disclosure.

In some embodiments of the present disclosure, the polypeptide having a modified structure or the pharmaceutically acceptable salt thereof has a polypeptide sequence as shown in Formula Z-5:

YX₀QGT XₐTSDY SIX₁KD KKAQX₀ X₄FIX₁₃Y LLX₁₅GG PSSGA PPPSo Formula Z-5

and the polypeptide compound has the following modifications:
1) side chains of amino acids at positions i and i+j in the sequence can be connected by a modification to form a modified structure similar to a "staple" structure (stapled body), where i is independently selected from 14; and j is independently selected from 3,
2) the modification is formed by the condensation connection of amino or carboxyl groups on the side chains of two amino acid unit structures to

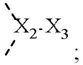

X₂ is selected from 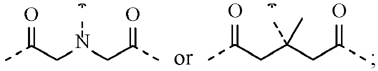 where, "*" indicates a position connected to X₃, and two ends are positions connected to the corresponding amino or carboxyl groups in the modified amino acids;
X₃ is selected from: 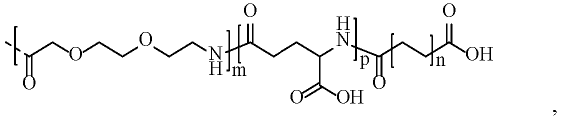 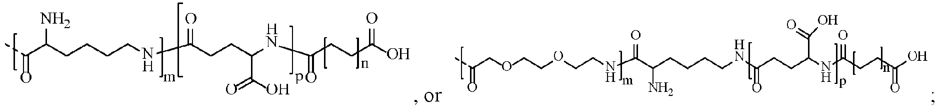
m is selected from 1, 2 or 3;
p is selected from 1 or 2;
n is selected from 8, 9 or 10; and other variables are as defined in the present disclosure.

In some embodiments of the present disclosure, the polypeptide having a modified structure or the pharmaceutically acceptable salt thereof has a polypeptide sequences as shown in Formulas Z-6, Z-7, Z-8, Z-9, Z-10, Z-11, Z-12, or Z-13, respectively:

X₅X₀X₆GT FTSDY SIX₁LX₈ KKX₁₀X₁₁X₀ X₄FX₁₂X₁₃X₁₄ LLX₁₅GG PSSGA PPPSo Formula Z-6

YX₀QGT FTSDY SIX₁LD KKAX₁₁X₀ X₄FIX₁₃X₁₄ LLX₁₅GG PSSGA PPPSo Formula Z-7

YX₀QGT FTSDY SIX₁X₇D KKAQX₀ X₄FIX₁₃Y LLX₁₅GG PSSGA PPPS₀ Formula Z-8

YX₀QGT FTSDY SXbX₁LD KKAQX₀ X₄FX₁₂EX₁₄ X_{c}XdX₁₅GG PSSGA PPPSo Formula Z-9

YX₀QGT FTSDY SX₁X₁LD KKAQX₀ KFX₁₂EX₁₄ LX₄X₁₅GG PSSGA PPPSo Formula Z-10

YX₀QGT FTSDY SX₁X₁KD KKAQX₀ X₄FIEY LLX₁₅GG PSSGA PPPSo Formula Z-11

YX₀QGT FTSDY SIX₁KD KKAQX₀ X₄FIEY LLEGG PSSGA PPPSo Formula Z-12

YX₀QGT XₐTSDY SIX₁KD KKAQX₀ X₄FIEY LLX₁₅GG PSSGA PPPS₀ Formula Z-13

the compound (polypeptide) has the following modifications:
1) side chains of amino acids at positions i and i+j in the sequence can be connected by a modification to form a modified structure similar to a "staple" structure (stapled body), where i is independently selected from 12, 14, 16, 17, 19, 21, 24 or 25, and j is independently selected from 2, 3, 4, 5 or 7,
2) the modification is formed by the condensation connection of amino or carboxyl groups on the side chains of two amino acid unit structures to 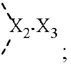 X₂ is selected from 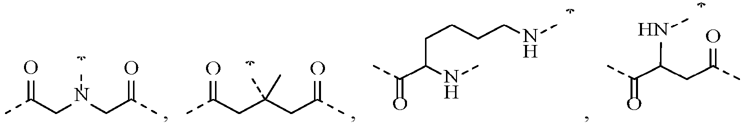 or 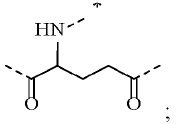 where, "*" indicates a position connected to X₃, and two ends are positions connected to the corresponding amino or carboxyl groups in the modified amino acids;

X₃ is selected from: 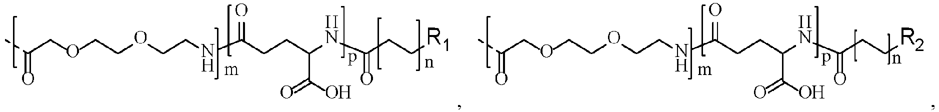 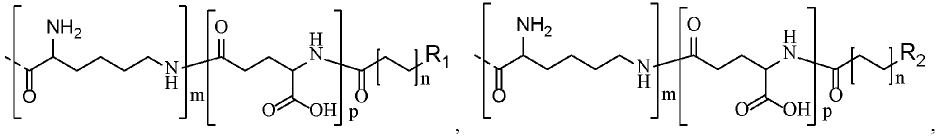 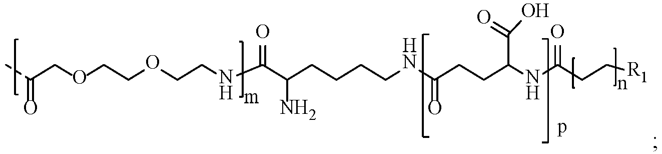
R₁ and R₂ are independently selected from 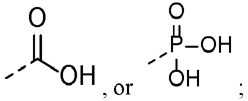
m is selected from 1, 2 or 3;
p is selected from 1 or 2;
n is selected from 8, 9 or 10; and other variables are as defined in the present disclosure.

In some embodiments of the present disclosure, S₀ in the polypeptide sequence is 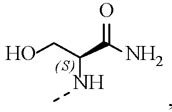 i.e., the C-terminal amino acid is amidated to a primary amide, and other variables are as defined in the present disclosure.

**In** some embodiments of the present disclosure, the amino acid X₀ at position 2 in the polypeptide sequence is Aib, and other variables are as defined in the present disclosure.

**In** some embodiments of the present disclosure, the amino acid X₀ at position 20 in the polypeptide sequence is Aib, and other variables are as defined in the present disclosure.

**In** some embodiments of the present disclosure, the amino acids X₀ at positions 2 and 20 in the polypeptide sequence are both Aib, and other variables are as defined in the present disclosure.

**In** some embodiments of the present disclosure, i is 12, j is 4, i+j is 16, and other variables are as defined in the present disclosure.

In some embodiments of the present disclosure, i is 14, j is 3, i+j is 17, and other variables are as defined in the present disclosure.

In some embodiments of the present disclosure, i is 16, j is 3, i+j is 19, and other variables are as defined in the present disclosure.

In some embodiments of the present disclosure, i is 17, j is 4, i+j is 21, and other variables are as defined in the present disclosure.

In some embodiments of the present disclosure, i is 17, j is 7, i+j is 24, and other variables are as defined in the present disclosure.

In some embodiments of the present disclosure, i is 19, j is 5, i+j is 24, and other variables are as defined in the present disclosure.

In some embodiments of the present disclosure, i is 21, j is 7, i+j is 28, and other variables are as defined in the present disclosure.

In some embodiments of the present disclosure, i is 24, j is 4, i+j is 28, and other variables are as defined in the present disclosure.

In some embodiments of the present disclosure, i is 25, j is 3, i+j is 28, and other variables are as defined in the present disclosure.

In some embodiments of the present disclosure, the amino acid X₅ at position 1 in the polypeptide sequence is Y, and other variables are as defined in the present disclosure.

In some embodiments of the present disclosure, the amino acid X₅ at position 1 in the polypeptide sequence is H, and other variables are as defined in the present disclosure.

In some embodiments of the present disclosure, the amino acid X₆ at position 3 in the polypeptide sequence is Q, and other variables are as defined in the present disclosure.

In some embodiments of the present disclosure, the amino acid X₆ at position 3 in the polypeptide sequence is H, and other variables are as defined in the present disclosure.

In some embodiments of the present disclosure, the amino acid Xₐ at position 6 in the polypeptide sequence is F, and other variables are as defined in the present disclosure.

In some embodiments of the present disclosure, the amino acid Xₐ at position 6 in the polypeptide sequence is α-MeF, and other variables are as defined in the present disclosure.

In some embodiments of the present disclosure, the amino acid X_{b} at position 12 in the polypeptide sequence is I, and other variables are as defined in the present disclosure.

In some embodiments of the present disclosure, the amino acid X_{b} at position 12 in the polypeptide sequence is K, and other variables are as defined in the present disclosure.

In some embodiments of the present disclosure, the amino acid X₁ at position 13 in the polypeptide sequence is α-MeL, and other variables are as defined in the present disclosure.

In some embodiments of the present disclosure, the amino acid X₁ at position 13 in the polypeptide sequence is Y, and other variables are as defined in the present disclosure.

In some embodiments of the present disclosure, the amino acid X₁ at position 13 in the polypeptide sequence is Aib, and other variables are as defined in the present disclosure.

In some embodiments of the present disclosure, the amino acid X₇ at position 14 in the polypeptide sequence is L, and other variables are as defined in the present disclosure.

In some embodiments of the present disclosure, the amino acid X₇ at position 14 in the polypeptide sequence is K, and other variables are as defined in the present disclosure.

In some embodiments of the present disclosure, the amino acid X₈ at position 15 in the polypeptide sequence is D, and other variables are as defined in the present disclosure.

In some embodiments of the present disclosure, the amino acid X₈ at position 15 in the polypeptide sequence is E, and other variables are as defined in the present disclosure.

In some embodiments of the present disclosure, the amino acid X₉ at position 17 in the polypeptide sequence is K, and other variables are as defined in the present disclosure.

In some embodiments of the present disclosure, the amino acid X₉ at position 17 in the polypeptide sequence is Q, and other variables are as defined in the present disclosure.

In some embodiments of the present disclosure, the amino acid X₉ at position 17 in the polypeptide sequence is E, and other variables are as defined in the present disclosure.

In some embodiments of the present disclosure, the amino acid X₁₀ at position 18 in the polypeptide sequence is A, and other variables are as defined in the present disclosure.

In some embodiments of the present disclosure, the amino acid X₁₀ at position 18 in the polypeptide sequence is Y, and other variables are as defined in the present disclosure.

In some embodiments of the present disclosure, the amino acid X₁₁ at position 19 in the polypeptide sequence is Q, and other variables are as defined in the present disclosure.

In some embodiments of the present disclosure, the amino acid X₁₁ at position 19 in the polypeptide sequence is A, and other variables are as defined in the present disclosure.

In some embodiments of the present disclosure, the amino acid X₁₁ at position 19 in the polypeptide sequence is K, and other variables are as defined in the present disclosure.

In some embodiments of the present disclosure, the amino acid X₄ at position 21 in the polypeptide sequence is K, and other variables are as defined in the present disclosure.

In some embodiments of the present disclosure, the amino acid X₄ at position 21 in the polypeptide sequence is A, and other variables are as defined in the present disclosure.

In some embodiments of the present disclosure, the amino acid X₄ at position 21 in the polypeptide sequence is E, and other variables are as defined in the present disclosure.

In some embodiments of the present disclosure, the amino acid X₄ at position 21 in the polypeptide sequence is α-MeK, and other variables are as defined in the present disclosure.

In some embodiments of the present disclosure, the amino acid X₄ at position 21 in the polypeptide sequence is d-K, and other variables are as defined in the present disclosure.

In some embodiments of the present disclosure, the amino acid X₄ at position 21 in the polypeptide sequence is L-Om, and other variables are as defined in the present disclosure.

In some embodiments of the present disclosure, the amino acid X₄ at position 21 in the polypeptide sequence is L, and other variables are as defined in the present disclosure.

**In** some embodiments of the present disclosure, the amino acid X₁₂ at position 23 in the polypeptide sequence is I, and other variables are as defined in the present disclosure.

**In** some embodiments of the present disclosure, the amino acid X₁₂ at position 23 in the polypeptide sequence is V, and other variables are as defined in the present disclosure.

**In** some embodiments of the present disclosure, the amino acid X₁₃ at position 24 in the polypeptide sequence is E, and other variables are as defined in the present disclosure.

**In** some embodiments of the present disclosure, the amino acid X₁₃ at position 24 in the polypeptide sequence is K, and other variables are as defined in the present disclosure.

**In** some embodiments of the present disclosure, the amino acid X₁₃ at position 24 in the polypeptide sequence is Q, and other variables are as defined in the present disclosure.

**In** some embodiments of the present disclosure, the amino acid X₁₄ at position 25 in the polypeptide sequence is Y, and other variables are as defined in the present disclosure.

**In** some embodiments of the present disclosure, the amino acid X₁₄ at position 25 in the polypeptide sequence is W, and other variables are as defined in the present disclosure.

**In** some embodiments of the present disclosure, the amino acid X₁₄ at position 25 in the polypeptide sequence is K, and other variables are as defined in the present disclosure.

**In** some embodiments of the present disclosure, the amino acid X₁₄ at position 25 in the polypeptide sequence is E, and other variables are as defined in the present disclosure.

**In** some embodiments of the present disclosure, the amino acid X₁₄ at position 25 in the polypeptide sequence is F, and other variables are as defined in the present disclosure.

**In** some embodiments of the present disclosure, the amino acid X_{c} at position 26 in the polypeptide sequence is L, and other variables are as defined in the present disclosure.

**In** some embodiments of the present disclosure, the amino acid X_{c} at position 26 in the polypeptide sequence is F, and other variables are as defined in the present disclosure.

**In** some embodiments of the present disclosure, the amino acid X_{d} at position 27 in the polypeptide sequence is L, and other variables are as defined in the present disclosure.

**In** some embodiments of the present disclosure, the amino acid X_{d} at position 27 in the polypeptide sequence is I, and other variables are as defined in the present disclosure.

**In** some embodiments of the present disclosure, the amino acid X₁₅ at position 28 in the polypeptide sequence is E, and other variables are as defined in the present disclosure.

**In** some embodiments of the present disclosure, the amino acid X₁₅ at position 28 in the polypeptide sequence is K, and other variables are as defined in the present disclosure.

**In** some embodiments of the present disclosure, the X₃ is selected from: 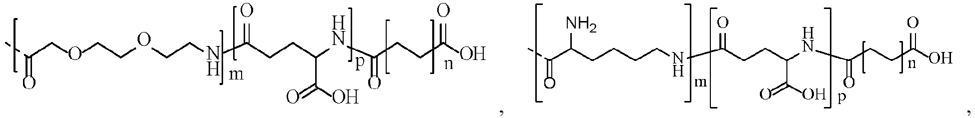 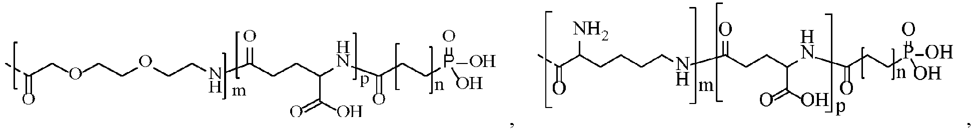 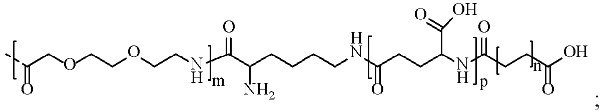 and other variables are as defined in the present disclosure.

In some embodiments of the present disclosure, m in the X₃ structure is selected from 1 or 2, and other variables are as defined in the present disclosure.

In some embodiments of the present disclosure, p in the X₃ structure is selected from 1, and other variables are as defined in the present disclosure.

In some embodiments of the present disclosure, n in the X₃ structure is selected from 9, and other variables are as defined in the present disclosure.

In some embodiments of the present disclosure, the X₃ is selected from: 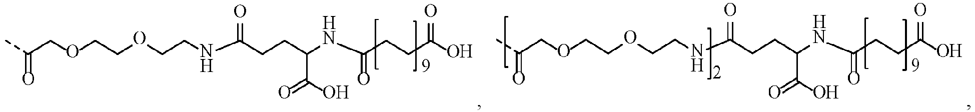 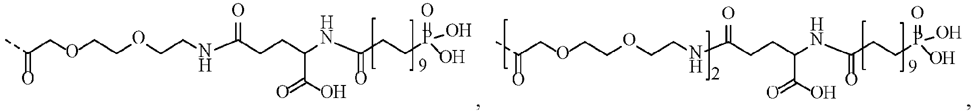 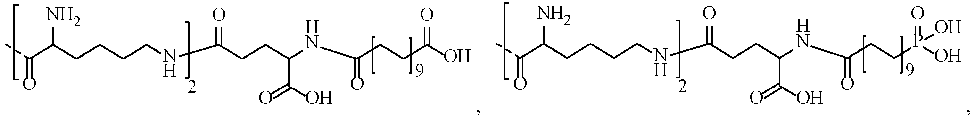 or 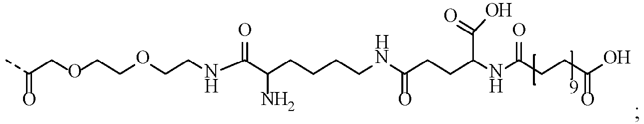 and other variables are as defined in the present disclosure.

In some embodiments of the present disclosure, the X₃ is selected from: 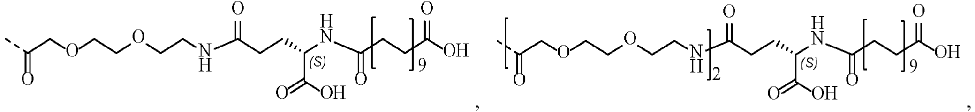 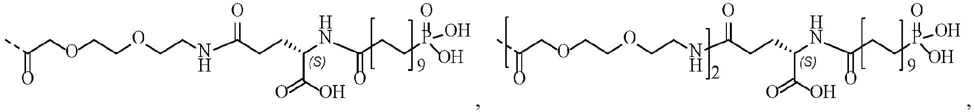 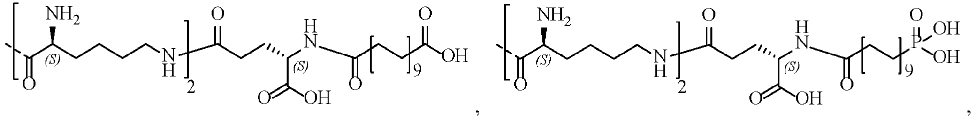 or 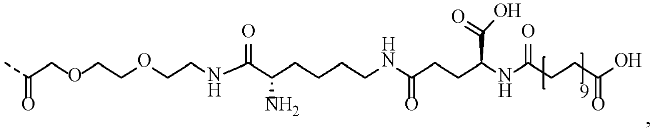 and other variables are as defined in the present disclosure.

In some embodiments of the present disclosure, the structural unit 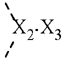 is: 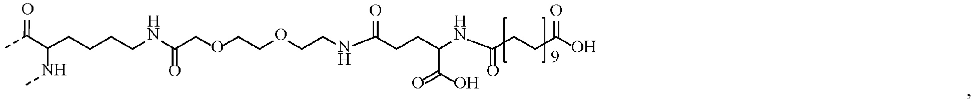 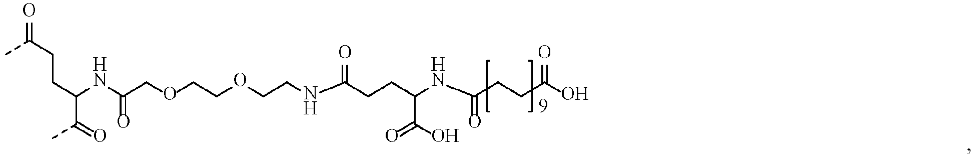 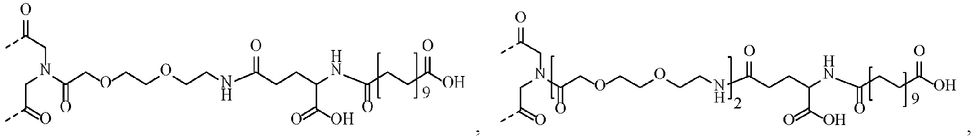 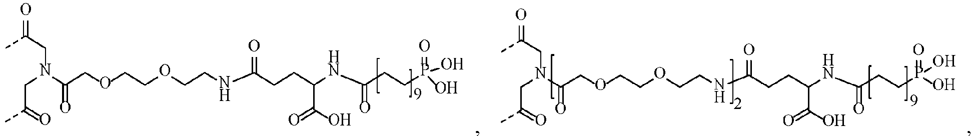 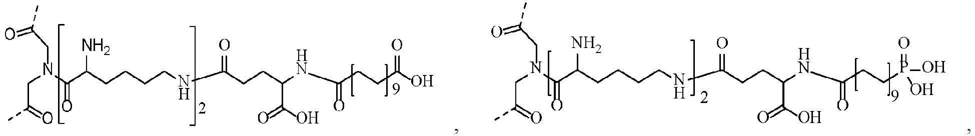 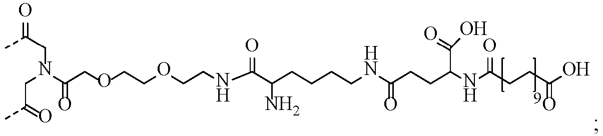 and other variables are as defined in the present disclosure.

In some embodiments of the present disclosure, the structural unit 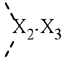 is: 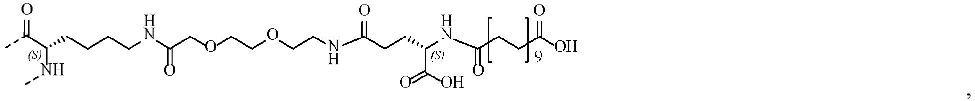 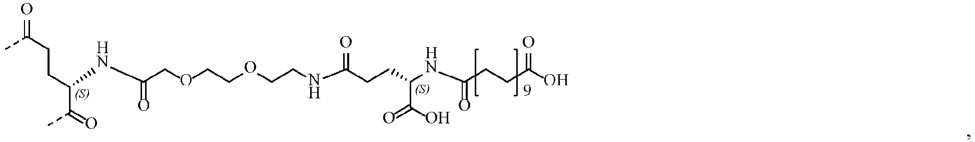 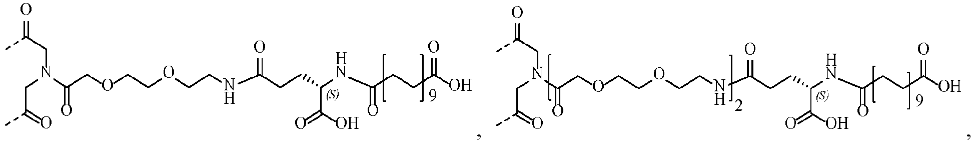 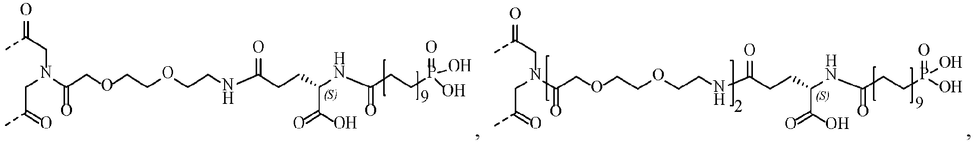 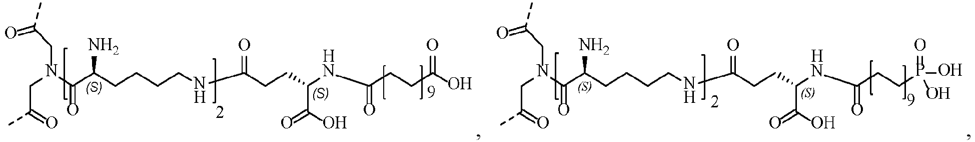 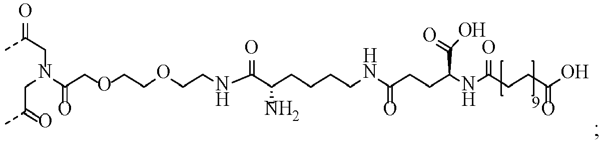 and other variables are as defined in the present disclosure.

The present disclosure provides a polypeptide having a modified structure as shown in Formula I or a pharmaceutically acceptable salt thereof:

Formula I: X₅X₀X₆GT FTSDY SIX₁X₇X₈ KX₉X₁₀X₁₁X₀ X₄FX₁₂X₁₃X₁₄ LLX₁₅GG PSSGA PPPS₀

wherein:
X₀ is independently selected from 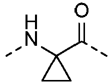 or Aib or alanine (Ala, A), with the structure of Aib being 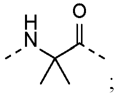
X₁ is independently selected from α-methyl-substituted leucine (α-MeLeu, α-MeL) or tyrosine (Tyr, Y), with the structure of α-MeL being 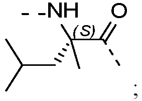
X₄ is selected from α-methyl-substituted lysine (α-MeLys, α-MeK) of a structure of 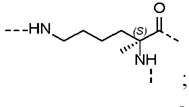 or D-lysine (d-K) of a structure of 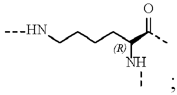 or L-omithine (L-Om) of a structure of 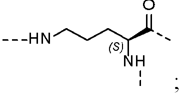 or alanine (Ala, A), lysine (Lys, K) or glutamic acid (Glu, E);
X₅ is selected from tyrosine (Tyr, Y) or histidine (His, H);
X₆ is selected from glutamine (Gln, Q) or histidine (His, H);
X₇ is selected from leucine (Leu, L) or lysine (Lys, K);
X₈ is selected from aspartic acid (Asp, D) or glutamic acid (Glu, E);
X₉ is selected from lysine (Lys, K), glutamine (Gln, Q) or glutamic acid (Glu, E);
X₁₀ is selected from alanine (Ala, A) or tyrosine (Tyr, Y);
X₁₁ is selected from lysine (Lys, K), glutamine (Gln, Q) or alanine (Ala, A);
X₁₂ is selected from valine (Val, V) or isoleucine (Ile, I);
X₁₃ is selected from lysine (Lys, K), glutamine (Gln, Q) or glutamic acid (Glu, E);
X₁₄ is selected from lysine (Lys, K), tryptophan (Trp, W), tyrosine (Tyr, Y), glutamic acid (Glu, E) or phenylalanine (Phe, F);
X₁₅ is selected from lysine (Lys, K) or glutamic acid (Glu, E);
S₀ is selected from 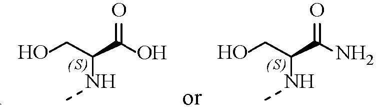 (i.e., the C-terminal amino acid is optionally amidated to a C-terminal primary amide);
and the compound has the following modifications:
   1) side chains of amino acids at positions i and i+j in the sequence can be connected by a modification to form a modified structure similar to a "staple" structure (stapled body), where i is independently selected from 12, 14, 16, 17, 19, 21, 24 or 25, and j is independently selected from 2, 3, 4, 5 or 7,
   2) the modification is formed by the condensation connection of amino or carboxyl groups on the side chains of two amino acid unit structures to 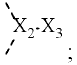
X₂ is selected from 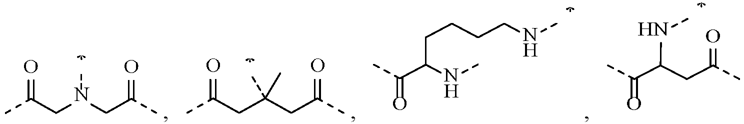 or 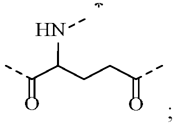 where, "*" indicates a position connected to X₃, and two ends are positions connected to the corresponding amino or carboxyl groups in the modified amino acids;
X₃ is selected from: 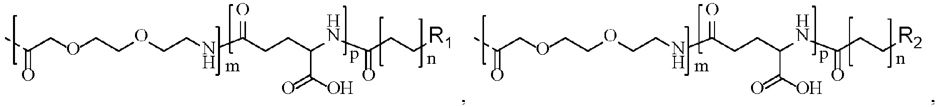 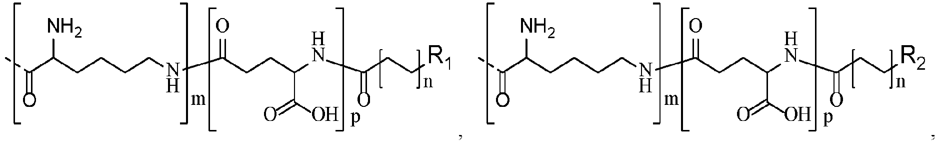 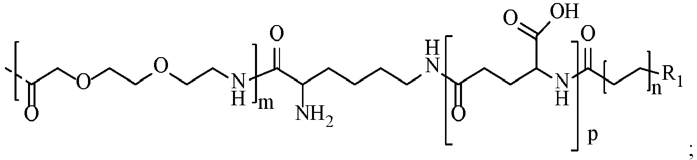
R₁ and R₂ are independently selected from 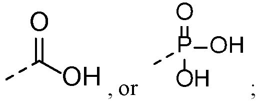
m is selected from 1, 2 or 3; p is selected from 1 or 2; and n is selected from 8, 9 or 10.

In some embodiments of the present disclosure, the X₃ is selected from 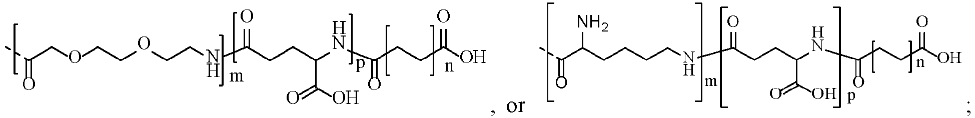 and other variables are as defined in the present disclosure.

In some embodiments of the present disclosure, the X₃ is selected from 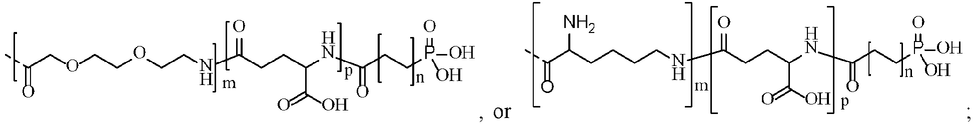 and other variables are as defined in the present disclosure.

In some embodiments of the present disclosure, the X₃ is selected from 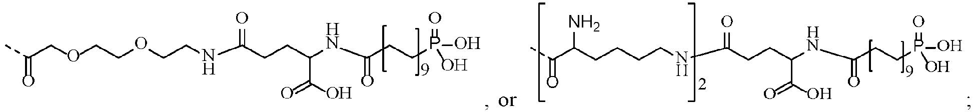 and other variables are as defined in the present disclosure.

In some embodiments of the present disclosure, the structural unit 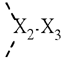 is 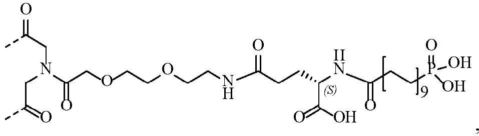 and other variables are as defined in the present disclosure.

In some embodiments of the present disclosure, the structural unit 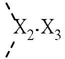 is 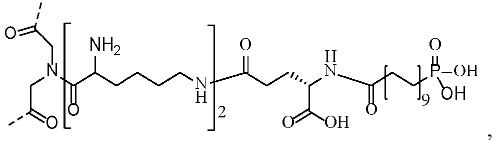 and other variables are as defined in the present disclosure.

The present disclosure provides a polypeptide having a modified structure as shown in Formula I or a pharmaceutically acceptable salt thereof:

Formula I: X₅X₀X₆GT FTSDY SIX₁X₇X₈ KX₉X₁₀X₁₁X₀ X₄FX₁₂X₁₃X₁₄ LLX₁₅GG PSSGA PPPS₀

wherein:
X₀ may be independently selected from 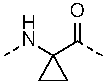 or Aib or alanine (Ala, A), with the structure of Aib being 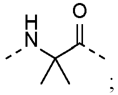
X₁ may be independently selected from α-methyl-substituted leucine (α-MeLeu, α-MeL) or tyrosine (Tyr, Y), with the structure of α-MeL being 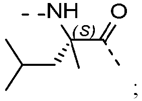
X₄ is selected from α-methyl-substituted lysine (α-MeLys, α-MeK) of a structure of 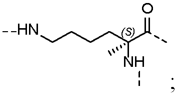 or D-lysine (d-K) of a structure of 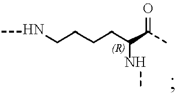 or L-omithine (L-Om) of a structure of 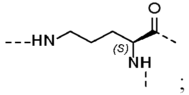 or alanine (Ala, A), lysine (Lys, K) or glutamic acid (Glu, E);
X₅ is selected from tyrosine (Tyr, Y) or histidine (His, H);
X₆ is selected from glutamine (Gln, Q) or histidine (His, H);
X₇ is selected from leucine (Leu, L) or lysine (Lys, K);
X₈ is selected from aspartic acid (Asp, D) or glutamic acid (Glu, E);
X₉ is selected from lysine (Lys, K), glutamine (Gln, Q) or glutamic acid (Glu, E);
X₁₀ is selected from alanine (Ala, A) or tyrosine (Tyr, Y);
X₁₁ is selected from lysine (Lys, K), glutamine (Gln, Q) or alanine (Ala, A);
X₁₂ is selected from valine (Val, V) or isoleucine (Ile, I);
X₁₃ is selected from lysine (Lys, K), glutamine (Gln, Q) or glutamic acid (Glu, E);
X₁₄ is selected from lysine (Lys, K), tryptophan (Trp, W), tyrosine (Tyr, Y), glutamic acid (Glu, E) or phenylalanine (Phe, F);
X₁₃ is selected from lysine (Lys, K) or glutamic acid (Glu, E);
S₀ is selected from 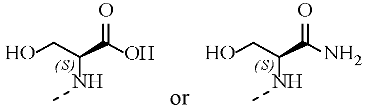 (i.e., the C-terminal amino acid is optionally amidated to a C-terminal primary amide);
and the compound has the following modifications:
   1) side chains of amino acids at positions i and i+j in the sequence can be connected by a modification to form a modified structure similar to a "staple" structure (stapled body), where i is independently selected from 12, 14, 16, 17, 19, 21, 24 or 25, and j is independently selected from 2, 3, 4, 5 or 7,
   2) the modification is formed by the condensation connection of amino or carboxyl groups on the side chains of two amino acid unit structures to 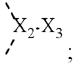
X₂ is selected from 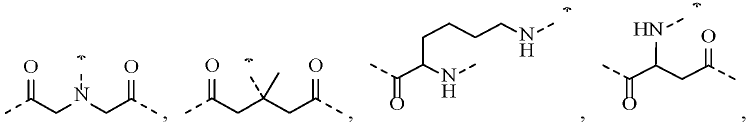 or 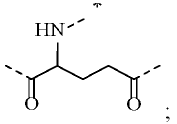 where, "*" indicates a position connected to X₃, and two ends are positions connected to the corresponding amino or carboxyl groups in the modified amino acids;
   X₃ is selected from: 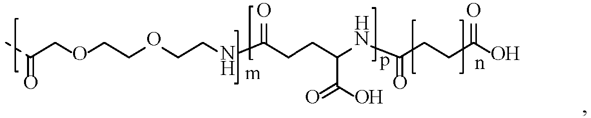 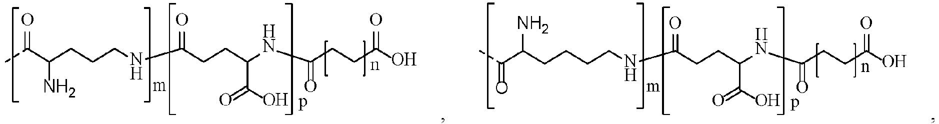 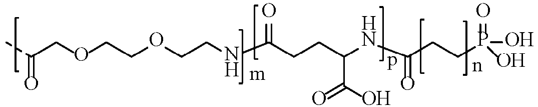 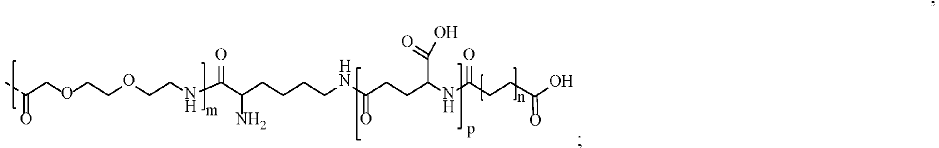
m is selected from 1, 2 or 3; p is selected from 1 or 2; and n is selected from 8, 9 or 10.

In some embodiments of the present disclosure, the amino acids with the same superscript number in the peptide chain sequence of the polypeptide having a modified structure can be connected by a modification to form a modified group similar to a "staple" structure (stapled body), that is, the side chains of amino acids at positions i and i+j in the sequence can be connected by a modification to form a modified structure similar to a "staple" structure (stapled body), where i is any integer independently selected from 10 to 28, e.g., 10, 12, 14, 16, 17, 19, 21, 24 or 25, and j is any integer independently selected from 1 to 8, e.g., 1, 2, 3, 4, 5, 7 or 8. The number of the "staple" structures is 1-2. In some embodiments, the first connected amino acid is located at position 25 of the peptide, and the second connected amino acid is located at position 28 of the peptide. In some embodiments, the first connected amino acid is located at position 24 of the peptide, and the second connected amino acid is located at position 28 of the peptide. In some embodiments, the first connected amino acid is located at position 17 of the peptide, and the second connected amino acid is located at position 21 of the peptide. In some embodiments, the first connected amino acid is located at position 16 of the peptide, and the second connected amino acid is located at position 19 of the peptide. In some embodiments, the first connected amino acid is located at position 17 of the peptide, and the second connected amino acid is located at position 24 of the peptide. In some embodiments, the first connected amino acid is located at position 19 of the peptide, and the second connected amino acid is located at position 24 of the peptide. In some embodiments, the first connected amino acid is located at position 21 of the peptide, and the second connected amino acid is located at position 28 of the peptide. In some embodiments, the first connected amino acid is located at position 19 of the peptide, and the second connected amino acid is located at position 24 of the peptide.

In some embodiments of the present disclosure, the polypeptide having a modified structure may have a peptide chain sequence with at most 0, 1, 2, 3 or 4 amino acid insertions, deletions, modifications or substitutions. For example, isoleucine (I) at position 12 may be replaced by lysine (K); leucine (L) at position 26 or 27 may be replaced by isoleucine (I) or phenylalanine (F); X₁ at position 13 may be replaced by X₀; or X₀ at position 20 may be replaced by a natural amino acid such as alanine (A), etc.

The present disclosure provides a series of polypeptides with sequences as shown below:
YX₀QGT FTSDY SIX₁LD KKAQX₀ AFIE¹K LL¹EGG PSSGA PPPSo
YX₀QGT FTSDY SIX₁LD KKAQX₀ AFI¹KW LL¹KGG PSSGA PPPSo
YX₀QGT FTSDY SIX₁LD K¹KAQX₀ ¹KFIEY LLEGG PSSGA PPPSo
HX₀HGT FTSDY SIYLE KKYAX₀ EFV¹KW LL¹KGG PSSGA PPPSo
HX₀HGT FTSDY SIYLE K¹KYAX₀ ¹KFVQW LLEGG PSSGA PPPSo
YX₀QGT FTSDY SIX₁LD KKAQX₀ AFIQ¹K LL¹KGG PSSGA PPPSo
YX₀QGT FTSDY SIX₁LD ¹KQA¹QX₀ AFIE²K LL²KGG PSSGA PPPSo
YX₀QGT FTSDY SIX₁LD KEAQX₀AFIE¹K LL¹KGG PSSGA PPPSo
HX₀QGT FTSDY SIX₁LD KKAQX₀ AFIE¹K LL¹KGG PSSGA PPPSo
YX₀QGT FTSDY SIX₁LD KKYQX₀ AFIE¹K LL¹KGG PSSGA PPPSo
YX₀QGT FTSDY SIX₁LE KKAQX₀ AFIE¹K LL¹KGG PSSGA PPPSo
HX₀HGT FTSDY SIYLE KKYAX₀ EFVQ¹E LL¹KGG PSSGA PPPSo
YX₀QGT FTSDY SIX₁LD KKAQX₀ AFIE¹E LL¹KGG PSSGA PPPSo
YX₀QGT FTSDY SIX₁LD KKAQX₀ AFI¹EW LL¹KGG PSSGA PPPSo
YX₀QGT FTSDY SIX₁LD K¹KAQX₀ ¹KFIEF LLEGG PSSGA PPPSo
YX₀QGT FTSDY SIX₁LD K¹KAQX₀ ¹EFIEY LLEGG PSSGA PPPSo
YX₀QGT FTSDY SIX₁LD K¹KAQX₀ AFI¹KY LLEGG PSSGA P PPSo
YX₀QGT FTSDY SIX₁LD K¹KAQX₀ ¹KFIEY LLEGG PSSGA P PPSo
YX₀QGT FTSDY SIX₁LD KKA¹KX₀ AFI¹KY LLEGG PSSGA PPPSo
YX₀QGT FTSDY SIX₁LD KKAQX₀ ¹KFIEY LL¹KGG PSSGA PPPSo
YX₀QGT FTSDY SIX₁LD K¹KAQA ¹X₄FIEY LLEGG PSSGA PPPSo
YX₀QGT FTSDY SIX₁LD K¹KAQX₀ ¹X₄FIEY LLEGG PSSGA PPPSo
YX₀QGT FTSDY SIX₁LD K¹KAQX₀ ¹X₄FIEY LLEGG PSSGA PPPSo
YX₀QGT FTSDY SIX₁¹KD K¹KAQX₀ AFIEY LLEGG PSSGA PPPSo

The superscript (left superscript) in the sequence refers to the modified amino acid unit structure, and the amino acids with the same superscript number refer to the modified amino acid unit structures connected by a modifying group to form a structure similar to a "staple" (stapled body). For example, ¹K and ¹E in the above sequences represent modified lysine and glutamic acid, and the two are connected by a modifying group (K-E modification); further for example, ¹K and ¹K in the above sequences represent two modified lysines, and the two are connected by a modifying group (K-K modification); and further for example, ¹K and ¹X₄ in the above sequences represent modified lysine and X₄, and the two are connected by a modifying group (K-X₄ modification); where X₀, X₁, X₄ and S₀ as well as the modification manner are as defined in the present disclosure.

The present disclosure provides a series of polypeptides with sequences as shown below:
YX₀QGT FTSDY SIX₁LD K¹KAQX₀ ¹KFIEF LLEGG PSSGA PPPSo
YX₀QGT FTSDY SIX₁LD K¹KAQX₀ ¹KFIEF LIEGG PSSGA PPPSo
YX₀QGT FTSDY SIX₁LD K¹KAQX₀ ¹KFVEF LIEGG PSSGA PPPSo
YX₀QGT FTSDY SIX₁LD KKAQX₀ AFIE¹K FI¹KGG PSSGA PPPSo
YX₀QGT FTSDY SIX₁LD KKAQX₀ ¹KFIEF LL¹KGG PSSGA PPPSo
YX₀QGT FTSDY SIX₀LD K¹KAQX₀ ¹KFIEF LLEGG PSSGA PPPSo
YX₀QGT FTSDY S¹KX₁LD ¹KKAQX₀ AFIEF LLEGG PSSGA PPPSo
wherein, X₀, X₁ and S₀ as well as the modification manner are as defined above.

The present disclosure provides a series of polypeptides with sequences as shown below:
YX₀QGT FTSDY SIX₁LD K¹KAQX₀ ¹KFIEF LLEGG PSSGA PPPS-NH₂
YX₀QGT FTSDY SIX₁LD K¹KAQX₀ ¹KFIEF LIEGG PSSGA PPPS-NH₂
YX₀QGT FTSDY SIX₁LD K¹KAQX₀ ¹KFVEF LIEGG PSSGA PPPS-NH₂
YX₀QGT FTSDY SIX₁LD KKAQX₀ AFIE¹K FI¹KGG PSSGA PPPS-NH₂
YX₀QGT FTSDY SIX₁LD KKAQX₀ ¹KFIEF LL¹KGG PSSGA PPPS-NH₂
YX₀QGT FTSDY SIX₀LD K¹KAQX₀ ¹KFIEF LLEGG PSSGA PPPS-NH₂
YX₀QGT FTSDY S¹KX₁LD ¹KKAQX₀ AFIEF LLEGG PSSGA PPPS-NH₂
wherein, S₀ is 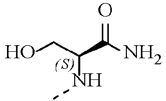 (i.e., the C-terminal amino acid is amidated to a C-terminal primary amide), X₀, X₁ and S₀ as well as the modification manner are as defined above.

The present disclosure provides a series of polypeptides with sequences as shown below, where some X₀ are Aib:
YX₀QGT FTSDY SIX₁LD K¹KAQAib ¹KFIEF LLEGG PSSGA PPPS-NH₂
YX₀QGT FTSDY SIX₁LD K¹KAQAib ¹KFIEF LIEGG PSSGA PPPS-NH₂
YX₀QGT FTSDY SIX₁LD K¹KAQAib ¹KFVEF LIEGG PSSGA PPPS-NH₂
YX₀QGT FTSDY SIX₁LD KKAQAib AFIE¹K FI¹KGG PSSGA PPPS-NH₂
YX₀QGT FTSDY SIX₁LD KKAQAib ¹KFIEF LL¹KGG PSSGA PPPS-NH₂
YX₀QGT FTSDY SIAibLD K¹KAQAib ¹KFIEF LLEGG PSSGA PPPS-NH₂
YX₀QGT FTSDY S¹KX₁LD ¹KKAQAib AFIEF LLEGG PSSGA PPPS-NH₂,
and other variables are as defined above.

The present disclosure provides a series of polypeptides with sequences as shown below, where X₀ are all Aib, and X₁ is α-MeL:
YAibQGT FTSDY SIX₁LD K¹KAQAib ¹KFIEF LLEGG PSSGA PPPS-NH₂
YAibQGT FTSDY SIX₁LD K¹KAQAib ¹KFIEF LIEGG PSSGA PPPS-NH₂
YAibQGT FTSDY SIX₁LD K¹KAQAib ¹KFVEF LIEGG PSSGA PPPS-NH₂
YAibQGT FTSDY SIX₁LD KKAQAib AFIE¹K FI¹KGG PSSGA PPPS-NH₂
YAibQGT FTSDY SIX₁LD KKAQAib ¹KFIEF LL¹KGG PSSGA PPPS-NH₂
YAibQGT FTSDY SIAibLD K¹KAQAib ¹KFIEF LLEGG PSSGA PPPS-NH₂
YAibQGT FTSDY S¹KX₁LD ¹KKAQAib AFIEF LLEGG PSSGA PPPS-NH₂,
and other variables are as defined above.

The present disclosure provides a series of polypeptides with a sequence as shown below:
YX₀QGT FTSDY SIX₁¹KD K¹KAQX₀ AFIEY LLEGG PSSGA PPPS-NH₂
wherein,
S₀ is 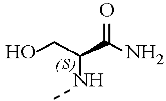 (i.e., the C-terminal amino acid is amidated to a C-terminal primary amide);
X₀ is independently selected from 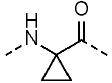 or Aib, with the structure of Aib being 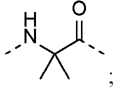
X₁ is α-methyl-substituted leucine (α-MeLeu, α-MeL) of a structure of 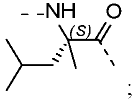
the superscript in the sequence refers to the modified amino acid unit structure, and the amino acids with the same superscript number refer to the modified amino acid unit structures which are connected by a modification to form a structure similar to a "staple". For example, ¹K and ¹K in the above sequences represent two modified lysines, and the two are connected by a modifying group (K-K modification);
the modification is formed by the condensation connection of amino or carboxyl groups on the side chains of two amino acid unit structures to 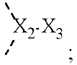
X₂ is selected from 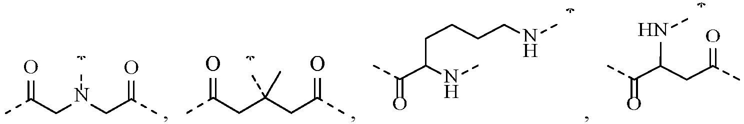 or 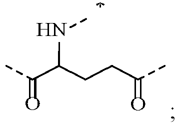 where, "*" indicates a position connected to X₃, and two ends are positions connected to the corresponding amino or carboxyl groups in the modified amino acids; X₃ is selected from 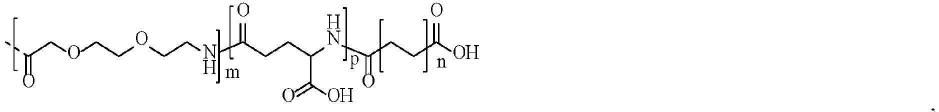 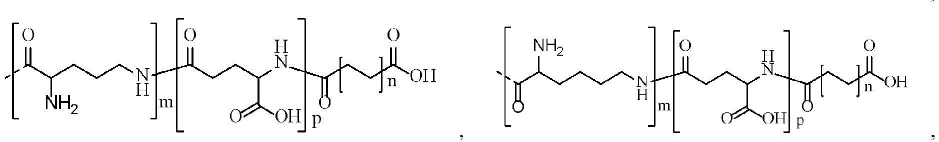 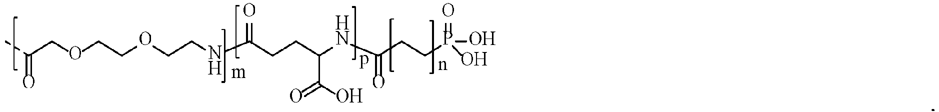 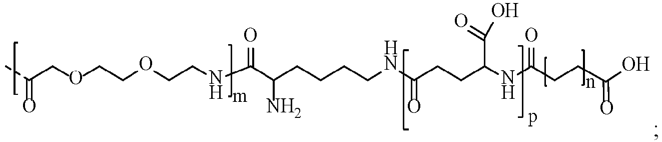
m is selected from 1, 2 or 3; p is selected from 1 or 2; and n is selected from 8, 9 or 10.

The present disclosure provides a series of polypeptides with sequences as shown below:
YX₀QGT FTSDY SIX₁LD KKAQX₀ AFIE¹K LL¹EGG PSSGA PPPS-NH₂
YX₀QGT FTSDY SIX₁LD KKAQX₀ AFI¹KW LL¹KGG PSSGA PPPS-NH₂
YX₀QGT FTSDY SIX₁LD K¹KAQX₀ ¹KFIEY LLEGG PSSGA PPPS-NH₂
HX₀HGT FTSDY SIYLE KKYAX₀ EFV¹KW LL¹KGG PSSGA PPPS-NH₂
HX₀HGT FTSDY SIYLE K¹KYAX₀ ¹KFVQW LLEGG PSSGA PPPS-NH₂
YX₀QGT FTSDY SIX₁LD KKAQX₀ AFIQ¹K LL¹KGG PSSGA PPPS-NH₂
YX₀QGT FTSDY SIX₁LD ¹KQA¹QX₀ AFIE²K LL ²KGG PSSGA PPPS-NH₂
YX₀QGT FTSDY SIX₁LD KEAQX₀AFIE¹K LL¹KGG PSSGA PPPS-NH₂
HX₀QGT FTSDY SIX₁LD KKAQX₀ AFIE¹K LL¹KGG PSSGA PPPS-NH₂
YX₀QGT FTSDY SIX₁LD KKYQX₀ AFIE¹K LL¹KGG PSSGA PPPS-NH₂
YX₀QGT FTSDY SIX₁LE KKAQX₀ AFIE¹K LL¹KGG PSSGA PPPS-NH₂
HX₀HGT FTSDY SIYLE KKYAX₀ EFVQ¹E LL¹KGG PSSGA PPPS-NH₂
YX₀QGT FTSDY SIX₁LD KKAQX₀ AFIE¹E LL¹KGG PSSGA PPPS-NH₂
YX₀QGT FTSDY SIX₁LD KKAQX₀ AFI¹EW LL¹KGG PSSGA PPPS-NH₂
YX₀QGT FTSDY SIX₁LD K¹KAQX₀ ¹KFIEF LLEGG PSSGA PPPS-NH₂
YX₀QGT FTSDY SIX₁LD K¹KAQX₀ ¹EFIEY LLEGG PSSGA PPPS-NH₂
YX₀QGT FTSDY SIX₁LD K¹KAQX₀ AFI'KY LLEGG PSSGA PPPS-NH₂
YX₀QGT FTSDY SIX₁LD K¹KAQX₀ ¹KFIEY LLEGG PSSGA PPPS-NH₂
YX₀QGT FTSDY SIX₁LD KKA¹KX₀ AFI'KY LLEGG PSSGA PPPS-NH₂
YX₀QGT FTSDY SIX₁LD KKAQX₀ ¹KFIEY LL¹KGG PSSGA PPPS-NH₂
YX₀QGT FTSDY SIX₁LD K¹KAQA ¹X₄FIEY LLEGG PSSGA PPPS-NH₂
YX₀QGT FTSDY SIX₁LD K¹KAQX₀ ¹X₄FIEY LLEGG PSSGA PPPS-NH₂
YX₀QGT FTSDY SIX₁LD K¹KAQX₀ ¹X₄FIEY LLEGG PSSGA PPPS-NH₂
wherein,
S₀ is 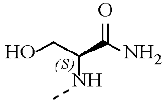 (i.e., the C-terminal amino acid is amidated to a C-terminal primary amide);
X₀ is independently selected from 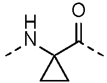 or Aib, with the structure of Aib being 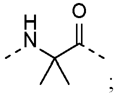
X₁ is α-methyl-substituted leucine (α-MeLeu, α-MeL) of a structure of 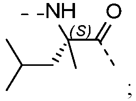
X₄ may be independently selected from α-methyl-substituted lysine (α-MeLys, α-MeK) of a structure of 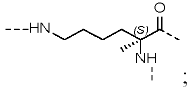 or D-lysine (d-K) of a structure of 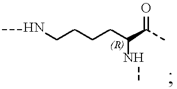 or L-omithine (L-Om) of a structure of 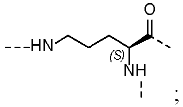
the superscript in the sequence refers to the modified amino acid unit structure, and the amino acids with the same superscript number refer to the modified amino acid unit structures connected by a modifying group to form a structure similar to a "staple". For example, ¹K and ¹E in the above sequences represent modified lysine and glutamic acid, and the two are connected by a modifying group (K-E modification); further for example, ¹K and ¹K in the above sequences represent two modified lysines, and the two are connected by a modifying group (K-K modification); and further for example, ¹K and ¹X₄ in the above sequences represent modified lysine and X₄, and the two are connected by a modifying group (K-X₄ modification);
the modification is formed by the condensation connection of amino or carboxyl groups on the side chains of two amino acid unit structures to 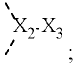
X₂ is selected from 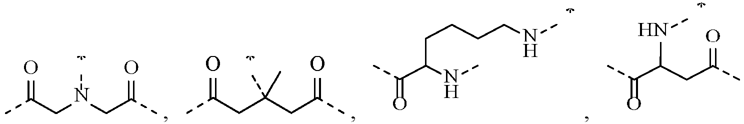 or 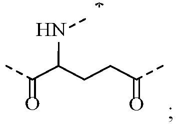 where, "*" indicates a position connected to X₃, and two ends are positions connected to the corresponding amino or carboxyl groups in the modified amino acids; X₃ is selected from 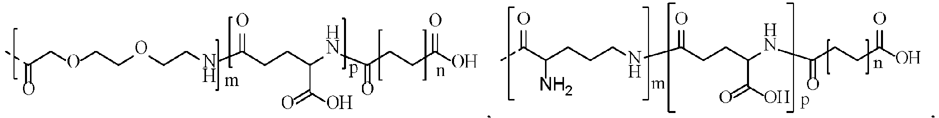 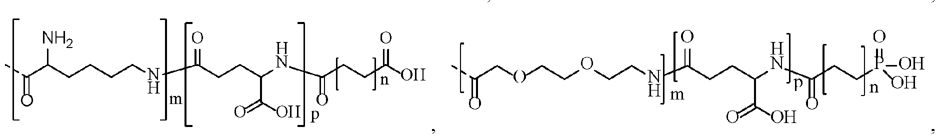 or 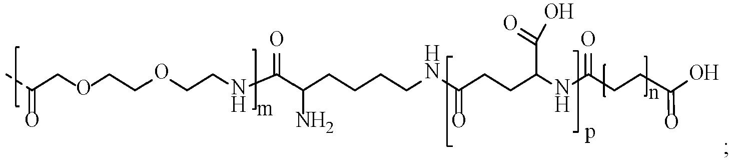
m is selected from 1, 2 or 3; p is selected from 1 or 2; and n is selected from 8, 9 or 10.

The present disclosure further provides a series of polypeptides with sequences as shown below:
YX₀QGT FTSDY SIX₁LD KKAQX₀ AFIE¹K LL¹EGG PSSGA PPPS-NH₂
YX₀QGT FTSDY SIX₁LD KKAQX₀ AFI¹KW LL¹KGG PSSGA PPPS-NH₂
YX₀QGT FTSDY SIX₁LD K¹KAQX₀ ¹KFIEY LLEGG PSSGA PPPS-NH₂
HX₀HGT FTSDY SIYLE KKYAX₀ EFV¹KW LL¹KGG PSSGA PPPS-NH₂
HX₀HGT FTSDY SIYLE K¹KYAX₀ ¹KFVQW LLEGG PSSGA PPPS-NH₂
YX₀QGT FTSDY SIX₁LD KKAQX₀ AFIQ¹K LL¹KGG PSSGA PPPS-NH₂
YX₀QGT FTSDY SIX₁LD ¹KQA¹QX₀ AFIE²K LL²KGG PSSGA PPPS-NH₂
YX₀QGT FTSDY SIX₁LD KEAQX₀AFIE¹K LL¹KGG PSSGA PPPS-NH₂
HX₀QGT FTSDY SIX₁LD KKAQX₀ AFIE¹K LL¹KGG PSSGA PPPS-NH₂
YX₀QGT FTSDY SIX₁LD KKYQX₀ AFIE¹K LL¹KGG PSSGA PPPS-NH₂
YX₀QGT FTSDY SIX₁LE KKAQX₀ AFIE¹K LL¹KGG PSSGA PPPS-NH₂
HX₀HGT FTSDY SIYLE KKYAX₀ EFVQ¹E LL¹KGG PSSGA PPPS-NH₂
YX₀QGT FTSDY SIX₁LD KKAQX₀ AFIE¹E LL¹KGG PSSGA PPPS-NH₂
YX₀QGT FTSDY SIX₁LD KKAQX₀ AFI¹EW LL¹KGG PSSGA PPPS-NH₂
wherein,
X₀ is independently selected from 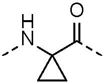 or Aib, with the structure of Aib being 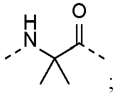
X₁ is α-methyl-substituted leucine (α-MeLeu, α-MeL) of a structure of 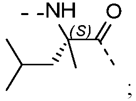
the superscript in the sequence refers to the modified amino acid unit structure, and the amino acids with the same superscript number refer to the modified amino acid unit structures connected by a modifying group to form a structure similar to a "staple". For example, ¹K and ¹E in the above sequences represent modified lysine and glutamic acid, and the two are connected by a modifying group (K-E modification); further for example, ¹K and ¹K in the above sequences represent two modified lysines, and the two are connected by a modifying group (K-K modification).
the modification is formed by the condensation connection of amino or carboxyl groups on the side chains of two amino acid unit structures to 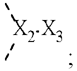
X₂ is selected from 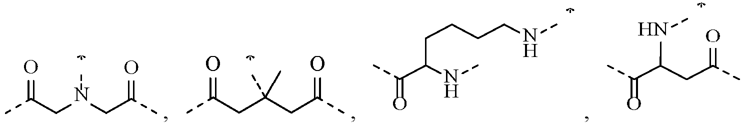 or 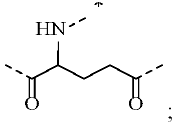 where, "*" indicates a position connected to X₃, and two ends are positions connected to the corresponding amino or carboxyl groups in the modified amino acids; X₃ is selected from 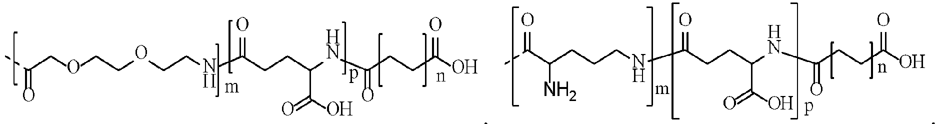 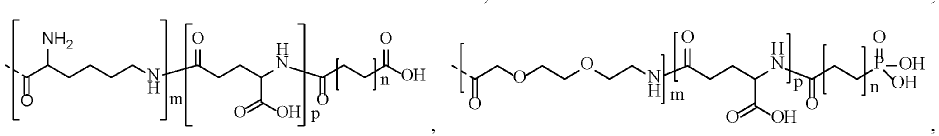 or 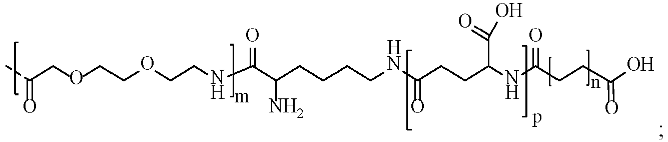
m is selected from 1, 2 or 3; p is selected from 1 or 2; and n is selected from 8, 9 or 10.

In some embodiments of the present disclosure, the polypeptide having a modified structure has a sequence of the peptide chain as shown below:
YX₀QGT FTSDY SIX₁LD KKAQAib AFIE¹K LL¹EGG PSSGA PPPS-NH₂
YX₀QGT FTSDY SIX₁LD KKAQAib AFI¹KW LL¹KGG PSSGA PPPS-NH₂
YX₀QGT FTSDY SIX₁LD K¹KAQAib ¹KFIEY LLEGG PSSGA PPPS-NH₂
HX₀HGT FTSDY SIYLE KKYAAib EFV¹KW LL¹KGG PSSGA PPPS-NH₂
HX₀HGT FTSDY SIYLE K¹KYAAib ¹KFVQW LLEGG PSSGA PPPS-NH₂
YX₀QGT FTSDY SIX₁LD KKAQAib AFIQ¹K LL¹KGG PSSGA PPPS-NH₂
YX₀QGT FTSDY SIX₁LD ¹KQA¹QAib AFIE²K LL²KGG PSSGA PPPS-NH₂
YX₀QGT FTSDY SIX₁LD KEAQAib AFIE¹K LL¹KGG PSSGA PPPS-NH₂
HX₀QGT FTSDY SIX₁LD KKAQAib AFIE¹K LL¹KGG PSSGA PPPS-NH₂
YX₀QGT FTSDY SIX₁LD KKYQAib AFIE¹K LL¹KGG PSSGA PPPS-NH₂
YX₀QGT FTSDY SIX₁LE KKAQAib AFIE¹K LL¹KGG PSSGA PPPS-NH₂
HX₀HGT FTSDY SIYLE KKYAAib EFVQ¹E LL¹KGG PSSGA PPPS-NH₂
YX₀QGT FTSDY SIX₁LD KKAQAib AFIE¹E LL¹KGG PSSGA PPPS-NH₂
YX₀QGT FTSDY SIX₁LD KKAQAib AFI¹EW LL¹KGG PSSGA PPPS-NH₂,
and other variables are as defined in the present disclosure.

In some embodiments of the present disclosure, the polypeptide having a modified structure has a sequence of the peptide chain as shown below:
YX₀QGT FTSDY SIX₁LD K¹KAQAib ¹KFIEF LLEGG PSSGA PPPS-NH₂
YX₀QGT FTSDY SIX₁LD K¹KAQAib ¹EFIEY LLEGG PSSGA PPPS-NH₂
YX₀QGT FTSDY SIX₁LD K¹KAQAib AFI'KY LLEGG PSSGA PPPS-NH₂
YX₀QGT FTSDY SIX₁LD K¹KAQAib ¹KFIEY LLEGG PSSGA PPPS-NH₂
YX₀QGT FTSDY SIX₁LD KKA¹KAib AFI'KY LLEGG PSSGA PPPS-NH₂
YX₀QGT FTSDY SIX₁LD KKAQAib ¹KFIEY LL¹KGG PSSGA PPPS-NH₂
YX₀QGT FTSDY SIX₁LD K¹KAQA ¹X₄FIEY LLEGG PSSGA PPPS-NH₂
YX₀QGT FTSDY SIX₁LD K¹KAQAib ¹X₄FIEY LLEGG PSSGA PPPS-NH₂
YX₀QGT FTSDY SIX₁LD K¹KAQAib ¹X₄FIEY LLEGG PSSGA PPPS-NH₂,
and other variables are as defined in the present disclosure.

In some embodiments of the present disclosure, the polypeptide having a modified structure has a sequence of the peptide chain as shown below:
YX₀QGT FTSDY SIX₁¹KD K¹KAQAib AFIEY LLEGG PSSGA PPPS-NH₂,
and other variables are as defined in the present disclosure.

In some embodiments of the present disclosure, the polypeptide having a modified structure has a sequence of the peptide chain as shown in any one of:
YX₀QGT FTSDY SIX₁¹KD K¹KAQAib EFIEY LLEGG PSSGA PPPS-NH₂
YX₀QGT FTSDY SIX₁¹KD K¹KAQAib AFIEY LLKGG PSSGA PPPS-NH₂,
and other variables are as defined in the present disclosure.

In some embodiments of the present disclosure, in the polypeptide having a modified structure, X₀ are all Aib, and X₁ is α-MeL, i.e., the sequence of the peptide chain is as shown in any one of:
YAibQGT FTSDY SIX₁¹KD K¹KAQAib EFIEY LLEGG PSSGA PPPS-NH₂
YAibQGT FTSDY SIX₁¹KD K¹KAQAib AFIEY LLKGG PSSGA PPPS-NH₂,
and other variables are as defined in the present disclosure.

In some embodiments of the present disclosure, in the polypeptide having a modified structure, X₀ are all Aib, and X₁ is α-MeL, i.e., the sequence of the peptide chain is as shown below:
YAibQGT FTSDY SIX₁LD KKAQAib AFIE¹K LL¹EGG PSSGA PPPS-NH₂ (SEQ ID NO:3)
YAibQGT FTSDY SIX₁LD KKAQAib AFI¹KW LL¹KGG PSSGA PPPS-NH₂ (SEQ ID NO:2)
YAibQGT FTSDY SIX₁LD K¹KAQAib ¹KFIEY LLEGG PSSGA PPPS-NH₂ (SEQ ID NO: 1)
HAibHGT FTSDY SIYLE KKYAAib EFV¹KW LL¹KGG PSSGA PPPS-NH₂ (SEQ ID NO:4)
HAibHGT FTSDY SIYLE K¹KYAAib ¹KFVQW LLEGG PSSGA PPPS-NH₂ (SEQ ID NO:5)
YAibQGT FTSDY SIX₁LD KKAQAib AFIQ¹K LL¹KGG PSSGA PPPS-NH₂ (SEQ ID NO:6)
YAibQGT FTSDY SIX₁LD ¹KQA¹QAib AFIE²K LL²KGG PSSGA PPPS-NH₂ (SEQ ID NO:7)
YAibQGT FTSDY SIX₁LD KEAQAib AFIE¹K LL¹KGG PSSGA PPPS-NH₂ (SEQ ID NO:8)
HAibQGT FTSDY SIX₁LD KKAQAib AFIE¹K LL¹KGG PSSGA PPPS-NH₂ (SEQ ID NO:9)
YAibQGT FTSDY SIX₁LD KKYQAib AFIE¹K LL¹KGG PSSGA PPPS-NH₂ (SEQ ID NO: 10)
YAibQGT FTSDY SIX₁LE KKAQAib AFIE¹K LL¹KGG PSSGA PPPS-NH₂ (SEQ ID NO: 11)
HAibHGT FTSDY SIYLE KKYAAib EFVQ¹E LL¹KGG PSSGA PPPS-NH₂ (SEQ ID NO: 12)
YAibQGT FTSDY SIX₁LD KKAQAib AFIE¹E LL¹KGG PSSGA PPPS-NH₂ (SEQ ID NO: 13)
YAibQGT FTSDY SIX₁LD KKAQAib AFI¹EW LL¹KGG PSSGA PPPS-NH₂ (SEQ ID NO: 14),
and other variables are as defined in the present disclosure.

In some embodiments of the present disclosure, in the polypeptide having a modified structure, X₀ are all Aib, the sequence of the peptide chain is as shown below:
YAibQGT FTSDY SIX₁LD K¹KAQAib ¹KFIEF LLEGG PSSGA PPPS-NH₂
YAibQGT FTSDY SIX₁LD K¹KAQAib ¹EFIEY LLEGG PSSGA PPPS-NH₂
YAibQGT FTSDY SIX₁LD K¹KAQAib AFI'KY LLEGG PSSGA PPPS-NH₂
YAibQGT FTSDY SIX₁LD K¹KAQAib ¹KFIEY LLEGG PSSGA PPPS-NH₂
YAibQGT FTSDY SIX₁LD KKA¹KAib AFI'KY LLEGG PSSGA PPPS-NH₂
YAibQGT FTSDY SIX₁LD KKAQAib ¹KFIEY LL¹KGG PSSGA PPPS-NH₂
YAibQGT FTSDY SIX₁LD K¹KAQA ¹X₄KFIEY LLEGG PSSGA PPPS-NH₂
YAibQGT FTSDY SIX₁LD K¹KAQAib ¹X₄FIEY LLEGG PSSGA PPPS-NH₂
YAibQGT FTSDY SIX₁LD K¹KAQAib ¹X₄FIEY LLEGG PSSGA PPPS-NH₂,
and other variables are as defined in the present disclosure.

In some embodiments of the present disclosure, in the polypeptide having a modified structure, X₁ is α-MeL, the sequence of the peptide chain is as shown below:
YAibQGT FTSDY SIX₁LD K¹KAQAib ¹KFIEF LLEGG PSSGA PPPS-NH₂
YAibQGT FTSDY SIX₁LD K¹KAQAib ¹EFIEY LLEGG PSSGA PPPS-NH₂
YAibQGT FTSDY SIX₁LD K¹KAQAib AFI¹KY LLEGG PSSGA PPPS-NH₂
YAibQGT FTSDY SIX₁LD K¹KAQAib ¹KFIEY LLEGG PSSGA PPPS-NH₂
YAibQGT FTSDY SIX₁LD KKA¹KAib AFI¹KY LLEGG PSSGA PPPS-NH₂
YAibQGT FTSDY SIX₁LD KKAQAib ¹KFIEY LL¹KGG PSSGA PPPS-NH₂
YAibQGT FTSDY SIX₁LD K¹KAQA ¹α-MeKFIEY LLEGG PSSGA PPPS-NH₂
YAibQGT FTSDY SIX₁LD K¹KAQAib ¹d-KFIEY LLEGG PSSGA PPPS-NH₂
YAibQGT FTSDY SIX₁LD K¹KAQAib ¹L-OrnFIEY LLEGG PSSGA PPPS-NH₂,
and other variables are as defined in the present disclosure.

In some embodiments of the present disclosure, in the polypeptide having a modified structure, X₀ are all Aib, the sequence of the peptide chain is as shown below:
YAibQGT FTSDY SIX₁ ¹KD K¹KAQAib AFIEY LLEGG PSSGA PPPS-NH₂,
and other variables are as defined in the present disclosure.

The present disclosure further provides a series of polypeptides having a modified structure or a pharmaceutically acceptable salt thereof:
YX₀QGT FTSDY SIX₁LD KKAQX₀¹KFVEF LL¹KGG PSSGA PPPSo
YX₀QGT FTSDY SIX₁LD KKAQX₀¹KFVEF LI¹KGG PSSGA PPPSo
YX₀QGT FTSDY SIX₁LD K¹KAQX₀ ¹KFIEF LLKGG PSSGA PPPSo
YX₀QGT FTSDY SIX₁LD K¹KAQX₀¹KFIKF LLEGG PSSGA PPPSo
YX₀QGT FTSDY S¹KX₁LD ¹KKAQX₀ KFIEF LLEGG PSSGA PPPS₀,
and the compound has the following modifications:
the modification is formed by the condensation connection of amino or carboxyl groups on the side chains of two amino acid unit structures to 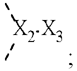
X₂ is selected from 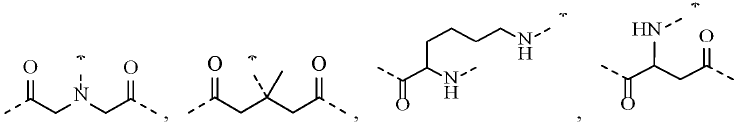 or 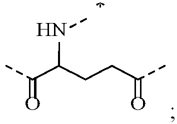 where, "*" indicates a position connected to X₃, and two ends are positions connected to the corresponding amino or carboxyl groups in the modified amino acids;
X₃ is selected from 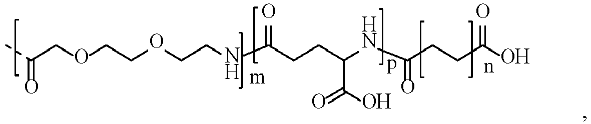 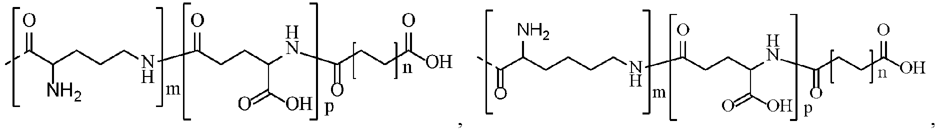 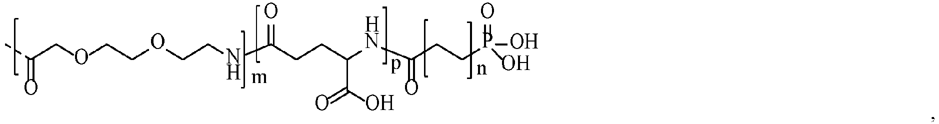 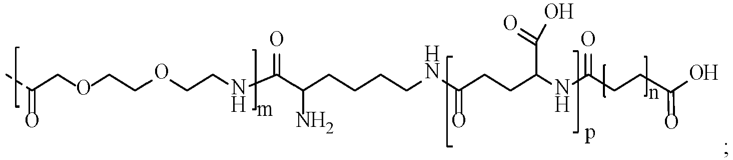
m is selected from 1, 2 or 3; p is selected from 1, 2; n is selected from 8, 9 or 10, and other variables are as defined in the present disclosure.

In some embodiments of the present disclosure, the polypeptide having a modified structure or the pharmaceutically acceptable salt thereof has a sequence of the peptide chain as shown below:
YX₀QGT FTSDY SIX₁LD KKAQX₀¹KFVEF LL¹KGG PSSGA PPPS-NH₂
YX₀QGT FTSDY SIX₁LD KKAQX₀¹KFVEF LI¹KGG PSSGA PPPS-NH₂
YX₀QGT FTSDY SIX₁LD K¹KAQX₀ ¹KFIEF LLKGG PSSGA PPPS-NH₂
YX₀QGT FTSDY SIX₁LD K¹KAQX₀ ¹KFIKF LLEGG PSSGA PPPS-NH₂
YX₀QGT FTSDY S'KX₁LD ¹KKAQX₀ KFIEF LLEGG PSSGA PPPS-NH₂,
and other variables are as defined in the present disclosure.

In some embodiments of the present disclosure, in the polypeptide having a modified structure or the pharmaceutically acceptable salt thereof, some X₀ are Aib, i.e., the sequence of the peptide chain is as shown below:
YAibQGT FTSDY SIX₁LD KKAQX₀¹KFVEF LL¹KGG PSSGA PPPS-NH₂
YAibQGT FTSDY SIX₁LD KKAQX₀¹KFVEF LI¹KGG PSSGA PPPS-NH₂
YAibQGT FTSDY SIX₁LD K¹KAQX₀ ¹KFIEF LLKGG PSSGA PPPS-NH₂
YAibQGT FTSDY SIX₁LD K¹KAQX₀ ¹KFIKF LLEGG PSSGA PPPS-NH₂
YAibQGT FTSDY S'KX₁LD ¹KKAQX₀ KFIEF LLEGG PSSGA PPPS-NH₂,
and other variables are as defined in the present disclosure.

In some embodiments of the present disclosure, in the polypeptide having a modified structure or the pharmaceutically acceptable salt thereof, X₀ are all Aib, and X₁ is α-MeL, i.e., the sequence of the peptide chain is as shown below:
YAibQGT FTSDY SIX₁LD KKAQAib ¹KFVEF LL¹KGG PSSGA PPPS-NH₂
YAibQGT FTSDY SIX₁LD KKAQAib ¹KFVEF LI¹KGG PSSGA PPPS-NH₂
YAibQGT FTSDY SIX₁LD K¹KAQAib ¹KFIEF LLKGG PSSGA PPPS-NH₂
YAibQGT FTSDY SIX₁LD K¹KAQAib ¹KFIKF LLEGG PSSGA PPPS-NH₂
YAibQGT FTSDY S'KX₁LD ¹KKAQAib KFIEF LLEGG PSSGA PPPS-NH₂,
and other variables are as defined in the present disclosure.

In some embodiments of the present disclosure, the polypeptide having a modified structure or the pharmaceutically acceptable salt thereof has a sequence of the peptide chain as shown below:
YX₀QGT FTSDY SIX₁¹KD K¹KAQX₀ AFIQY LLEGG PSSGA PPPS-NH₂,
and other variables are as defined in the present disclosure.

In some embodiments of the present disclosure, in the polypeptide having a modified structure or the pharmaceutically acceptable salt thereof, some X₀ are Aib, and the sequence of the peptide chain is as shown below:
YAibQGT FTSDY SIX₁¹KD K¹KAQX₀ AFIQY LLEGG PSSGA PPPS-NH₂,
and other variables are as defined in the present disclosure.

In some embodiments of the present disclosure, in the polypeptide having a modified structure or the pharmaceutically acceptable salt thereof, X₀ are all Aib, and X₁ is α-MeL, and the sequence of the peptide chain is as shown below:
YAibQGT FTSDY SIX₁¹KD K¹KAQAib AFIQY LLEGG PSSGA PPPS-NH₂,
and other variables are as defined in the present disclosure.

In some embodiments of the present disclosure, the m is selected from 1 or 2, and other variables are as defined in the present disclosure.

In some embodiments of the present disclosure, the p is selected from 1, and other variables are as defined in the present disclosure.

In some embodiments of the present disclosure, the n is selected from 9, and other variables are as defined in the present disclosure.

In some embodiments of the present disclosure, the X₃ is selected from 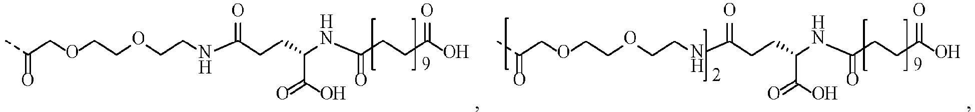 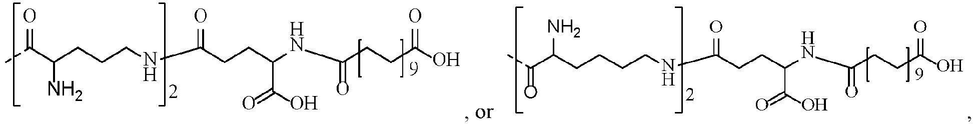 and other variables are as defined in the present disclosure.

In some embodiments of the present disclosure, the structural unit 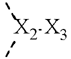 is 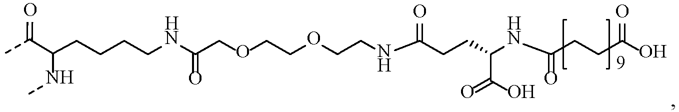 and other variables are as defined in the present disclosure.

In some embodiments of the present disclosure, the structural unit 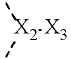 is 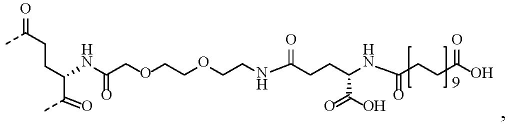 and other variables are as defined in the present disclosure.

In some embodiments of the present disclosure, the structural unit 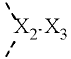 is 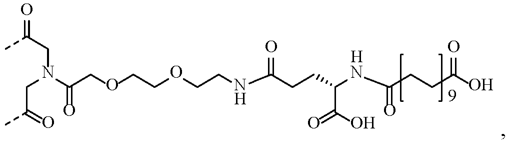 and other variables are as defined in the present disclosure.

In some embodiments of the present disclosure, the structural unit 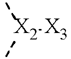 is 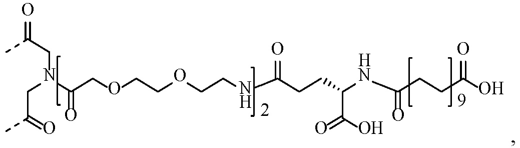 and other variables are as defined in the present disclosure.

In some embodiments of the present disclosure, the structural unit 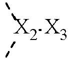 is 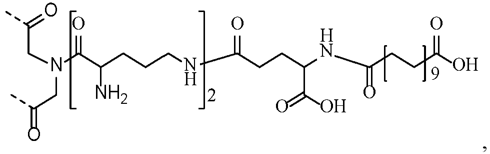 and other variables are as defined in the present disclosure.

In some embodiments of the present disclosure, the structural unit 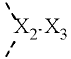 is 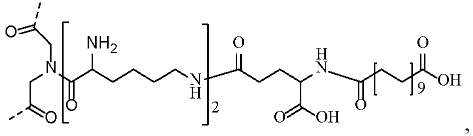 and other variables are as defined in the present disclosure.

In some embodiments of the present disclosure, the structural unit 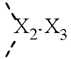 is 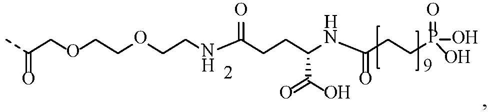 and other variables are as defined in the present disclosure.

In some embodiments of the present disclosure, the structural unit 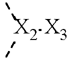 is 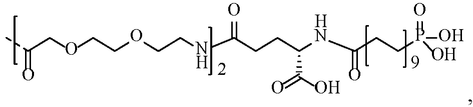 and other variables are as defined in the present disclosure.

In some embodiments of the present disclosure, the structural unit 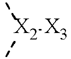 is 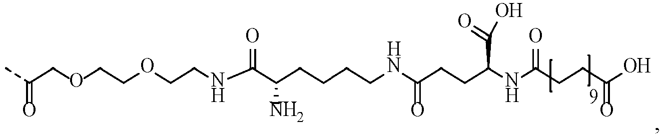 and other variables are as defined in the present disclosure.

In some embodiments of the present disclosure, the "staple" structure (stapled body) in the polypeptide having a modified structure is any one below, and other variables are as defined in the present disclosure: 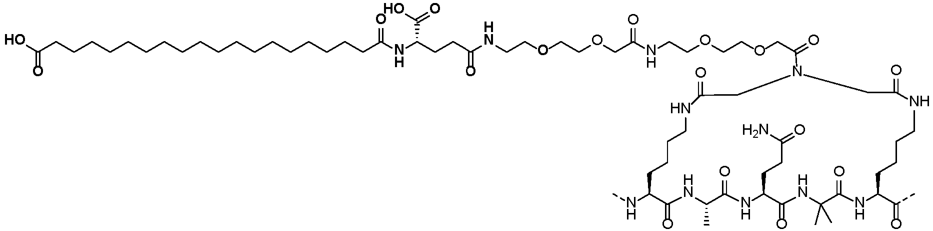 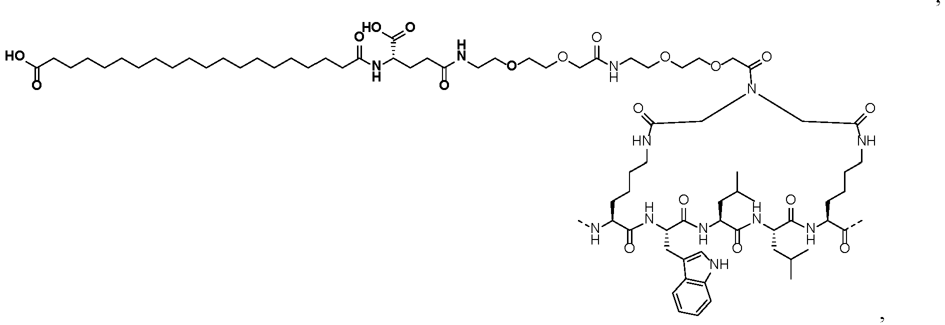 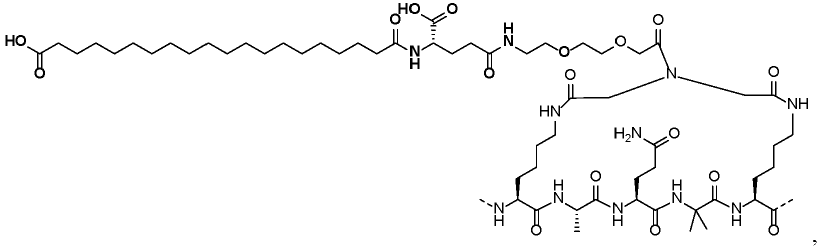 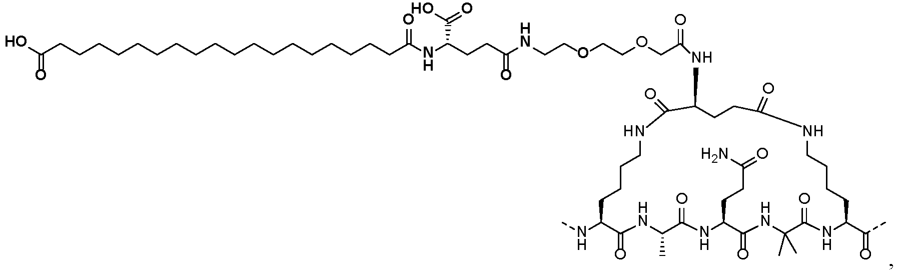 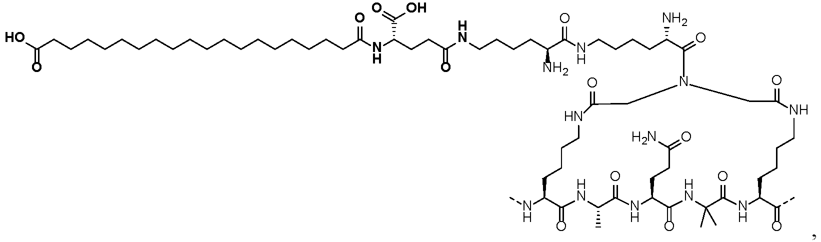 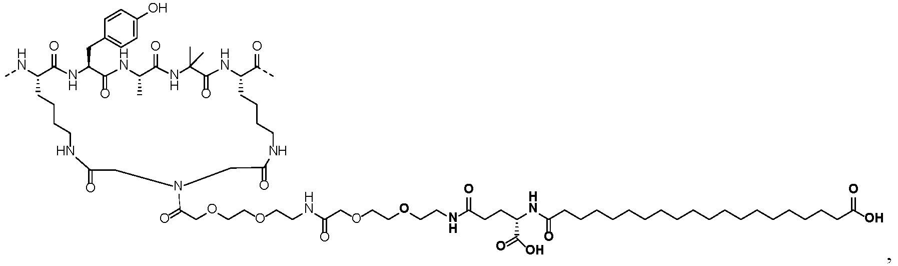 or

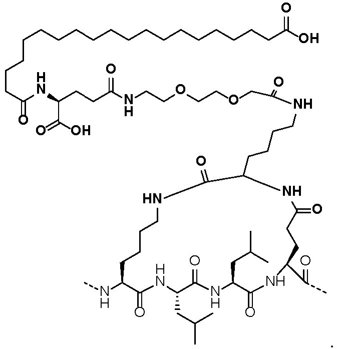

In some embodiments of the present disclosure, the "staple" structure (stapled body) in the polypeptide having a modified structure is any one below, and other variables are as defined in the present disclosure: 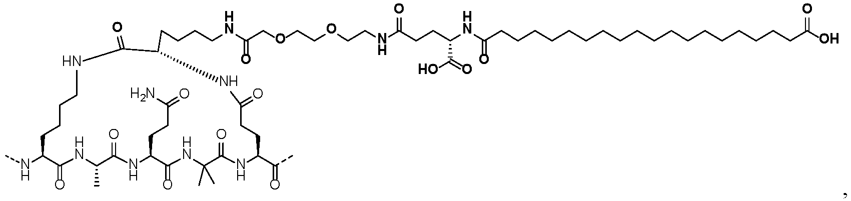 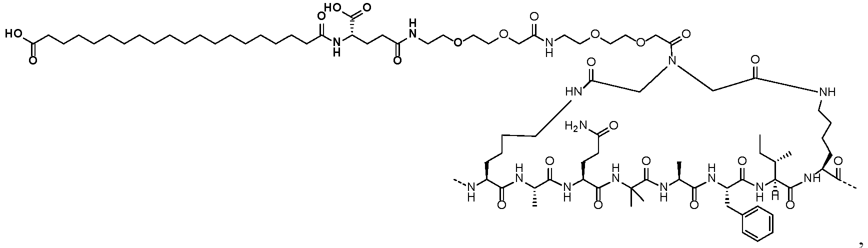 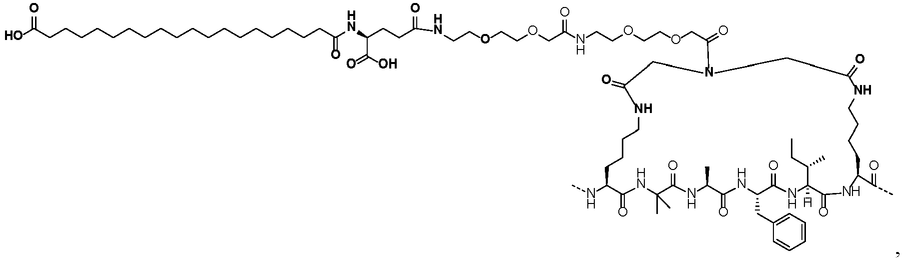 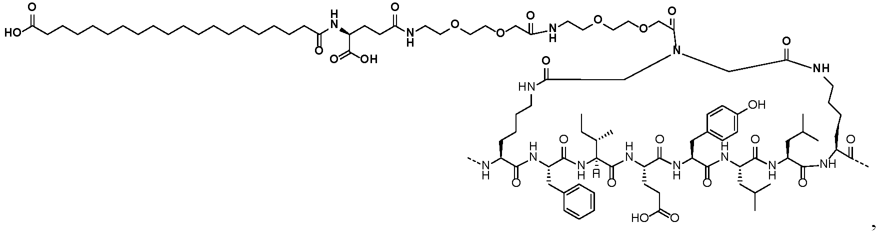 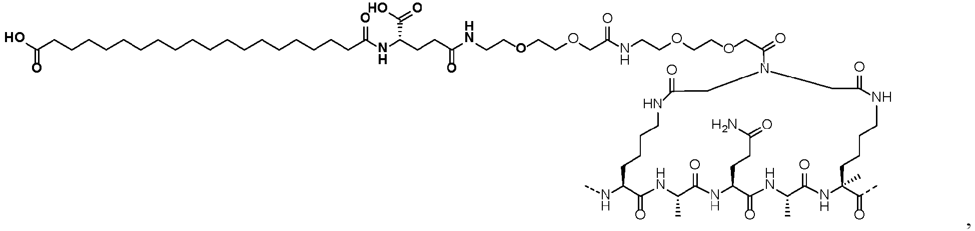 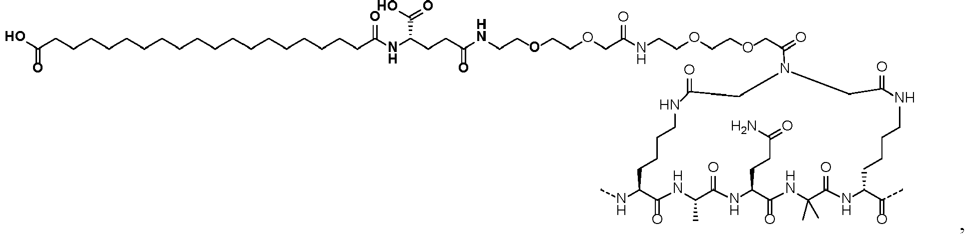 or

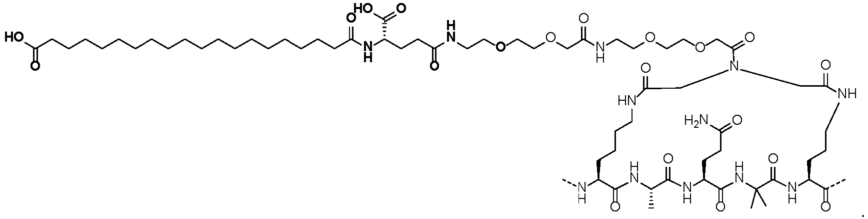

In some embodiments of the present disclosure, the "staple" structure (stapled body) in the polypeptide having a modified structure is any one below, and other variables are as defined in the present disclosure: 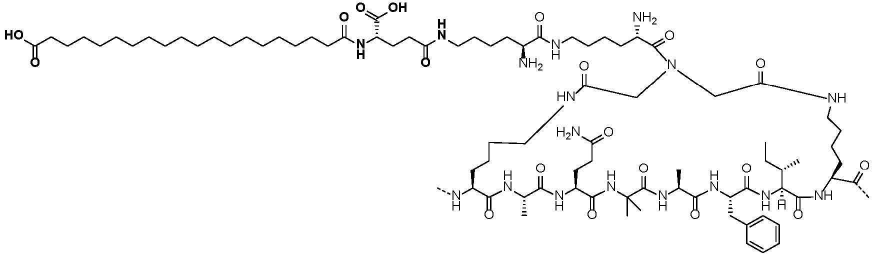 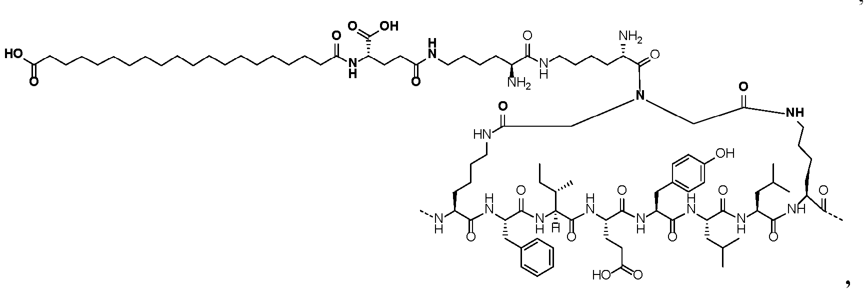 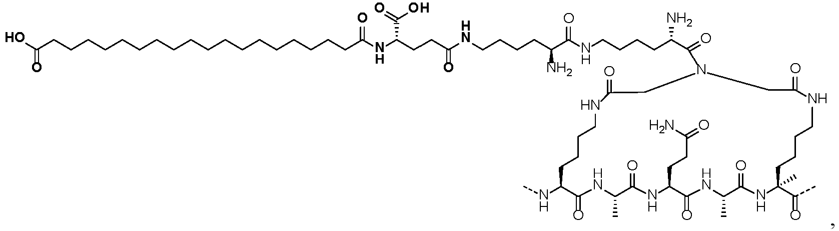 or

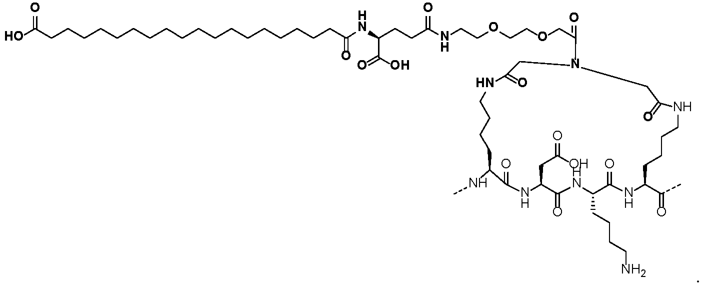

In some embodiments of the present disclosure, the "staple" structure (stapled body) in the polypeptide having a modified structure is any one below, and other variables are as defined in the present disclosure: 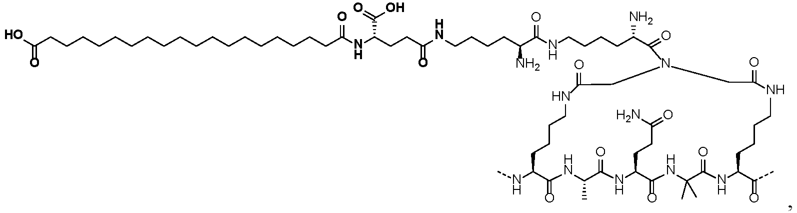 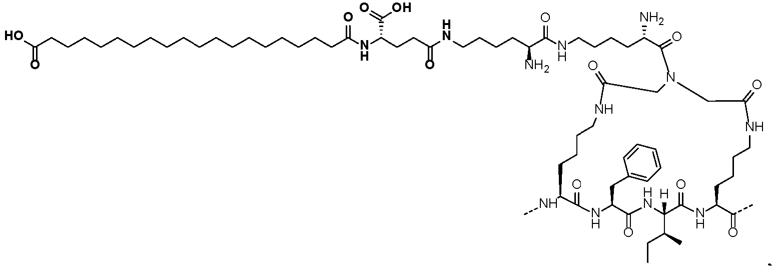 or

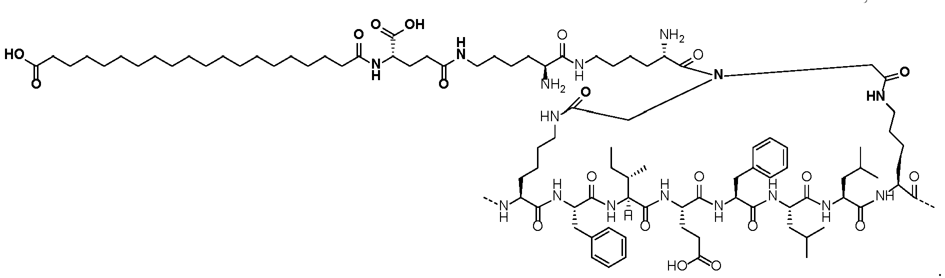

In some embodiments of the present disclosure, the "staple" structure (stapled body) in the polypeptide having a modified structure is any one below, and other variables are as defined in the present disclosure:

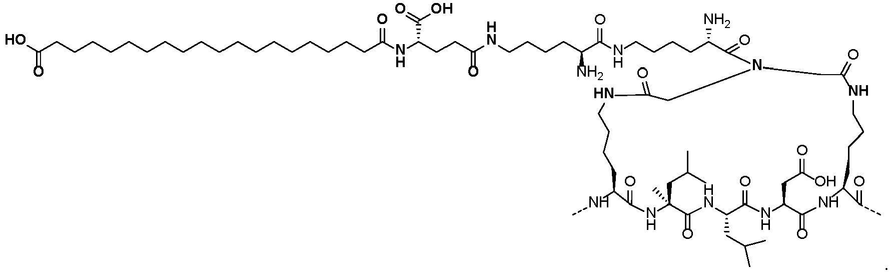

In some embodiments of the present disclosure, the "staple" structure (stapled body) in the polypeptide having a modified structure is any one below, and other variables are as defined in the present disclosure: 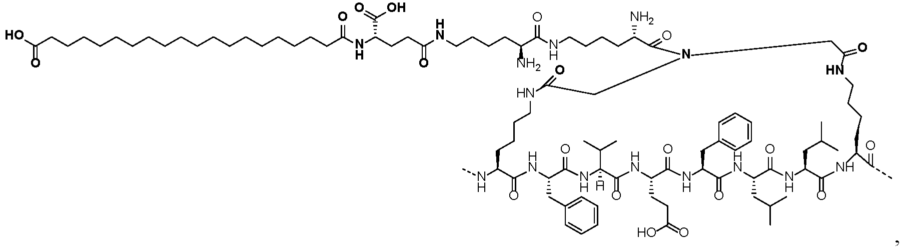 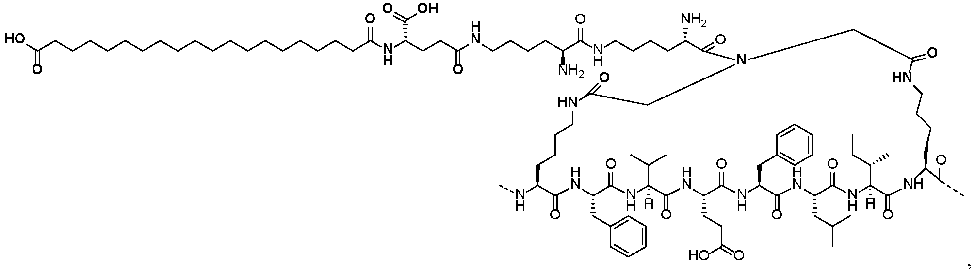 or

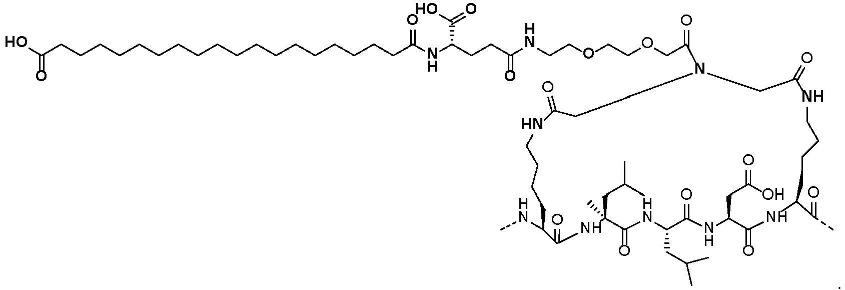

In some embodiments of the present disclosure, the "staple" structure (stapled body) in the polypeptide having a modified structure is as shown below, and other variables are as defined in the present disclosure:

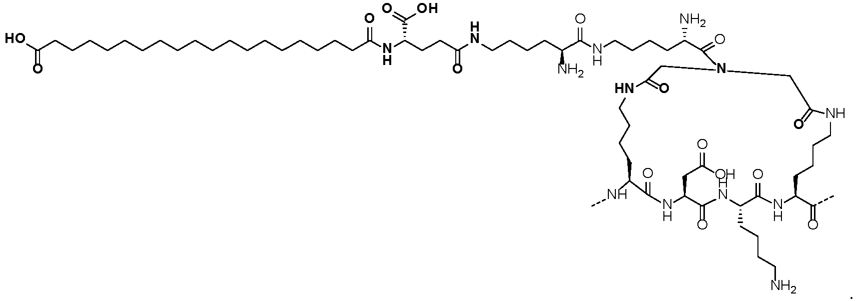

In some specific embodiments of the present disclosure, the structure of the polypeptide having a modified structure is as shown in Table 1 below, the structure of Aib is 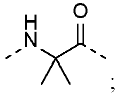 the α-MeL is α-methyl-substituted leucine of a structure of 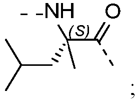 the α-MeK is α-methyl-substituted lysine of a structure of 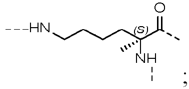 and other variables are as defined in the present disclosure. For example, the superscript in the sequence refers to the modified amino acid unit structure, and the amino acids with the same superscript number refer to the modified amino acid unit structures connected by a modifying group to form a structure similar to a "staple", and the like.

**Table 1. Polypeptide having a modified structure**

| Example/ Compound | Polypeptide sequence | Stapled body structure |
|---|---|---|
| 1 | YAibQGT FTSDY SI*α-MeL*LD K¹KAQAib ¹KFIEY LLEGG PSSGA PPPS-NH₂ (SEQ ID NO:1) i.e., i and i+j are 17 and 21, respectively | 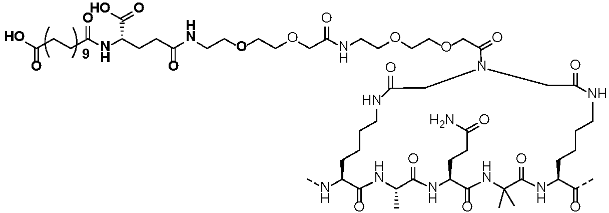 |
| 2 | YAibQGT FTSDY SI*α*-*MeL*LD KKAQAib AFI¹KW LL¹KGG PSSGA PPPS-NH₂ (SEQ ID NO:2) i.e., i and i+j are 24 and 28, respectively | 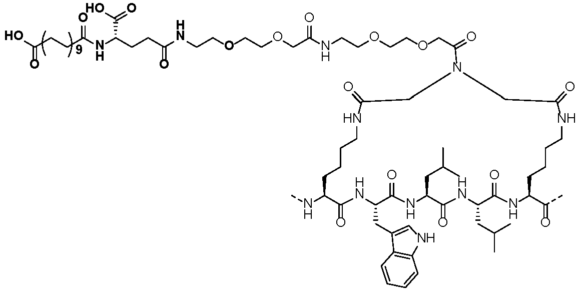 |
| 3 | YAibQGT FTSDY SI*α*-*MeL*LD KKAQAib AFIE¹K LL¹EGG PSSGA PPPS-NH₂ (SEQ ID NO:3) i.e., i and i+j are 25 and 28, respectively | 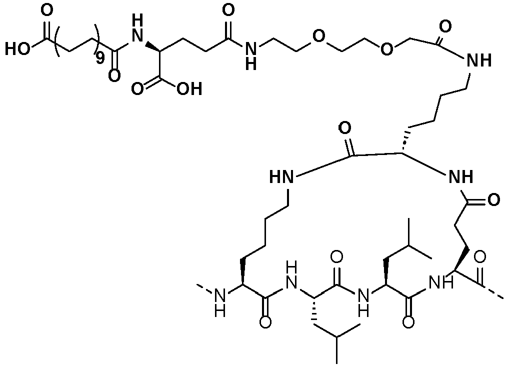 |
| 4 | HAibHGT FTSDY SIYLE KKYAAib EFV¹KW LL¹KGG PSSGA PPPS-NH₂ (SEQ ID NO:4) i.e., i and i+j are 24 and 28, respectively | 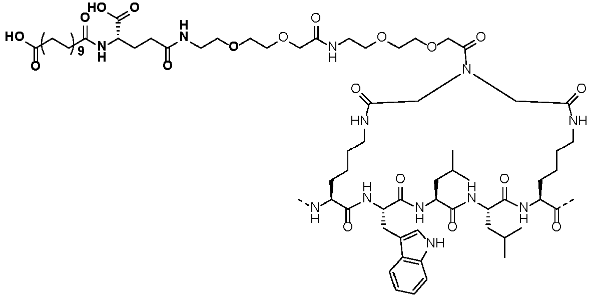 |
| 5 | HAibHGT FTSDY SIYLE K¹KYAAib ¹KFVQW LLEGG PSSGA PPPS-NH₂ (SEQ ID NO:5) i.e., i and i+j are 17 and 21, respectively | 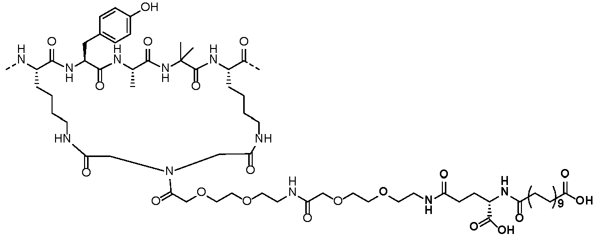 |
| 6 | YAibQGT FTSDY SI*α*-*MeL*LD K¹KAQAib ¹KFIEY LLEGG PSSGA PPPS-NH₂ (SEQ ID NO:1) i.e., i and i+j are 17 and 21, respectively | 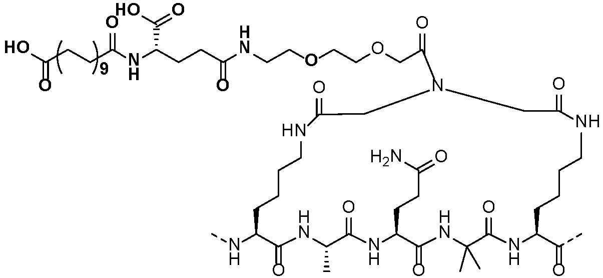 |
| 7 | YAibQGT FTSDY SI*α*-*MeL*LD K¹KAQAib ¹KFIEY LLEGG PSSGA PPPS-NH₂ (SEQ ID NO:1) i.e., i and i+j are 17 and 21, respectively | 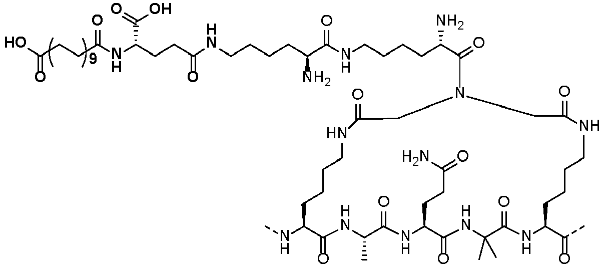 |
| 8 | YAibQGT FTSDY SI*α*-*MeL*LD K¹KAQAib ¹KFIEY LLEGG PSSGA PPPS-NH₂ (SEQ ID NO:1) i.e., i and i+j are 17 and 21, respectively | 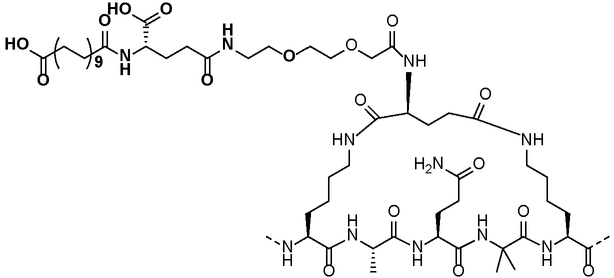 |

In some specific embodiments of the present disclosure, the structure of the polypeptide having a modified structure is as shown in Table 2 below, the structure of Aib is 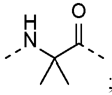 the α-MeL is α-methyl-substituted leucine of a structure of 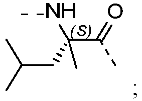 the α-MeK is α-methyl-substituted lysine of a structure of 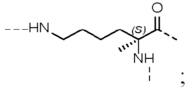 the d-K is D-lysine of a structure of 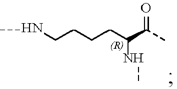 the L-Omithine or L-Om is L-omithine of a structure of 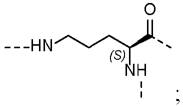 and other variables are as defined in the present disclosure, e.g., the superscript in the sequence refers to the modified amino acid unit structure, and the amino acids with the same superscript number refer to the modified amino acid unit structures connected by a modifying group to form a structure similar to a "staple" (stapled body), and the like.

**Table 2. Polypeptide having a modified structure**

| Example/ Compound | Polypeptide sequence | Stapled body formed |
|---|---|---|
| 9 | YAibQGT FTSDY SI*α*-*MeL*LD K¹KAQAib ¹KFIEF LLEGG PSSGA PPPS-NH₂ (SEQ ID NO:15) i.e., i and i+j are 17 and 21, respectively | 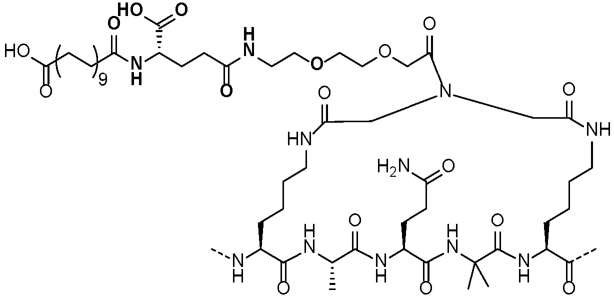 |
| 10 | YAibQGT FTSDY SI*α*-*MeL*LD K¹KAQAib ¹EFIEY LLEGG PSSGA PPPS-NH₂ (SEQ ID NO:16) i.e., i and i+j are 17 and 21, respectively | 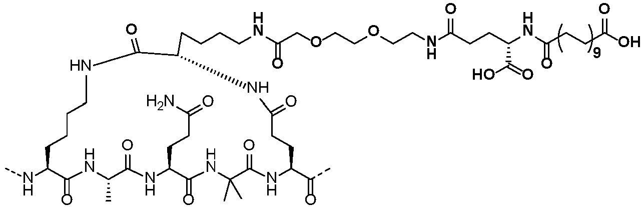 |
| 11 | YAibQGT FTSDY SI*α*-*MeL*LD K¹KAQAib AFI'KY LLEGG PSSGA PPPS-NH₂ (SEQ ID NO:17) i.e., i and i+j are 17 and 24, respectively | 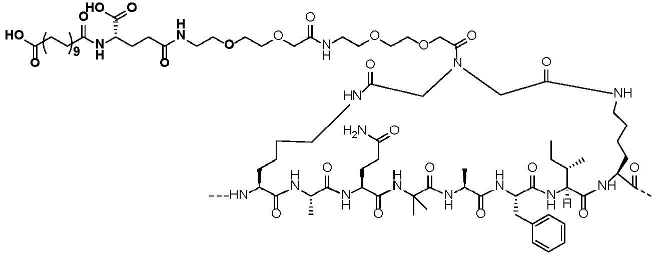 |
| 12 | YAibQGT FTSDY SI*α*-*MeL*LD K¹KAQAib ¹KFIEY LLEGG PSSGA PPPS-NH₂ (SEQ ID NO:18) i.e., i and i+j are 17 and 21, respectively | 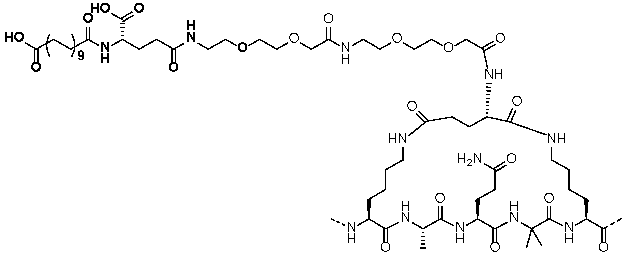 |
| 13 | YAibQGT FTSDY SI*α*-*MeL*LD KKA¹KAib AFI¹KY LLEGG PSSGA PPPS-NH₂ (SEQ ID NO:19) i.e., i and i+j are 19 and 24, respectively | 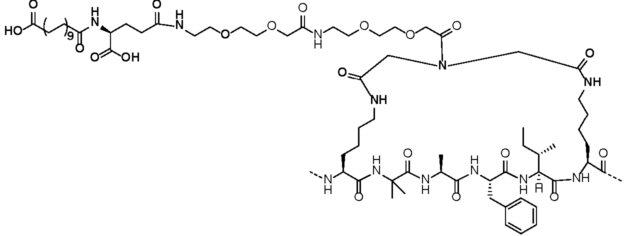 |
| 14 | YAibQGT FTSDY SI*α*-*MeL*LD KKAQAib ¹KFIEY LL¹KGG PSSGA PPPS-NH₂ (SEQ ID NO:20) i.e., i and i+j are 21 and 28, respectively | 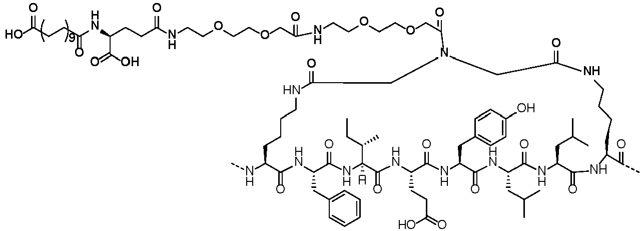 |
| 15 | YAibQGT FTSDY SI*α*-*MeL*LD K¹KAQA ¹*α-MeK*FIEY LLEGG PSSGA PPPS-NH₂ (SEQ ID NO:21) i.e., i and i+j are 17 and 21, respectively | 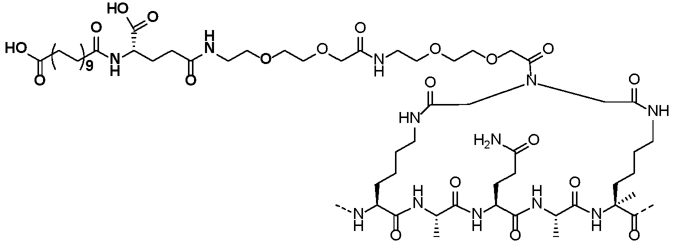 |
| 16 | YAibQGT FTSDY SI*α*-*MeL*LD K¹KAQAib *¹d-K*FIEY LLEGG PSSGA PPPS-NH₂ (SEQ ID NO:22) i.e., i and i+j are 17 and 21, respectively | 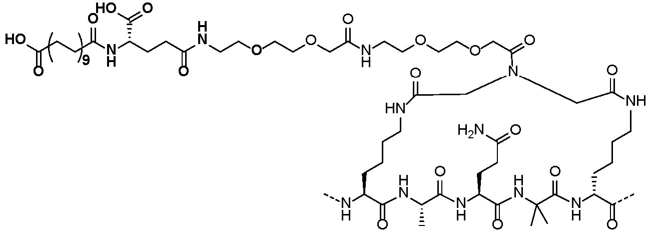 |
| 17 | YAibQGT FTSDY SI*α-MeL*LD K¹KAQAib ¹*L-Orn*FIEY LLEGG PSSGA PPPS-NH₂ (SEQ ID NO:23) i.e., i and i+j are 17 and 21, respectively | 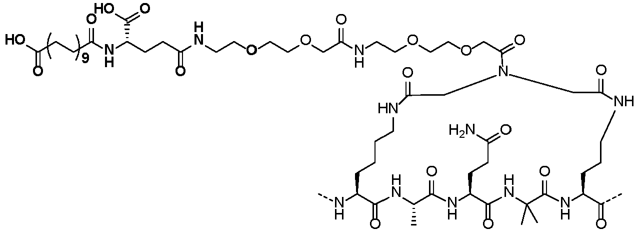 |

In some specific embodiments of the present disclosure, the structure of the polypeptide having a modified structure is as shown in Table 3 below, the structure of Aib is 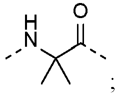 the α-MeL is α-methyl-substituted leucine of a structure of 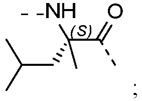 the α-MeK is α-methyl-substituted lysine of a structure of 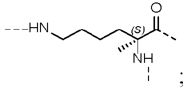 and other variables are as defined in the present disclosure. For example, the superscript in the sequence refers to the modified amino acid unit structure, and the amino acids with the same superscript number refer to the modified amino acid unit structures connected by a modifying group to form a structure similar to a "staple", and the like.

**Table 3. Polypeptide having a modified structure**

| Example/ Compound | Polypeptide sequence | Stapled body structure |
|---|---|---|
| 18 | YAibQGT FTSDY SI*α*-*MeL*LD K¹KAQAib AFI¹KY LLEGG PSSGA PPPS-NH₂ (SEQ ID NO:17) i.e., i and i+j are 17 and 24, respectively | 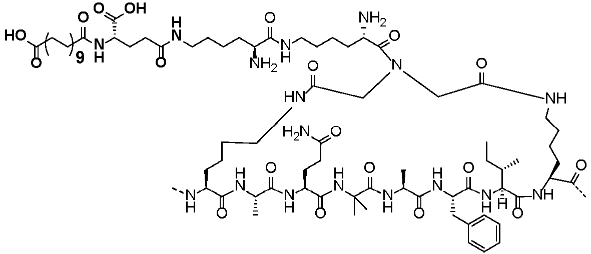 |
| 19 | YAibQGT FTSDY SI*α*-*MeL*LD KKAQAib ¹KFIEY LL¹KGG PSSGA PPPS-NH₂ (SEQ ID NO:20) i.e., i and i+j are 21 and 28, respectively | 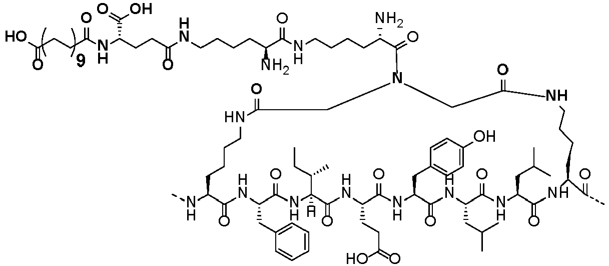 |
| 20 | YAibQGT FTSDY SI*α*-*MeL*LD K¹KAQA ¹α-*MeK*FIEY LLEGG PSSGA PPPS-NH₂ (SEQ ID NO:21) i.e., i and i+j are 17 and 21, respectively | 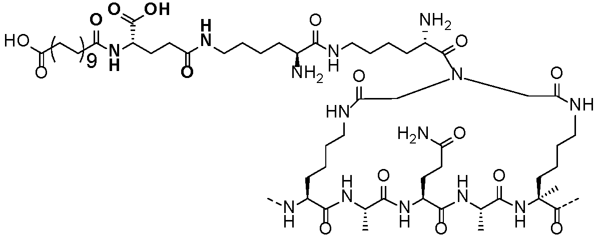 |
| 21 | YAibQGT FTSDY SI*α-MeL*¹KD K¹KAQAib AFIEY LLEGG PSSGA PPPS-NH₂ (SEQ ID NO:24) i.e., i and i+j are 14 and 17, respectively | 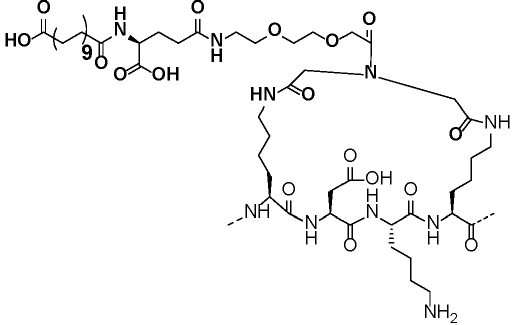 |

In some specific embodiments of the present disclosure, X₁ is α-MeL, the structure of the polypeptide having a modified structure is as shown in Table 4 below, the structure of Aib is 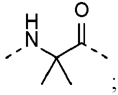 the α-MeL is α-methyl-substituted leucine of a structure of 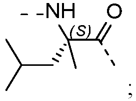 and other variables are as defined in the present disclosure. For example, the superscript in the sequence refers to the modified amino acid unit structure, and the amino acids with the same superscript number refer to the modified amino acid unit structures connected by a modifying group to form a structure similar to a "staple", and the like.

**Table 4. Polypeptide having a modified structure**

| Example/ Compound | Polypeptide sequence | Stapled body structure |
|---|---|---|
| 22 | YAibQGT FTSDY SI*α*-*MeL*LD K¹KAQAib ¹KFIEF LLEGG PSSGA PPPS-NH₂ (SEQ ID NO:25) i.e., i and i+j are 17 and 21, respectively | 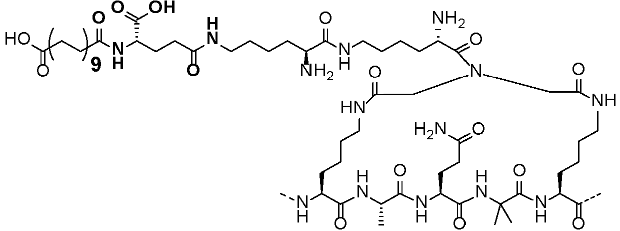 |
| 23 | YAibQGT FTSDY SI*α*-*MeL*LD K¹KAQAib ¹KFIEF LIEGG PSSGA PPPS-NH₂ (SEQ ID NO:26) i.e., i and i+j are 17 and 21, respectively | 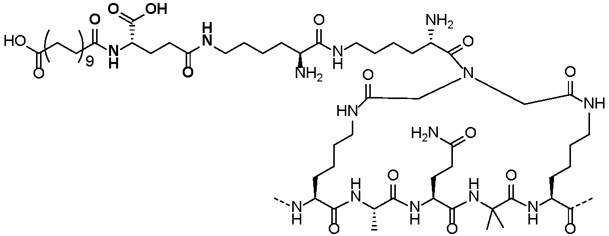 |
| 24 | YAibQGT FTSDY SI*α*-*MeL*LD K¹KAQAib ¹KFVEF LIEGG PSSGA PPPS-NH₂ (SEQ ID NO:27) i.e., i and i+j are 17 and 21, respectively | 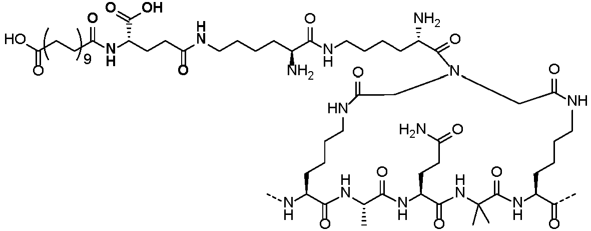 |
| 25 | YAib QGT FTSDY SI*α*-*MeL*LD KKAQAib AFIE¹K FI¹KGG PSSGA PPPS-NH₂ (SEQ ID NO:28) i.e., i and i+j are 25 and 28, respectively | 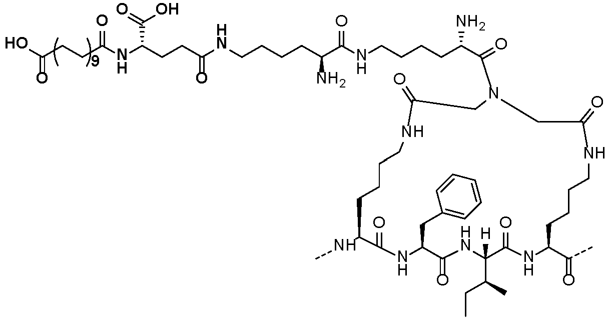 |
| 26 | YAibQGT FTSDY SI*α*-*MeL*LD KKAQAib ¹KFIEF LL¹KGG PSSGA PPPS-NH₂ (SEQ ID NO:29) i.e., i and i+j are 21 and 28, respectively | 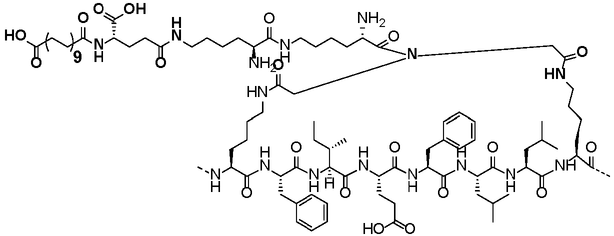 |
| 27 | YAibQGT FTSDY SIAibLD K¹KAQAib ¹KFIEF LLEGG PSSGA PPPS-NH₂ (SEQ ID NO: 30) i.e., i and i+j are 17 and 21, respectively | 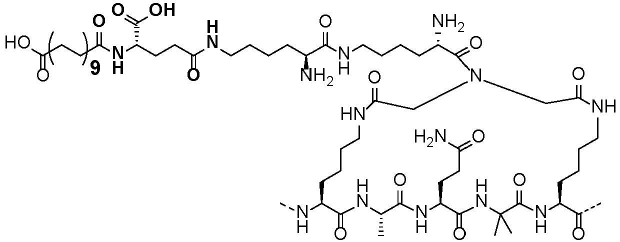 |
| 28 | YAibQGT FTSDY S¹K*α-*M*eL*LD ¹KKAQAib AFIEF LLEGG PSSGA PPPS-NH₂ (SEQ ID NO:31) i.e., i and i+j are 12 and 16, respectively | 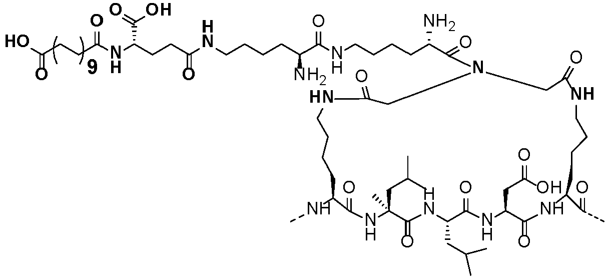 |

In some specific embodiments of the present disclosure, X₁ is α-MeL, and the structure of the polypeptide having a modified structure is as shown in Table 5 below:

**Table 5. Polypeptide having a modified structure**

| Example/ Compound | Polypeptide sequence | Stapled body structure |
|---|---|---|
| 29 | YAibQGT FTSDY SI*α-MeL*LD KKAQAib ¹KFVEF LL¹KGG PSSGA PPPS-NH₂ (SEQ ID NO:32) i.e., i and i+j are 21 and 28, respectively | 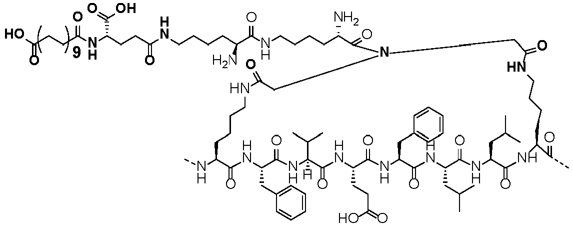 |
| 30 | YAibQGT FTSDY SI*α-MeL*LD KKAQAib ¹KFVEF LI¹KGG PSSGA PPPS-NH₂ (SEQ ID NO:33) i.e., i and i+j are 21 and 28, respectively | 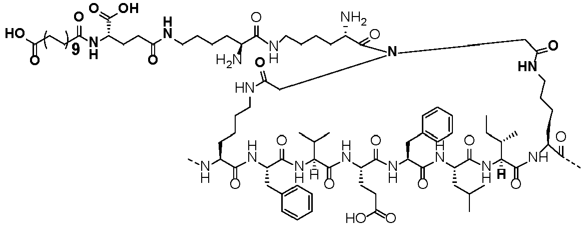 |
| 31 | YAibQGT FTSDY SI*α*-*MeL*LD K¹KAQAib ¹KFIEF LLKGG PSSGA PPPS-NH₂ (SEQ ID NO:34) i.e., i and i+j are 17 and 21, respectively | 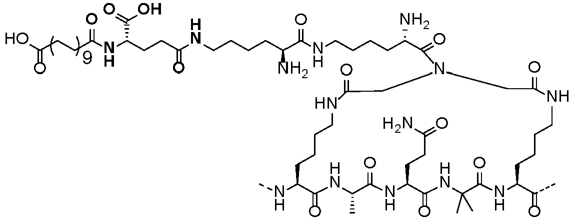 |
| 32 | YAibQGT FTSDY SI*α*-*MeL*LD K¹KAQAib ¹KFIEF LLKGG PSSGA PPPS-NH₂ (SEQ ID NO:34) i.e., i and i+j are 17 and 21, respectively | 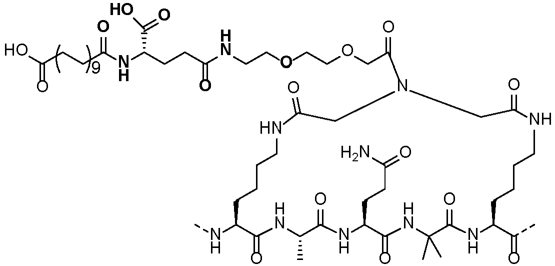 |
| 33 | YAibQGT FTSDY SI*α*-*MeL*LD K¹KAQAib ¹KFIKF LLEGG PSSGA PPPS-NH₂ (SEQ ID NO:35) i.e., i and i+j are 17 and 21, respectively | 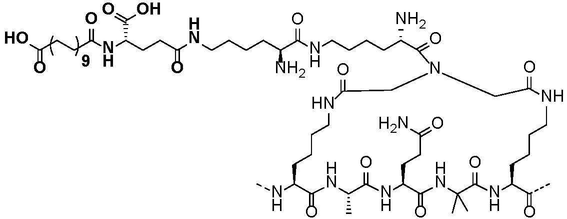 |
| 34 | YAibQGT FTSDY SI*α*-*MeL*LD K¹KAQAib ¹KFIKF LLEGG PSSGA PPPS-NH₂ (SEQ ID NO:35) i.e., i and i+j are 17 and 21, respectively | 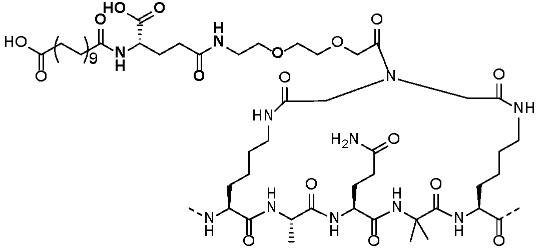 |
| 35 | YAibQGT FTSDY S¹K*α-MeL*LD ¹KKAQAib KFIEF LLEGG PSSGA PPPS-NH₂ (SEQ ID NO:36) i.e., i and i+j are 12 and 16, respectively | 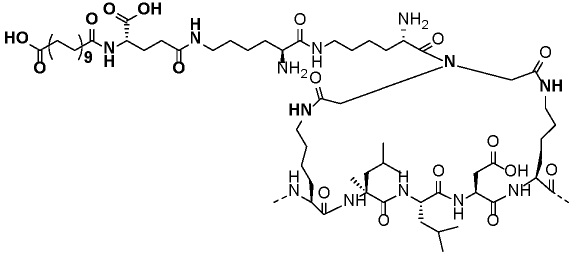 |
| 36 | YAibQGT FTSDY S¹K*α-MeL*LD ¹KKAQAib KFIEF LLEGG PSSGA PPPS-NH₂ (SEQ ID NO:36) i.e., i and i+j are 12 and 16, respectively | 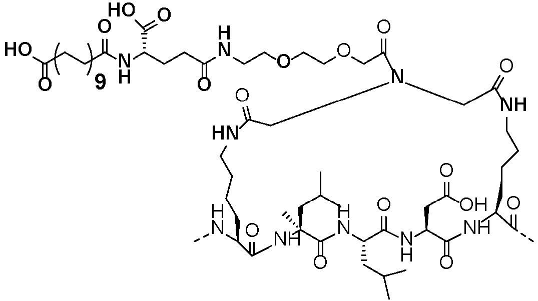 |

In some specific embodiments of the present disclosure, X₁ is α-MeL, and the structure of the polypeptide having a modified structure is as shown in Table 6 below:

**Table 6. Polypeptide having a modified structure**

| Example/ Compound | Polypeptide sequence | Stapled body structure |
|---|---|---|
| 37 | YAibQGT FTSDY SI*α-MeL*¹KD K¹KAQAib EFIEY LLEGG PSSGA PPPS-NH₂ (SEQ ID NO:37) i.e., i and i+j are 14 and 17, respectively | 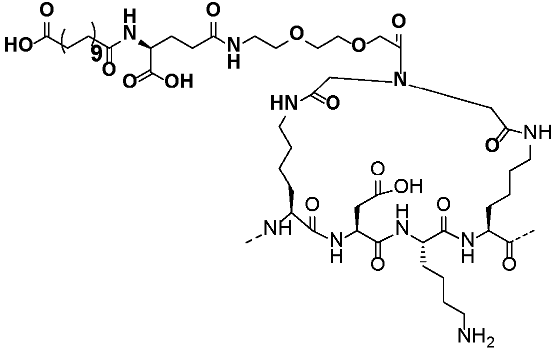 |
| 38 | YAibQGT FTSDY SI*α-MeL*¹KD K¹KAQAib AFIEY LLKGG PSSGA PPPS-NH₂ (SEQ ID NO:38) i.e., i and i+j are 14 and 17, respectively | 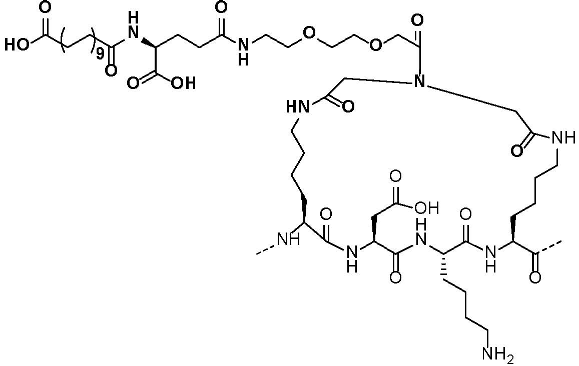 |
| 39 | YAibQGT FTSDY SI*α-MeL*¹KD K¹KAQAib AFIEY LLEGG PSSGA PPPS-NH₂ (SEQ ID NO:24) i.e., i and i+j are 14 and 17, respectively | 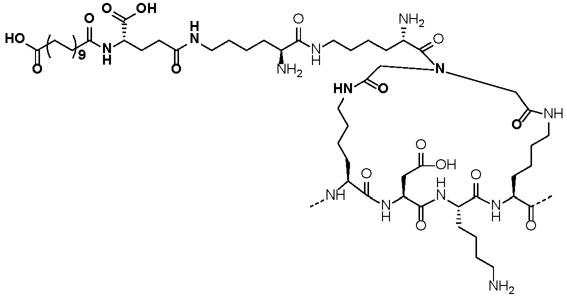 |
| 40 | YAibQGT FTSDY SI*α-MeL*¹KD K¹KAQAib EFIEY LLEGG PSSGA PPPS-NH₂ (SEQ ID NO:37) i.e., i and i+j are 14 and 17, respectively | 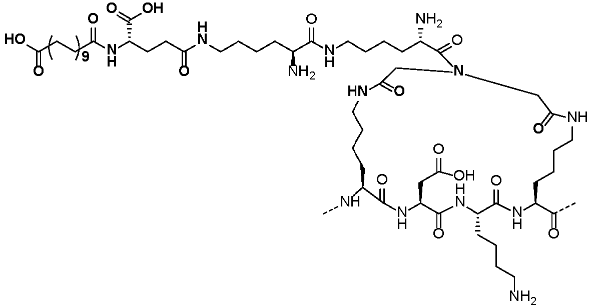 |
| 41 | YAibQGT FTSDY SI*α-MeL*¹KD K¹KAQAib AFIEY LLKGG PSSGA PPPS-NH₂ (SEQ ID NO:38) i.e., i and i+j are 14 and 17, respectively | 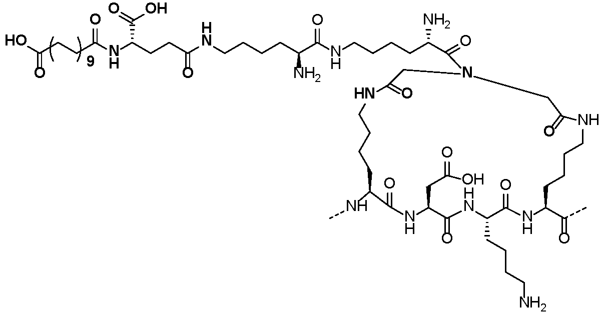 |
| 42 | YAibQGT FTSDY SI*α-MeL*¹KD K¹KAQAib AFIQY LLEGG PSSGA PPPS-NH₂ (SEQ ID NO:39) i.e., i and i+j are 14 and 17, respectively | 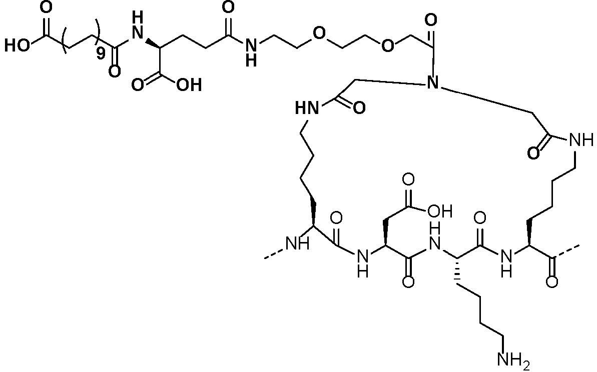 |

In some embodiments of the present disclosure, the "staple" structure (stapled body) in the polypeptide having a modified structure is as shown below, and other variables are as defined in the present disclosure: 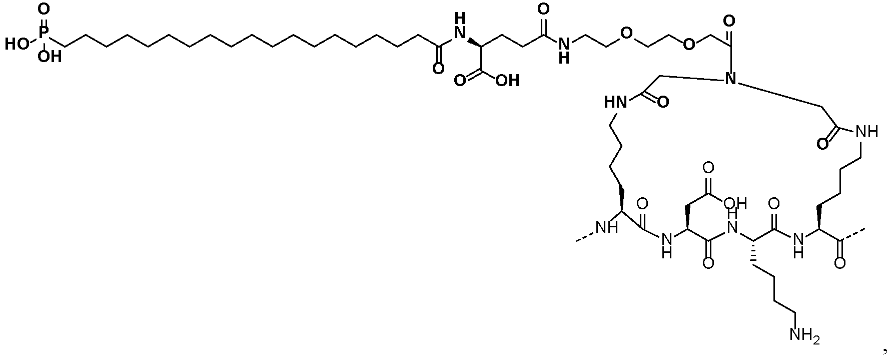 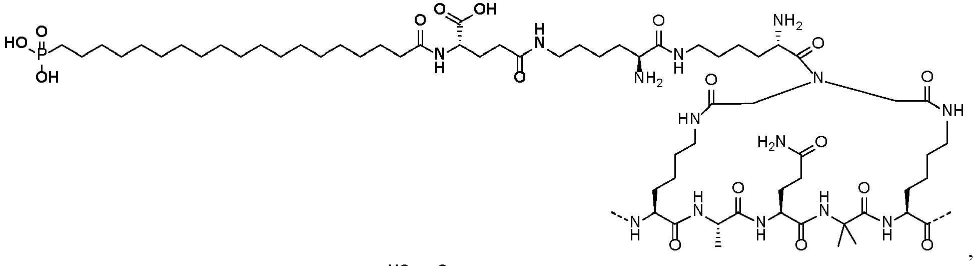 or

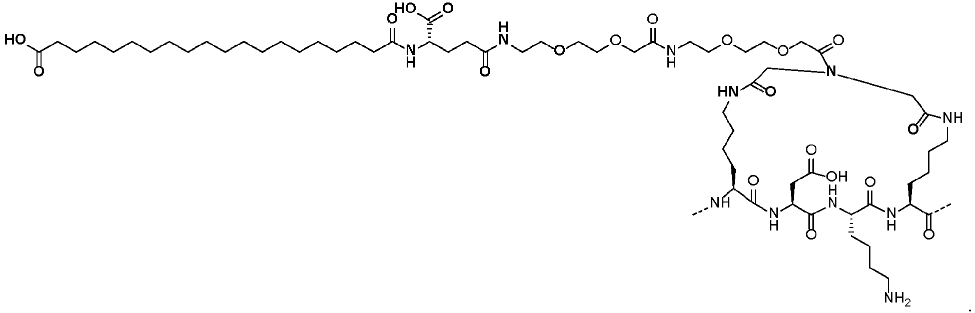

In some specific embodiments of the present disclosure, X₁ is α-MeL, and the structure of the polypeptide with a modified structure is as shown in Table 7 below:

**Table 7. Polypeptide having a modified structure**

| Example/ Compound | Polypeptide sequence | Stapled body structure |
|---|---|---|
| 43 | YAibQGT FTSDY SI*α-MeL*¹KD K¹KAQAib AFIEY LLEGG PSSGA PPPS-NH₂ (SEQ ID NO:24) | 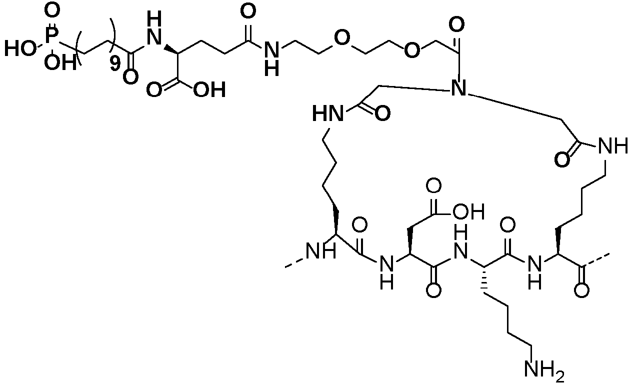 |
| | i.e., i and i+j are 14 and 17, respectively | |
| 44 | YAibQGT FTSDY SI*α*-*MeL*D K¹KAQAib ¹KFIEY LLEGG PSSGA PPPS-NH₂ (SEQ ID NO:1) | 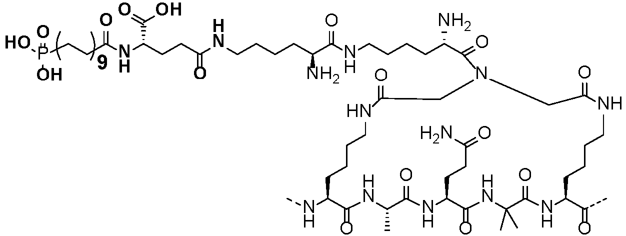 |
| | i.e., i and i+j are 17 and 21, respectively | |
| 45 | YAibQGT FTSDY SI*α-MeL*¹KD K¹KAQAib AFIEY LLEGG PSSGA PPPS-NH₂ (SEQ ID NO:24) | 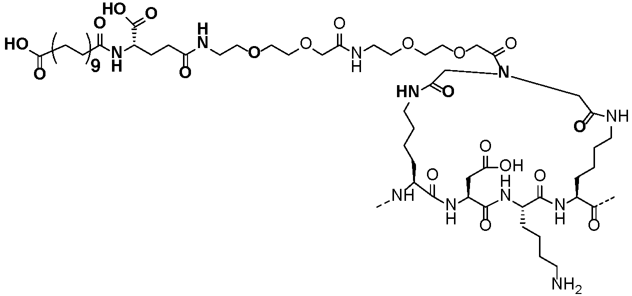 |
| | i.e., i and i+j are 14 and 17, respectively | |

In some embodiments of the present disclosure, the polypeptide having a modified structure or the pharmaceutically acceptable salt thereof has a sequence of the peptide chain as shown below:
YX₀QGT FTSDY SIX₁¹KD K¹KAQX₀ KFIEY LLEGG PSSGA PPPS-NH₂
YX₀QGT FTSDY SIX₁¹KD K¹KAQX₀ LFIEY LLEGG PSSGA PPPS-NH₂
YX₀QGT *a*-*MeF*TSDY SIX₁¹KD K¹KAQX₀ AFIEY LLEGG PSSGA PPPS-NH₂
YX₀QGT *a*-*MeF*TSDY SIX₁¹KD K¹KAQX₀ AFIEY LLKGG PSSGA PPPS-NH₂,
and other variables are as defined in the present disclosure.

In some embodiments of the present disclosure, in the polypeptide having a modified structure or the pharmaceutically acceptable salt thereof, some X₀ are Aib, and the sequence of the peptide chain is as shown below:
YAibQGT FTSDY SIX₁¹KD K¹KAQX₀ KFIEY LLEGG PSSGA PPPS-NH₂
YAibQGT FTSDY SIX₁¹KD K¹KAQX₀ LFIEY LLEGG PSSGA PPPS-NH₂
YAibQGT *a*-*MeF*TSDY SIX₁¹KD K¹KAQX₀ AFIEY LLEGG PSSGA PPPS-NH₂
YAibQGT *a*-*MeF*TSDY SIX₁¹KD K¹KAQX₀ AFIEY LLKGG PSSGA PPPS-NH₂,
and other variables are as defined in the present disclosure.

In some embodiments of the present disclosure, in the polypeptide having a modified structure or the pharmaceutically acceptable salt thereof, X₀ are all Aib, and X₁ is α-MeL, and the sequence of the peptide chain is as shown below:
YAibQGT FTSDY SIX₁¹KD K¹KAQAib KFIEY LLEGG PSSGA PPPS-NH₂
YAibQGT FTSDY SIX₁¹KD K¹KAQAib LFIEY LLEGG PSSGA PPPS-NH₂
YAibQGT *a*-*MeF*TSDY SIX₁¹KD K¹KAQAib AFIEY LLEGG PSSGA PPPS-NH₂
YAibQGT *a*-*MeF*TSDY SIX₁¹KD K¹KAQAib AFIEY LLKGG PSSGA PPPS-NH₂,
and other variables are as defined in the present disclosure.

In some embodiments of the present disclosure, the "staple" structure (stapled body) in the polypeptide having a modified structure is as shown below, and other variables are as defined in the present disclosure: 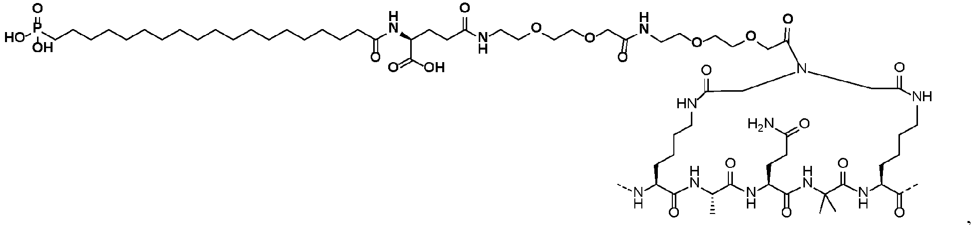 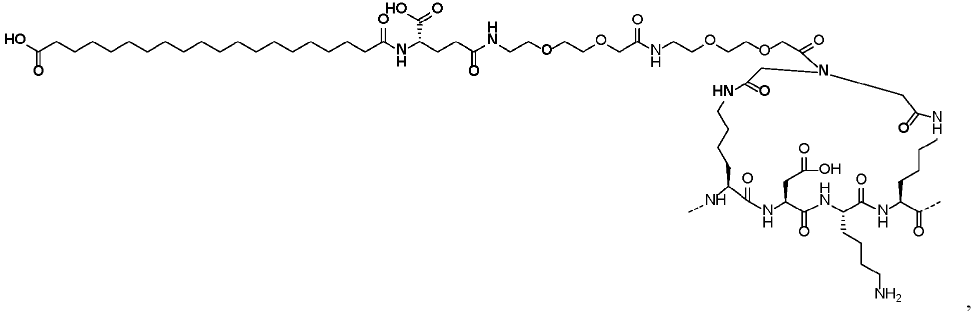 or

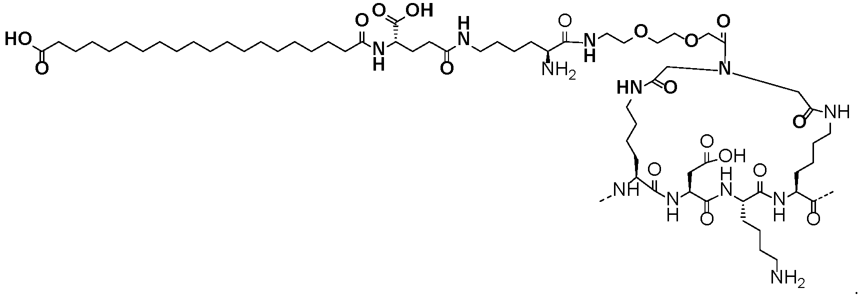

In some specific embodiments of the present disclosure, the structure of the polypeptide compound having a modified structure is as shown in Table 8 below:

**Table 8. Polypeptide having a modified structure**

| Example/ Compound | Polypeptide sequence | Stapled body structure |
|---|---|---|
| 46 | YAibQGT FTSDY SI*α-MeL*LD K¹KAQAib ¹KFIEY LLEGG PSSGA PPPS-NH₂ (SEQ ID NO:1) i.e., i and i+j are 17 and 21, respectively | 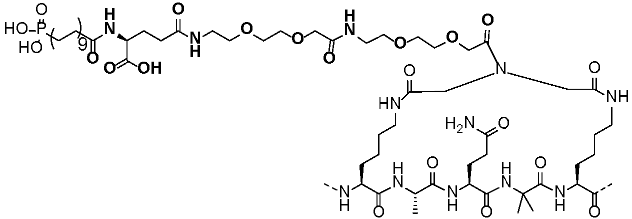 |
| 47 | YAibQGT FTSDY SI*α-MeL*¹KD K¹KAQAib KFIEY LLEGG PSSGA PPPS-NH₂ (SEQ ID NO:40) | 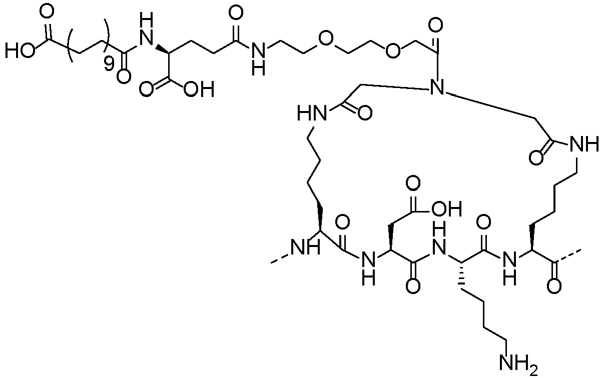 |
| | i.e., i and i+j are 14 and 17, respectively | |
| 48 | YAibQGT FTSDY SI*α-MeL*¹KD K¹KAQAib LFIEY LLEGG PSSGA PPPS-NH₂ (SEQ ID NO:41) | 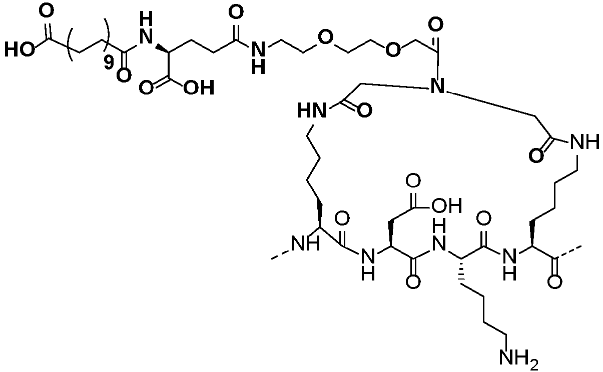 |
| | i.e., i and i+j are 14 and 17, respectively | |
| 49 | YAibQGT *a-MeF*TSDY SI*α-MeL*¹KD K¹KAQAib AFIEY LLEGG PSSGA PPPS-NH₂ (SEQ ID NO:42) | 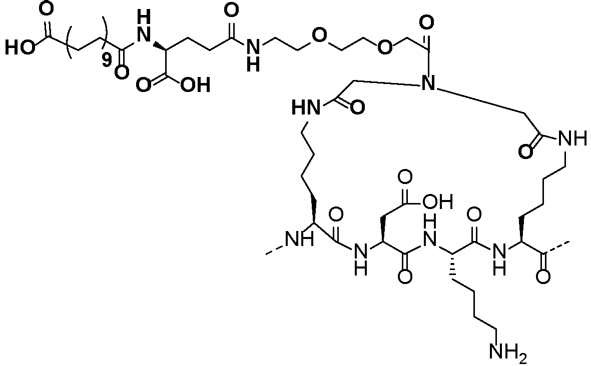 |
| | i.e., i and i+j are 14 and 17, respectively | |
| 50 | YAibQGT *a-MeF*TSDY SI*α-MeL*¹KD K¹KAQAib AFIEY LLEGG PSSGA PPPS-NH₂ (SEQ ID NO:42) | 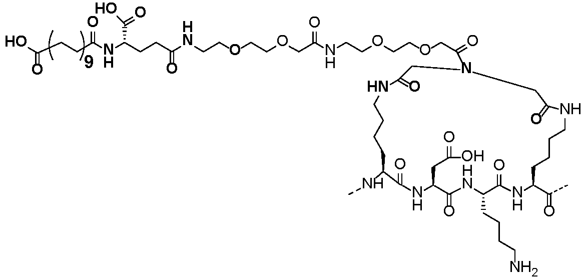 |
| | i.e., i and i+j are 14 and 17, respectively | |
| 51 | YAibQGT *a-MeF*TSDY SI*α-MeL*¹KD K¹KAQAib AFIEY LLKGG PSSGA PPPS-NH₂ (SEQ ID NO:43) | 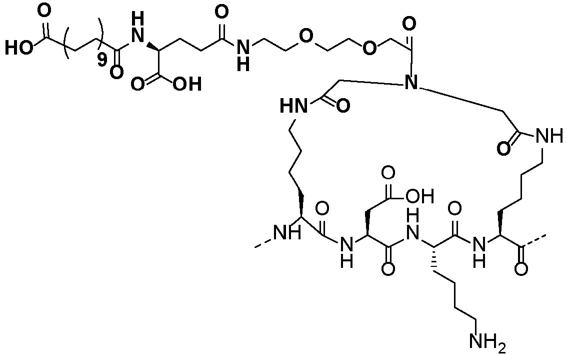 |
| | i.e., i and i+j are 14 and 17, respectively | |
| 52 | YAibQGT FTSDY SI*α-MeL*¹KD K¹KAQAib AFIEY LLEGG PSSGA PPPS-NH₂ (SEQ ID NO:24) | 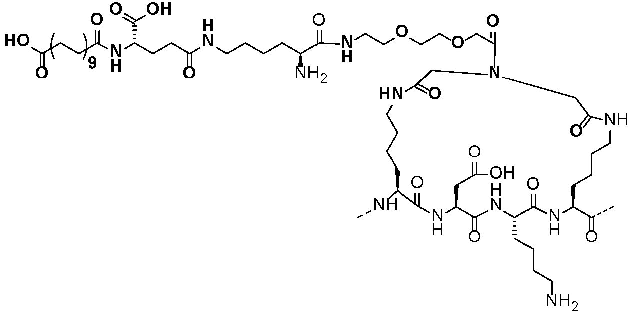 |
| | i.e., i and i+j are 14 and 17, respectively | |
| 53 | YAibQGT *a-MeF* TSDY SI*α-MeL*¹KD K¹KAQAib AFIEY LLEGG PSSGA PPPS-NH₂ (SEQ ID NO:42) | 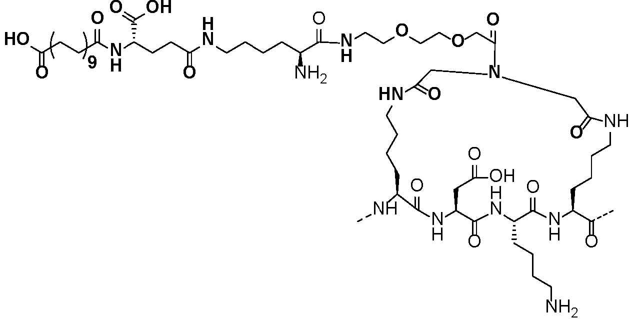 |
| | i.e., i and i+j are 14 and 17, respectively | |
| 54 | YAibQGT FTSDY SI*α-MeL*¹KD K¹KAQAib AFIEY LLKGG PSSGA PPPS-NH₂ (SEQ ID NO:38) | 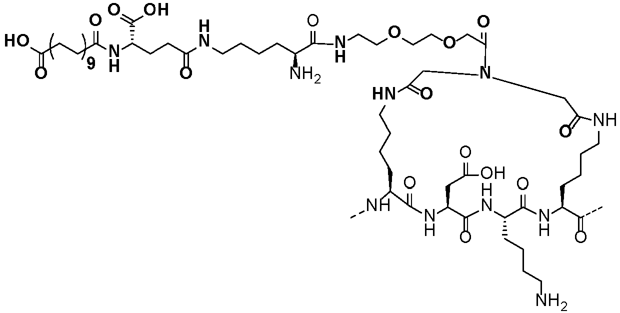 |
| | i.e., i and i+j are 14 and 17, respectively | |

The present disclosure further provides a pharmaceutical composition comprising a therapeutically effective amount of the polypeptide compound or the pharmaceutically acceptable salt thereof as an active ingredient, and a pharmaceutically acceptable carrier.

In some embodiments of the present disclosure, provided is use of the polypeptide compound or the pharmaceutically acceptable salt thereof or the composition in the preparation of a drug for preventing or treating metabolic diseases and related diseases thereof.

In some embodiments of the present disclosure, provided is use of the polypeptide compound or the pharmaceutically acceptable salt thereof or the composition in the preparation of a drug for preventing or treating diabetes (diabetes mellitus) and related diseases thereof.

In some embodiments of the present disclosure, provided is use of the polypeptide compound or the pharmaceutically acceptable salt thereof or the composition in the preparation of a drug for preventing or treating obesity and related diseases thereof.

In some embodiments of the present disclosure, provided is use of the polypeptide compound or the pharmaceutically acceptable salt thereof or the composition in the preparation of a drug for preventing or treating NASH (non-alcoholic steatohepatitis) and related diseases thereof.

### TECHNICAL EFFECTS

The polypeptides of the present disclosure show strong *in vitro* agonistic activity on GLP-1R/GIPR/GCGR, the compounds of the present disclosure exhibit excellent *in vitro* and *in vivo* pharmacokinetic properties, and the compounds of the present disclosure have significant weight reduction, hypoglycemic, and hypolipidemic effects.

### DEFINITION AND ILLUSTRATION

Unless otherwise specified, the following terms and phrases used herein are intended to have the following meanings. A particular term or phrase should not be considered indefinite or unclear in the absence of a particular definition, but should be understood according to its ordinary meaning. When a trade name is described herein, it is intended to refer to the corresponding product or its active ingredient.

The terms "polypeptide", "peptide", and "polypeptide compound" as used in the present disclosure can be understood as synonyms by persons skilled in the art by referring to the context, unless otherwise specified.

The terms "polypeptide sequence" and "peptide chain" as used in the present disclosure can be understood as synonyms by persons skilled in the art by referring to the context, unless otherwise specified.

The terms "staple" and "stapled body" as used in the present disclosure can be understood as synonyms by persons skilled in the art by referring to the context, unless otherwise specified.

Unless otherwise specified, the numbering of the example of the compounds in the present disclosure is consistent with the numbering of the compounds, i.e., the compounds indicated by "Example 1" and "Compound 1" are compounds of the same structure.

The term "pharmaceutically acceptable" as used herein refers to those compounds, materials, compositions, and/or dosage forms which are suitable for use in contact with human and animal tissues under reliable medical judgment, without undue toxicity, irritation, allergic response, or other problems or complications, and are commensurate with a reasonable benefit/risk ratio.

The term "pharmaceutically acceptable salt" refers to a salt of the compound of the present disclosure which is prepared from the compound having a specific substituent group of the present disclosure and a relatively non-toxic acid or base. When the compound of the present disclosure contains a relatively acidic functional group, a base addition salt can be obtained by contacting such a compound with a sufficient amount of base in a pure solution or a suitable inert solvent. Pharmaceutically acceptable base addition salts include sodium, potassium, calcium, ammonium, organic amine or magnesium salts, and the like. When the compound of the present disclosure contains a relatively basic functional group, an acid addition salt can be obtained by contacting such a compound with a sufficient amount of acid in a pure solution or a suitable inert solvent. Examples of pharmaceutically acceptable acid addition salts include: salts of inorganic acids including, such as, hydrochloric acid, hydrobromic acid, nitric acid, carbonic acid, bicarbonate, phosphoric acid, monohydrogen phosphate, dihydrogen phosphate, sulfuric acid, hydrosulfate, hydroiodic acid, phosphorous acid, and the like; salts of organic acids including, such as, acetic acid, trifluoroacetic acid, propionic acid, isobutyric acid, maleic acid, malonic acid, benzoic acid, succinic acid, octanedioic acid, fumaric acid, lactic acid, mandelic acid, phthalic acid, benzenesulfonic acid, p-toluenesulfonic acid, citric acid, tartaric acid, methanesulfonic acid, pamoic acid, and the like; salts of amino acids (such as arginine or the like), as well as salts of organic acids such as glucuronic acid and the like. Some specific compounds of the present disclosure contain both basic and acidic functional groups and can thus be converted to either base or acid addition salt.

The pharmaceutically acceptable salts of the present disclosure can be synthesized from parent compounds containing acid or base groups through conventional chemical methods. Generally, such salts are prepared by reacting such compounds in forms of free acid or base with a stoichiometric amount of an appropriate base or acid in water or an organic solvent or a mixture of both.

"Amino acid" refers to a naturally occurring or synthesized amino acid, as well as amino acid analogue and amino acid mimetic that functions similarly to a naturally occurring amino acid. Naturally occurring amino acids are those encoded by genetic codes, as well as those modified amino acids thereafter, such as hydroxyproline, γ-carboxyglutamic acid, and O-phosphoserine. Amino acid analogues refer to compounds having the same basic chemical structures (e.g., α-carbon bound to hydrogen, carboxyl group, amino group, and R group) as those of naturally occurring amino acids, such as homoserine, norleucine, methionine sulfoxide, and methionine methylsulfonium. Such analogues may have modified R groups (e.g., norleucine) or modified peptide backbone, but retain the same basic chemical structures as those of naturally occurring amino acids. Amino acid mimetics refer to chemical compounds that have different structures from those of conventional amino acids but perform a similar function to naturally occurring amino acids.

The natural amino acids and various expressions thereof described herein are well known to those skilled in the art, and are specifically and correspondingly as shown in the table below:

| **No.** | **Name** | **Single-letter** | **Three-letter** | **Structure** |
|---|---|---|---|---|
| 1 | Alanine | A | Ala | 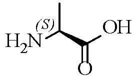 |
| 2 | Arginine | R | Arg | 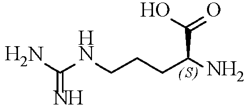 |
| 3 | Asparagine | N | Asn | 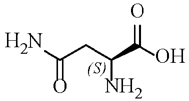 |
| 4 | Aspartic acid | D | Asp | 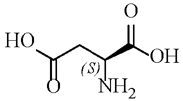 |
| 5 | Cysteine | C | Cys | 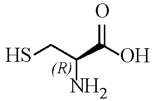 |
| 6 | Glutamine | Q | Gln | 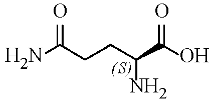 |
| 7 | Glutamic acid | E | Glu | 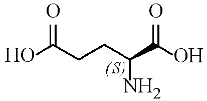 |
| 8 | Glycine | G | Gly | 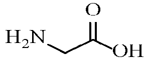 |
| 9 | Histidine | H | His | 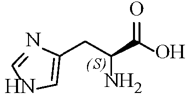 |
| 10 | Isoleucine | I | Ile | 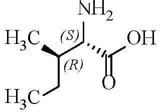 |
| 11 | Leucine | L | Leu | 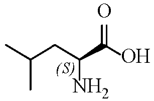 |
| 12 | Lysine | K | Lys | 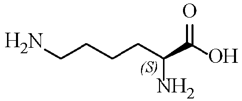 |
| 13 | Methionine | M | Met | 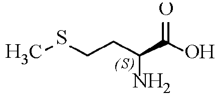 |
| 14 | Phenylalanine | F | Phe | 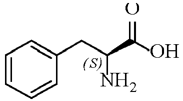 |
| 15 | Proline | P | Pro | 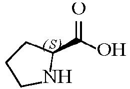 |
| 16 | Serine | S | Ser | 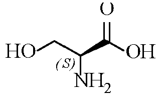 |
| 17 | Threonine | T | Thr | 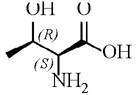 |
| 18 | Tryptophan | W | Trp | 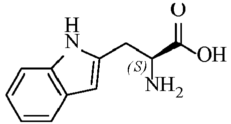 |
| 19 | Tyrosine | Y | Tyr | 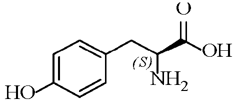 |
| 20 | Valine | V | Val | 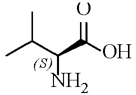 |

Some other unnatural amino acids, such as 2-amino isobutyric acid (Aib) or the like, are correspondingly as shown in the table below:

| **Name** | **Abbreviation of amino acids** | **Structure** |
|---|---|---|
| 2-methylalanine; 2-amino isobutyric acid | Aib | 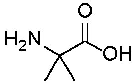 |
| 1-aminocyclopropane-carboxylic acid | | 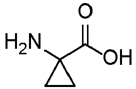 |
| α-methylleucine | α-MeLeu, α-MeL, α-Me-L | 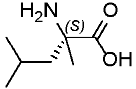 |
| α-methyllysine | α-MeLys, α-MeK, α-Me-K | 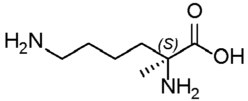 |
| L-omithine | L-Omithine, L-Orn | 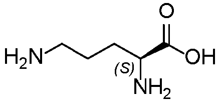 |
| D-lysine | d-K | 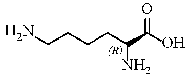 |
| α-methylphenylalanine | α-MePhe, α-MeF, α-Me-F | 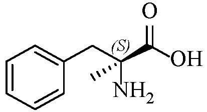 |

The term "treating" includes inhibiting, alleviating, stopping or reversing the progression or severity of an existing symptom or disease.

Unless otherwise specified, the term "isomer" is intended to include geometrical isomers, cis-trans isomers, stereoisomers, enantiomers, optical isomers, diastereomers, and tautomers.

The compounds of the present disclosure may exist in specific geometric or stereoisomeric form. The present disclosure contemplates all such compounds, including cis and trans isomers, (-)- and (+)- enantiomers, *(R)-* and (S)-enantiomers, diastereomers, (D)-isomers, (L)-isomers, and their racemic mixtures and other mixtures, such as enantiomerically or diastereomerically enriched mixtures, all of which are subject to the scope of the present disclosure. Additional asymmetric carbon atom may be present in substituent such as alkyl group. All these isomers and mixtures thereof are all included within the scope of the present disclosure.

Unless otherwise specified, the term "enantiomers" or "optical isomers" refers to stereoisomers that are mirror images of each other.

Unless otherwise specified, the term "cis-trans isomers" or "geometric isomers" refers to isomers that result from the restricted rotation around double bonds or single bonds of cyclic carbon atoms.

Unless otherwise specified, the term "diastereoisomers" refers to stereoisomers that have two or more chiral centers and exhibit non-mirror-image relationships between molecules.

Unless otherwise specified, "(+)" indicates dextrorotation, "(-)" indicates levorotation, and "(±)" indicates racemic.

Unless otherwise specified, a wedge solid line bond ( 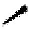 ) and a wedge dotted line bond ( 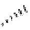 ) are used to indicate the absolute configuration of a stereocenter, a straight solid line bond ( 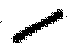 ) and a straight dotted line bond ( 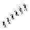 ) are used to indicate the relative configuration of a stereocenter, and a wavy line ( 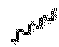 ) is used to indicate a wedge solid line bond ( 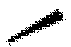 ) or a wedge dotted line bond ( 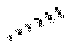 ), or a wavy line ( 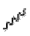 ) is used to indicate a straight solid line bond ( 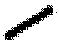 ) or a straight dotted line bond ( 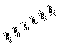 ).

Unless otherwise specified, the term "rich in one isomer", "isomerically enriched", "rich in one enantiomer" or "enantiomerrically enriched" means that the content of the one isomer or enantiomer therein is less than 100% but greater than or equal to 60%, or greater than or equal to 70%, or greater than or equal to 80%, or greater than or equal to 90%, or greater than or equal to 95%, or greater than or equal to 96%, or greater than or equal to 97%, or greater than or equal to 98%, or greater than or equal to 99%, or greater than or equal to 99.5%, or greater than or equal to 99.6%, or greater than or equal to 99.7%, or greater than or equal to 99.8%, or greater than or equal to 99.9%.

Unless otherwise specified, the term "isomerically excess" or "enantiomerically excess" refers to the difference between the relative percentages of two isomers or two enantiomers. For example, the content of one isomer or enantiomer is 90%, and the content of the other isomer or enantiomer is 10%, then the isomer or enantiomer is in excess of 80% (ee value).

Optically active (R)- and (S)-isomers as well as D and L-isomers can be prepared by chiral synthesis or using chiral reagents or other conventional techniques. To obtain one enantiomer of a compound of the present disclosure, it can be prepared through asymmetric synthesis or derivatization with a chiral auxiliary agent. The resulting diastereomeric mixture is then separated and the auxiliary group is cleaved to provide the pure desired enantiomer. Alternatively, when the molecule contains a basic functional group (such as an amino group) or an acidic functional group (such as a carboxyl group), salts of diastereomers can be formed by reacting with an appropriate optically active acid or base. The diastereomers can then be resolved through conventional methods known in the art, followed by recovery of the pure enantiomer. In addition, the separation of enantiomers and diastereomers is generally achieved by using chromatography in which chiral stationary phase is employed, optionally in combination with chemical derivatization (for example, generation of carbamate from amine).

The compounds of the present disclosure may contain unnatural proportion of atomic isotopes on one or more of the atoms that constitute the compounds. For example, the compound may be labeled with a radioactive isotope, e.g., tritium (³H), iodine-125 (¹²⁵ I) or C-14 (¹⁴C). Further for example, hydrogen may be replaced by deuterium to form deuterated drugs. The bond between deuterium and carbon is stronger than that between ordinary hydrogen and carbon, and compared with non-deuterated drugs, deuterated drugs have the advantages of reduced toxic and side effects, increased drug stability, enhanced efficacy, and extended biological half-life of drugs. All variations in isotopic composition of the compounds of the present disclosure, whether radioactive or not, are included within the scope of the present disclosure.

When the linking group listed does not indicate its linking direction, its linking direction is arbitrary. For example, the linking group L in 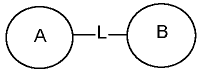 is -M-W-, and -M-W- may either link the ring A and ring B in the same direction as the reading order from left to right to form 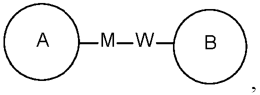 or link the ring A and ring B in the opposite direction to the reading order from left to right to form 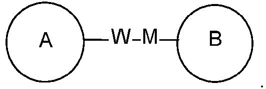 Combinations of the linking groups, substituents and/or variants thereof are permissible only if such combinations result in stable compounds.

Unless otherwise specified, when a group has one or more linkable sites, any one or more of the sites of the group may be linked to other groups through chemical bonds. **If** the connection mode of the chemical bond is not site-directed and there are hydrogen atoms at the connecting site, when the chemical bond is connected, the number of hydrogen atoms at that site will decrease correspondingly with the number of connected chemical bonds, forming a group of the corresponding valence. The chemical bonds between the site and other groups may be represented by a straight solid line bond ( 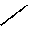 ), a straight dotted line bond ( 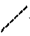 ), or a wavy line ( 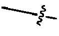 ). For example, the straight solid line bond in -OCH₃ indicates that the group is connected to other group through the oxygen atom in the group; the straight dotted line bonds in 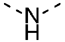 indicate that the group is connected to other groups at the two ends of the nitrogen atom in the group; and the wavy lines in 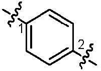 indicate that the group is connected to other groups through the carbon atoms at positions 1 and 2 of the phenyl group.

Unless otherwise specified, the term "C₁₋₃ alkyl" is used to represent a linear or branched saturated hydrocarbon group composed of 1 to 3 carbon atoms. The C₁₋₃ alkyl includes C₁₋₂ and C₂₋₃ alkyl, or the like; it may be monovalent (e.g., methyl), divalent (e.g., methylene), or multivalent (e.g., methylidyne). Examples of C₁₋₃ alkyl include, but are not limited to, methyl (Me), ethyl (Et), propyl (including n-propyl and isopropyl), etc.

The structure of the compounds of the present disclosure may be determined by conventional methods known to those skilled in the art. If the present disclosure involves the absolute configuration of the compounds, the absolute configuration may be confirmed using conventional techniques in the field. For example, single crystal X-ray diffraction (SXRD) may be used, in which a Bruker D8 venture diffractometer is used to collect diffraction intensity data of the cultured single crystal with CuKα radiation as the light source and in a scanning mode of φ/ω scanning, and after collecting relevant data, the crystal structure may be further resolved by a direct method (Shelxs97), thus confirming the absolute configuration.

The compounds of the present disclosure may be prepared by a variety of synthetic methods well known to those skilled in the art, including the specific embodiments listed below, the embodiments resulting from the combination with other chemical synthetic methods, and equivalent alternatives well-known to those skilled in the art. The preferred embodiments include, but are not limited to, the embodiments of the present disclosure.

The materials and solvents used in the present disclosure are commercially available.

The compounds are named according to conventional nomenclature in the art or using ChemDraw^{®} software, and commercially available compounds were named according to the supplier's catalogue name.

In the present disclosure, compound groups or reagents well known to those skilled in the art may be represented with abbreviations, including, but not limited to, the following compound groups: tert-butoxycarbonyl (BOC), benzyl (Bn), p-toluenesulfonyl (Tos), carbobenzyloxy (Cbz), fluorenylmethyloxycarbonyl (Fmoc), tosyl (Ts), allyloxycarbonyl (Alloc), etc.; including, but not limited to, conventional reagents: dimethyl sulfoxide (DMSO), dichloromethane (DCM), N,N-dimethylformamide (DMF), tetrahydrofuran (THF), trifluoroacetic acid (TFA), N,N'-diisopropylcarbodiimide (DIC), 1-hydroxybenzotriazole (HOBt), benzotriazole-N,N,N',N'-tetramethyl-uronium-hexafluorophosphate (HBTU), 1-hydroxy-7-azabenzotriazole (HOAT), tetrakis(triphenylphosphine)palladium (Pd(PPh₃)₄); Fmoc-AEEA-OH: 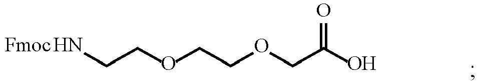 20-(tert-butoxy)-20-oxoicosanoic acid: 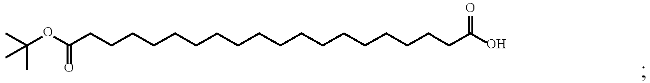 N-Boc-N'-Fmoc-Lys-OH: 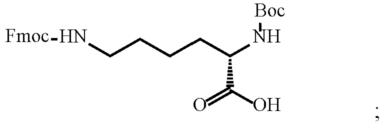 19-(bis(benzyloxy)phosphoryl)nonadecanoic acid: 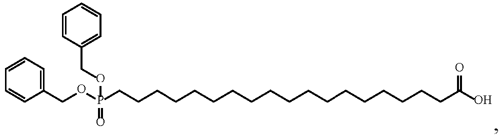 etc.

### DETAILED DESCRIPTION

The present disclosure will be described in detail by means of examples below, which are not intended to impose any adverse limitation to the present disclosure. Although the present disclosure has been described in detail herein, where specific embodiments thereof are also disclosed, it will be apparent to those skilled in the art that various changes and modifications may be made to the specific embodiments of the present disclosure without departing from the essence and scope of the present disclosure. The specific synthesis steps described in the present disclosure include new intermediates and methods for synthesizing the polypeptide compounds of the present disclosure or pharmaceutically acceptable salts thereof. According to the specific synthesis steps of the examples below, a person of ordinary skills in the art can prepare the polypeptide compounds of the present disclosure or salts thereof by combining different manners.

In general, the series of polypeptides having a modified structure of the present disclosure are prepared by using a polypeptide solid-phase synthesis method, starting from MBHA resin and employing a Fmoc-strategy, condensing protected amino acids one by one from the C-terminus to the N-terminus of the peptide sequence (including condensation, deprotection, elution cycle, and removal of side chain protection to form a lactam ring), cleavage, filtering and concentrating, precipitating, filtering, and drying, to obtain a branched cyclopeptide cleavage crude product, which is then purified, concentrated and lyophilized to obtain a finished product.

### Example 1

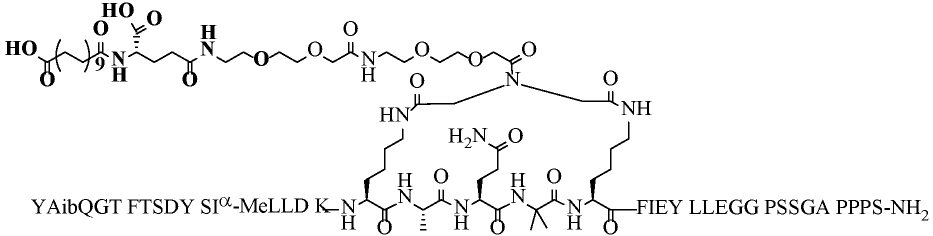

The synthesis of peptide resin was conducted by a conventional solid-phase synthesis process, using a solid-phase reaction column equipped with a sintered filter at the bottom for nitrogen bubbling during the reaction. MBHA Resin was used as the starting resin, and conventional Fmoc-protected amino acids were used as the feedings. The condensing reagent used was a system of DIC/HOBt, with DMF as the main reaction solvent, and a 20% PIP-DMF solution as the Fmoc removal solvent. The peptide backbone was synthesized from the C-terminus to the N-terminus by condensing amino acids one by one. After the backbone synthesis was completed, the ε-amino group of Fmoc-Lys(Mtt)-OH at position A-23 was deprotected by removing the -Mtt group. Then, positions 1 to 5 of side chain (including (2-2-oxoethyl)-glycine, AEEA, AEEA, y Glu, and 20-(tert-butoxy)-20-oxoicosanoic acid) were condensed sequentially. After that, the ε-amino protecting group of Lys at position A-19 and the carboxyl protecting group of position 1 of side chain ((2-2-oxoethyl)-glycine) were removed, and the condensing reagent was added to form a lactam ring, yielding the target peptide resin.

### Specific steps were as follows:

### Step 1: Coupling of resin with Fmoc-Rink Linker

1. 10.2 g (5 mmol) of MBHA Resin (S=0.49 mmol/g) was added into the solid-phase reaction column, into which was added 70 mL of DCM and left for 20 min to allow the resin to fully swell. DCM was drained, and 60 mL of DMF was added while bubbling nitrogen to wash the resin. The above steps were repeated once again, and the solvent was then drained. 10.8 g (20 mmol, 4.0 eq) of Fmoc-Rink Linker was weighed and dissolved in 40 mL of DMF, and then the mixture was added into the resin. Into the reaction column was then added 2.78 g (22 mmol, 4.4 eq) of DIC and supplemented with 10 mL of DMF while bubbling nitrogen. After complete dissolution, 2.70 g (20 mmol, 4.0 eq) of HOBt was added and nitrogen was continually bubbled for reaction.
2. After reaction at 25°C for over 2 h, a sample was taken and tested with ninhydrin as negative (with the resin being colorless or light yellow), the reaction was terminated.
3. The reaction solution was drawn off from the reaction column which was then washed with DMF (300 mL) for 5 times, each time for 1 min, and the waste was drained until no liquid flowed out.

### Step 2: Coupling of Fmoc-Ser(tBu)-OH

1. 20% of piperidine/DMF (100 mL) was added into the reaction column for reaction, while nitrogen bubbling for 10 min. The waste was drained until no liquid flowed out. 20% of piperidine/DMF (100 mL) was added into the reaction column again for further reaction, while nitrogen bubbling, for 5 min. A sample was taken and tested with ninhydrin, with the resin appearing blue.
2. 7.66 g (4.0 eq) of Fmoc-Ser(tBu)-OH, 2.78 g (4.4 eq) of DIC and 2.70 g (4.0 eq) of HOBt were weighed and dissolved in 60 mL of DMF, and then the mixture was added into the reaction column for reaction, while nitrogen bubbling, where the nitrogen was adjusted to make the resin bulge evenly.
3. After reaction at 25°C for 2.0 h, a sample was taken and tested with ninhydrin, with the resin appearing colorless or transparent.
4. The reaction solution was drawn off from the reaction column which was then washed with DMF (300 mL) for 5 times, each time for 1 min, and the waste was drained until no liquid flowed out.

### Coupling steps of the rest amino acids on the backbone

Referring to the coupling method of Fmoc-Ser(tBu)-OH and the equivalents of the feedings in Step 2, the amino acids were coupled sequentially to synthesize the amino acid sequence. The order and the protecting group-containing amino acids used are listed as follows:

| **Protecting group-containing amino acids** | **Note** |
|---|---|
| Fmoc-Pro-OH | |
| Fmoc-Pro-OH | |
| Fmoc-Pro-OH | |
| Fmoc-Ala-OH.H₂O | |
| Fmoc-Gly-OH | |
| Fmoc-Ser(tBu)-OH | |
| Fmoc-Ser(tBu)-OH | |
| Fmoc-Pro-OH | |
| Fmoc-Gly-Gly-OH | |
| Fmoc-Glu(OtBu)-OH·H₂O | |
| Fmoc-Leu-OH | |
| Fmoc-Leu-OH | |
| FmocTyr(tBu)-OH | |
| Fmoc-Glu(OtBu)-OH·H₂O | |
| Fmoc-Ile-OH | |
| Fmoc-Phe-OH | |
| Fmoc-Lys(Alloc)-OH | |
| Fmoc-Aib-OH | Deprotection monitored by HPLC |
| Fmoc-Gln(Trt)-OH | |
| Fmoc-Ala-OH.H₂O | |
| Fmoc-Lys(Mtt)-OH | |
| Fmoc-Lys(Boc)-OH | |
| Fmoc-Asp(OtBu)-OH | |
| Fmoc-Leu-OH | |
| Fmoc-α-MeLeu-OH | Deprotection monitored by HPLC |
| Fmoc-Ile-OH | |
| Fmoc-Ser(tBu)-OH | |
| Fmoc-Tyr(tBu)-OH | |
| Fmoc-Asp(OtBu)-OH | |
| Fmoc-Ser(tBu)-OH | |
| Fmoc-Thr(tBu)-OH | |
| Fmoc-Phe-OH | |
| Fmoc-Thr(tBu)-OH | |
| Fmoc-Gly-OH | |
| Fmoc-Gln(Trt)-OH | |
| Fmoc-Aib-OH | Deprotection monitored by HPLC |
| Boc-Tyr(tBu)-OH | |

### Step 3: Coupling of branched chain

1. 300 mL of DMF and 300 mL of DCM were sequentially added to the reaction column for cross-washing the resin 4 times. After draining the solvent, 300 mL of 20% hexafluoroisopropanol (HFIP)-DCM solution was added, and the reaction was performed while nitrogen bubbling for two times, each time for 30min.
2. The waste was drained until no liquid flowed out. A sample was taken and tested with ninhydrin, with the resin appearing blue.
3. 300 mL of DCM and 300 mL of DMF were sequentially added for cross-washing the resin 4 times. The waste was drained until no liquid flowed out.
4. 7.9 g (4.0 eq) of Fmoc-(2-(allyloxy)-2-oxoethyl)glycine (Fmoc-Ida (OAll)-OH), 2.78 g (4.4 eq) of DIC and 2.70 g (4.0 eq) of HOBt were weighed and dissolved in 100 mL of DMF, and then the mixture was added into the reaction column for reaction, while nitrogen bubbling, where the nitrogen was adjusted to make the resin bulge evenly.
5. After reaction at 25°C for 2.0 h, a sample was taken and tested with ninhydrin, with the resin appearing colorless or transparent.
6. The reaction solution was drawn off from the reaction column which was then washed with DMF (300 mL) for 5 times, each time for 1 min, and the waste was drained until no liquid flowed out.

### The rest steps of coupling branched chain

Referring to the coupling method of Fmoc-Ser(tBu)-OH and the equivalents of the feedings in Step 2, the compounds listed in the table below were coupled sequentially:

| **Protecting group-containing amino acids** | **Note** |
|---|---|
| Fmoc-AEEA-OH | |
| Fmoc-AEEA-OH | |
| Fmoc-Glu-OtBu | |
| 20-(tert-butoxy)-20-oxoicosanoic acid | |

### Step 4: Cyclization

1. 300 mL of DMF and 300 mL of DCM were sequentially added into the reaction column for cross-washing the resin 4 times. The waste was drained until no liquid flowed out.
2. 400 ml of a mixed solvent of DCM: CH₃CN: NMP: DEA at a volume ratio of 3:3:2:2 was added, and 0.58 g (0.1 eq) of Pd(PPh₃)₄ was then added, and the reaction was performed while nitrogen bubbling for 10 min. The waste was drained until no liquid flowed out.
3. The above steps were repeated 1 to 2 times.
4. A sample was taken and tested with ninhydrin, with the resin appearing blue.
5. 300 mL of DMF and 300 mL of DCM were sequentially added for cross-washing the resin 8 times.
6. 2.78 g (4.4 eq) of DIC and 2.70 g (4.0 eq) of HOBt were dissolved in 150 mL of DMF, and the mixture was added into the reaction column, where the reaction was performed while nitrogen bubbling for 48 h. A sample was taken and tested with ninhydrin, with the resin appearing colorless.
7. 300 mL of DMF and 300 mL of DCM were sequentially added for cross-washing the resin 8 times.
8. 200 mL of MeOH and 300 mL of DCM were sequentially added for cross-washing the resin 4 times.
9. The resin was taken out and dried in vacuum until the weight no longer decreased, and weighed, obtaining 17.8 g of peptide resin.

The obtained intermediate (peptide resin) has a structure formula as below:
Boc-Tyr(tBu)-Aib-Gln(Trt)-Gly-Thr(tBu)-Phe-Thr(tBu)-Ser(tBu)-Asp(OtBu)-Tyr(tBu)-Ser(tBu)-Ile-a-MeL eu-Leu-Asp(OtBu)-Lys(Boc)-Lys¹⁷-Ala-Gln(Trt)-Alb-Lys²¹-Phe-Ile-Glu(OtBu)-Tyr(tBu)-Leu-Leu-Glu(OtBu)-G ly-Gly-Pro-Ser(tBu)-Ser(tBu)-Gly-Ala-Pro-Pro-Pro-Ser(tBu)-Rink Linker -MBHA Resin (17,21(Nε(Na-OC-(CH₂)₁₈-COOtBu) -Glu(ε-OtBu)-AEEA-AEEA) -(2-2-oxoethyl)glycine))

### Step 5. Cleavage of peptide resin

250 mL of a cleavage reagent was formulated with trifluoroacetic acid (TFA), purified water (H₂O), triisopropylsilane (TIS), 1, 2-ethanedithiol (EDT), and phenol (ArOH) at a volume ratio of TFA: H₂O: EDT: TIS: ArOH = 86.5:5:2.5:1:5, and then pre-cooled to 10±3°C. The resin was slowly added into the cleavage reagent while stirring until the temperature of the reaction system was stable, and the reaction proceeded with stirring for 4 h while controlling the temperature at 18±3°C. The cleavage solution was filtered, concentrated to thick, and precipitated with a 12-15-fold concentrate volume of methyl tert-butyl ether. The precipitate was filtered, washed, and then dried at room temperature under reduced pressure to obtain 7.9 g of crude cyclopeptide.

### Step 6. Purification by chromatography

7.9 g of the crude cyclopeptide was dissolved in 10% acetonitrile and 10% aqueous acetic acid and purified by high-performance liquid chromatography using C18 silica gel matrix filler, and the fractions with a purity of >90% were collected and combined. The purification chromatography systems are listed as follows:

| Procedures | Aqueous phase | Organic phase | Gradient |
|---|---|---|---|
| First purification | 0.2% TFA | Acetonitrile | B% (0-70 min): 41%-51% |
| Second purification | 35 mmol/L K₂HPO₄ (pH 7.4) | Acetonitrile | B% (0-50 min): 33%-44% |
| Salt Conversion | 0.6 mmol/L NaOH | Acetonitrile | B%: 45% |

### Step 7. Concentration and lyophilization

The combined fraction obtained from the above step was concentrated to remove acetonitrile, and filtered through a 0.22 µm millipore filter membrane. The filtered solution was loaded into a vial lyophilizer and lyophilized to obtain 0.93 g of target polypeptide product (with a purity of 94.97%). The molecular weight of the polypeptide was confirmed by ESI-MS, with the [M+4H]/4 calculated value of 1258.7 and the detected value of 1258.7.

### Example 2

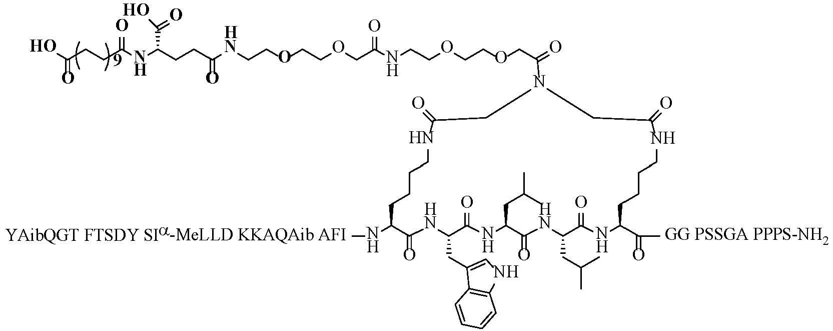

Referring to the synthesis of polypeptide in Example 1, the polypeptide as shown in Example 2 was obtained. The molecular weight of the polypeptide was confirmed by ESI-MS, with the [M+4H]/4 calculated value of 1249.7 and the detected value of 1249.4.

### Example 3

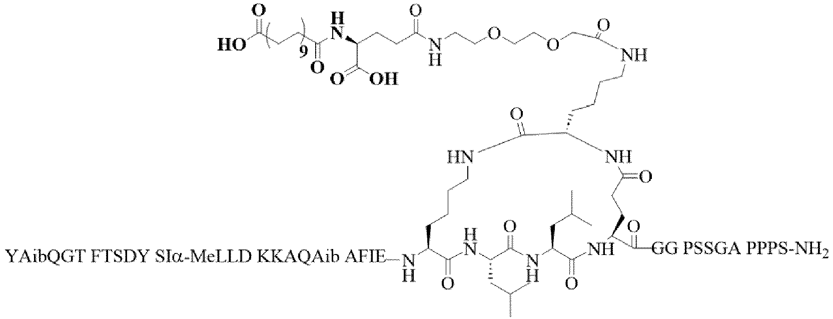

### Step 1: Coupling of resin with Fmoc-Rink Linker

1. 10.2 g (5 mmol) of MBHA Resin (S=0.49 mmol/g) was added into the solid-phase reaction column, into which was added 70 mL of DCM and left for 20 min to allow the resin to fully swell. DCM was drained, and 60 mL of DMF was added while bubbling nitrogen to wash the resin. The above steps were repeated once again, and the solvent was then drained. 10.8 g (20 mmol, 4.0 eq) of Fmoc-Rink Linker was weighed and dissolved in 40 mL of DMF, and then the mixture was added into the resin. Into the reaction column was then added 2.78 g (22 mmol, 4.4 eq) of DIC and supplemented with 10 mL of DMF while bubbling nitrogen. After complete dissolution, 2.70 g (20 mmol, 4.0 eq) of HOBt was added and nitrogen was continually bubbled for reaction.
2. After reaction at 25°C for over 2 h, a sample was taken and tested with ninhydrin as negative (with the resin being colorless or light yellow), the reaction was terminated.
3. The reaction solution was drawn off from the reaction column which was then washed with DMF (300 mL) for 5 times, each time for 1 min, and the waste was drained until no liquid flowed out.

### Step 2: Coupling of Fmoc-Ser(tBu)-OH

1. 20% of piperidine/DMF (100 mL) was added into the reaction column for reaction, while nitrogen bubbling, for 10 min. The waste was drained until no liquid flowed out. 20% of piperidine/DMF (100 mL) was added into the reaction column again for further reaction, while nitrogen bubbling, for 5 min. A sample was taken and tested with ninhydrin, with the resin appearing blue.
2. 7.66 g (4.0 eq) of Fmoc-Ser(tBu)-OH, 2.78 g (4.4 eq) of DIC and 2.70 g (4.0 eq) of HOBt were weighed and dissolved in 60 mL of DMF, and then the mixture was added into the reaction column for reaction, while nitrogen bubbling, where the nitrogen was adjusted to make the resin bulge evenly.
3. After reaction at 25°C for 2.0 h, a sample was taken and tested with ninhydrin, with the resin appearing colorless or transparent.
4. The reaction solution was drawn off from the reaction column which was then washed with DMF (300 mL) for 5 times, each time for 1 min, and the waste was drained until no liquid flowed out.

### Coupling steps of the rest amino acids on the backbone

Referring to the coupling method of Fmoc-Ser(tBu)-OH and the equivalents of the feedings in Step 2, the amino acids were coupled sequentially to synthesize the amino acid sequence. The order and the protecting group-containing amino acids used are listed as follows:

| **Protecting group-containing amino acids** | **Note** |
|---|---|
| Fmoc-Pro-OH | |
| Fmoc-Pro-OH | |
| Fmoc-Pro-OH | |
| Fmoc-Ala-OH.H₂O | |
| Fmoc-Gly-OH | |
| Fmoc-Ser(tBu)-OH | |
| Fmoc-Ser(tBu)-OH | |
| Fmoc-Pro-OH | |
| Fmoc-Gly-Gly-OH | |
| Fmoc-Glu(OAll)-OH | |
| Fmoc-Leu-OH | |
| Fmoc-Leu-OH | |
| Fmoc-Lys(Mtt)-OH | |
| Fmoc-Glu(OtBu)-OH.H₂O | |
| Fmoc-Ile-OH | |
| Fmoc-Phe-OH | |
| Fmoc-Ala-OH.H₂O | |
| Fmoc-Aib-OH | Deprotection monitored by HPLC |
| Fmoc-Gln(Trt)-OH | |
| Fmoc-Ala-OH.H₂O | |
| Fmoc-Lys(Boc)-OH | |
| Fmoc-Lys(Boc)-OH | |
| Fmoc-Asp(OtBu)-OH | |
| Fmoc-Leu-OH | |
| Fmoc-α-MeLeu-OH | Deprotection monitored by HPLC |
| Fmoc-Ile-OH | |
| Fmoc-Ser(tBu)-OH | |
| Fmoc-Tyr(tBu)-OH | |
| Fmoc-Asp(OtBu)-OH | |
| Fmoc-Ser(tBu)-OH | |
| Fmoc-Thr(tBu)-OH | |
| Fmoc-Phe-OH | |
| Fmoc-Thr(tBu)-OH | |
| Fmoc-Gly-OH | |
| Fmoc-Gln(Trt)-OH | |
| Fmoc-Aib-OH | Deprotection monitored by HPLC |
| Boc-Tyr(tBu)-OH | |

### Step 3: Coupling of branched chain

1. 300 mL of DMF and 300 mL of DCM were sequentially added to the reaction column for cross-washing the resin 4 times. After draining the solvent, 300 mL of 20% hexafluoroisopropanol (HFIP)-DCM solution was added, and the reaction was performed while nitrogen bubbling for two times, each time for 30min.
2. The waste was drained until no liquid flowed out. A sample was taken and tested with ninhydrin, with the resin appearing blue.
3. 300 mL of DCM and 300 mL of DMF were sequentially added for cross-washing the resin 4 times. The waste was drained until no liquid flowed out.
4. 9.05 g (4.0 eq) of Alloc-Lys(Fmoc)-OH, 2.78 g (4.4 eq) of DIC and 2.70 g (4.0 eq) of HOBt were weighed and dissolved in 100 mL of DMF, and then the mixture was added into the reaction column for reaction, while nitrogen bubbling, where the nitrogen was adjusted to make the resin bulge evenly.
5. After reaction at 25°C for 2.0 h, a sample was taken and tested with ninhydrin, with the resin appearing colorless or transparent.
6. The reaction solution was drawn off from the reaction column which was then washed with DMF (300 mL) for 5 times, each time for 1 min, and the waste was drained until no liquid flowed out.

### The rest steps of coupling branched chain

Referring to the coupling method of Fmoc-Ser(tBu)-OH and the equivalents of the feedings in Step 2, the compounds listed in the table below were coupled sequentially:

| **Protecting group-containing amino acids** | **Note** |
|---|---|
| Fmoc-AEEA-OH | |
| Fmoc-Glu-OtBu | |
| 20-(tert-butoxy)-20-oxoicosanoic acid | |

### Step 4: Cyclization

1. 300 mL of DMF and 300 mL of DCM were sequentially added into the reaction column for cross-washing the resin 4 times. The waste was drained until no liquid flowed out.
2. 400 mL of a mixed solvent of DCM: CH₃CN: NMP: DEA at a volume ratio of 3:3:2:2 was prepared and added into the reaction column, and 0.58 g (0.1 eq) of Pd(PPh₃)₄ was then added, and the reaction was performed while nitrogen bubbling for 10 min. The waste was drained until no liquid flowed out.
3. The above steps were repeated 1 to 2 times.
4. A sample was taken and tested with ninhydrin, with the resin appearing blue.
5. 300 mL of DMF and 300 mL of DCM were sequentially added for cross-washing the resin 8 times.
6. 2.78 g (4.4 eq) of DIC and 2.70 g (4.0 eq) of HOBt were dissolved in 150 mL of DMF, and the mixture was added into the reaction column, where the reaction was performed while nitrogen bubbling for 48 h. A sample was taken and tested with ninhydrin, with the resin appearing colorless.
7. 300 mL of DMF and 300 mL of DCM were sequentially added for cross-washing the resin 8 times.
8. 200 mL of MeOH and 300 mL of DCM were sequentially added for cross-washing the resin 4 times.
9. The resin was taken out and dried in vacuum until the weight no longer decreased, and weighed, obtaining 19.2 g of peptide resin.

The obtained intermediate (peptide resin) has a structure formula as below:
Boc-Tyr(tBu)-Aib-Gln(Trt)-Gly-Thr(tBu)-Phe-Thr(tBu)-Ser(tBu)-Asp(OtBu)-Tyr(tBu)-Ser(tBu)-Ile-a-MeL eu-Leu-Asp(OtBu)-Lys(Boc)-Lys(Boc)-Ala-Gln(Trt)-Aib-Ala-Phe-Ile-Glu(OtBu)-Lys²⁵-Leu-Leu-Glu²⁸-Gly-Gly -Pro-Ser(tBu)-Ser(tBu)-Gly-Ala-Pro-Pro-Pro-Ser(tBu)-Rink Linker-MBHA Resin (25,28(Ne(COOH-(CH₂)₁₈-COOtBu) -Glu(ε-OtBu)-AEEA) -(2-2-oxoethyl)glycine))

### Step 5. Cleavage of peptide resin

250 mL of a cleavage reagent was formulated with trifluoroacetic acid (TFA), purified water (H₂O), triisopropylsilane (TIS), 1, 2-ethanedithiol (EDT), and phenol (ArOH) at a volume ratio of TFA: H₂O: EDT: TIS: ArOH = 86.5:5:2.5:1:5, and then pre-cooled to 10±3°C. The resin was slowly added into the cleavage reagent while stirring until the temperature of the reaction system was stable, and the reaction proceeded with stirring for 4 h while controlling the temperature at 18±3°C. The cleavage solution was filtered, concentrated to thick, and precipitated with a 12-15-fold concentrate volume of methyl tert-butyl ether. The precipitate was filtered, washed, and then dried at room temperature under reduced pressure to obtain 8.2g of crude cyclopeptide.

### Step 6. Purification by chromatography

The 8.2 g of crude cyclopeptide was dissolved in 10% acetonitrile and 10% aqueous acetic acid and purified by high-performance liquid chromatography using C18 silica gel matrix filler, and the fractions with a purity of >90% were collected and combined. The purification chromatography systems are listed as follows:

| Procedures | Aqueous phase | Organic phase | Gradient |
|---|---|---|---|
| First purification | 0.2% TFA | Acetonitrile | B% (0-70 min): 41%-51% |
| Second purification | 35 mmol/L K₂HPO₄ (pH 7.4) | Acetonitrile | B% (0-50 min): 33%-44% |
| Salt Conversion | 0.6 mmol/L NaOH | Acetonitrile | B%: 45% |

### Step 7. Concentration and lyophilization

The combined fraction obtained from the above step was concentrated to remove acetonitrile, and filtered through a 0.22 µm millipore filter membrane. The filtered solution was loaded into a vial lyophilizer and lyophilized to obtain 1.01 g of the product as shown in Example 3 (with a purity of 94.97%). The molecular weight of the polypeptide was confirmed by ESI-MS, with the [M+4H]/4 calculated value of 1202.6 and the detected value of 1202.6.

### Example 4

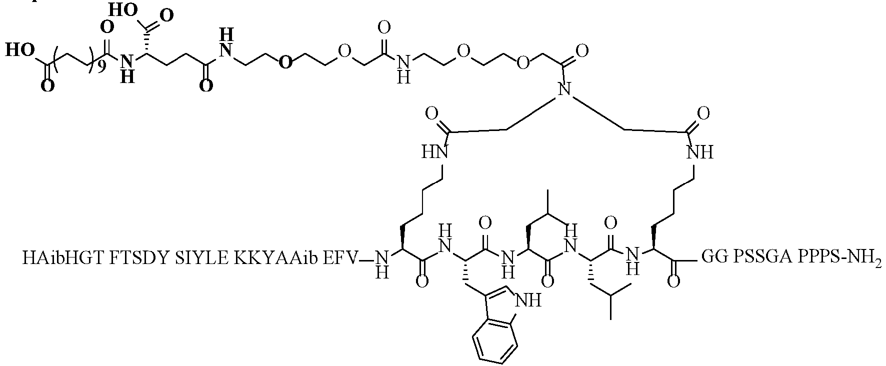

Referring to the synthesis of polypeptide in Example 1, the polypeptide as shown in Example 4 was obtained. The molecular weight of the polypeptide was confirmed by ESI-MS, with the [M+4H]/4 calculated value of 1277.7 and the detected value of 1277.6.

### Example 5

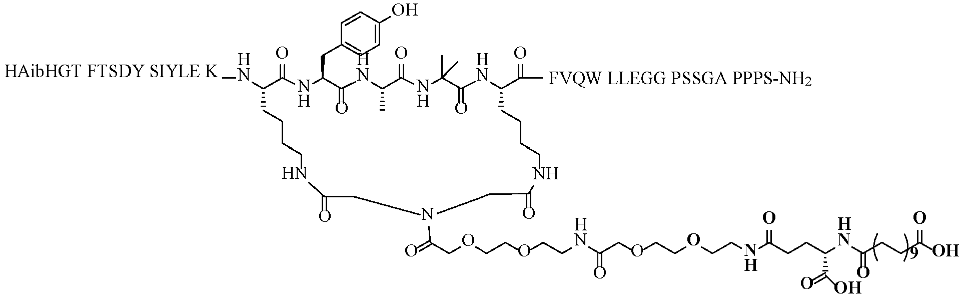

Referring to the synthesis of polypeptide in Example 1, the polypeptide as shown in Example 5 was obtained. The molecular weight of the polypeptide was confirmed by ESI-MS, with the [M+4H]/4 calculated value of 1277.7 and the detected value of 1277.5.

### Example 6

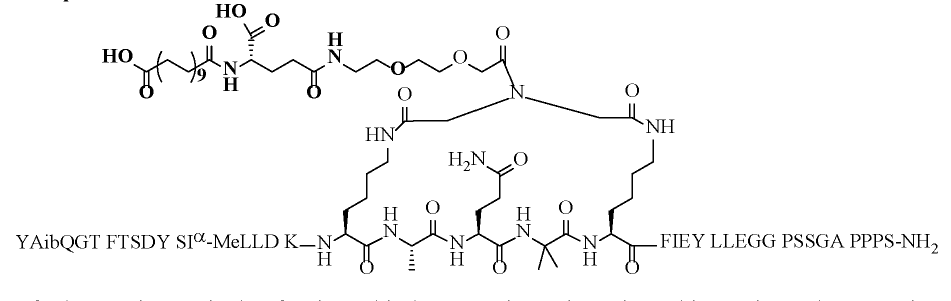

Referring to the synthesis of polypeptide in Example 1, the polypeptide as shown in Example 6 was obtained. The molecular weight of the polypeptide was confirmed by ESI-MS, with the [M+4H]/4 calculated value of 1122.4 and the detected value of 1122.5.

### Example 7

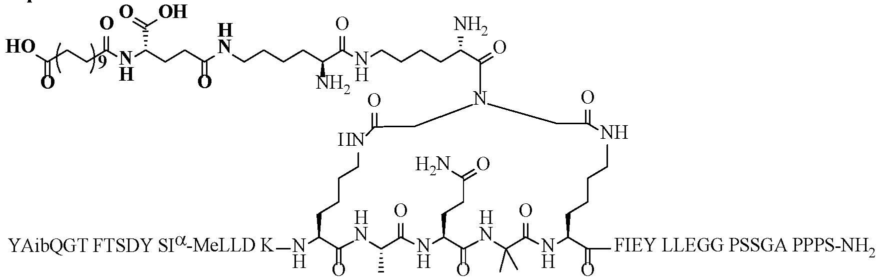

Referring to the synthesis of polypeptide in Example 1, the polypeptide as shown in Example 7 was obtained, where the protecting group-containing amino acids used in the coupling of branched chain were sequentially Fmoc-Ida (OAll)-OH, N-Boc-N'-Fmoc-Lys-OH, N-Boc-N'-Fmoc-Lys-OH, Fmoc-Glu-OtBu, and 20-(tert-butoxy)-20-oxoicosanoic acid. The molecular weight of the polypeptide was confirmed by ESI-MS, with the [M+4H]/4 calculated value of 1250.2 and the detected value of 1250.2.

### Example 8

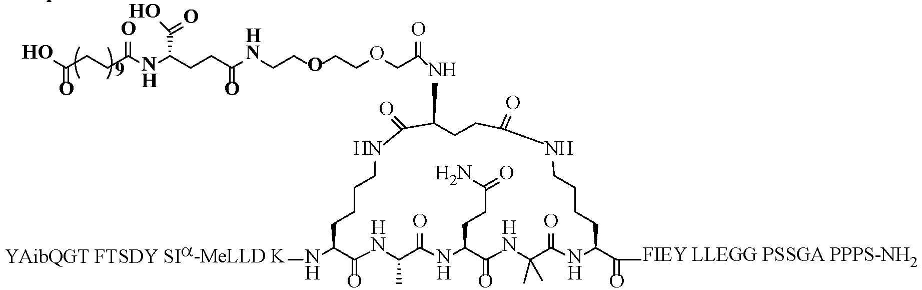

Referring to the synthesis of polypeptide in Example 1, the polypeptide as shown in Example 8 was obtained, where the protecting group-containing amino acids used in the coupling of branched chain were sequentially Fmoc-Glu(OAll)-OH, Fmoc-AEEA-OH, Fmoc-Glu-OtBu, and 20-(tert-butoxy)-20-oxoicosanoic acid. The molecular weight of the polypeptide was confirmed by ESI-MS, with the [M+4H]/4 calculated value of 1225.9 and the detected value of 1226.0.

### Example 9

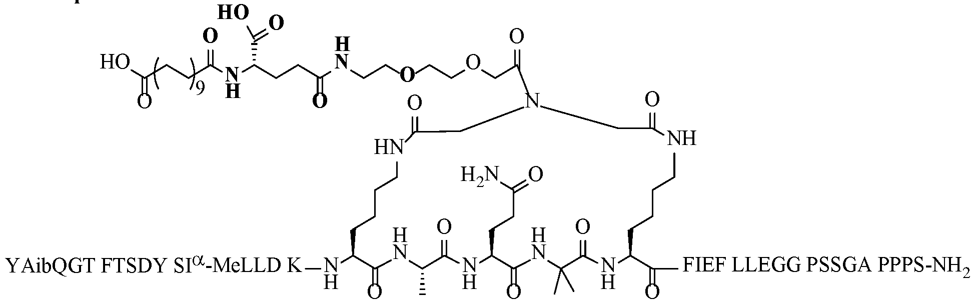

Referring to the synthesis of polypeptide in Example 1, the polypeptide as shown in Example 9 was obtained. The molecular weight of the polypeptide was confirmed by ESI-MS, with the [M+4H]/4 calculated value of 1218.4 and the detected value of 1218.3.

### Example 10

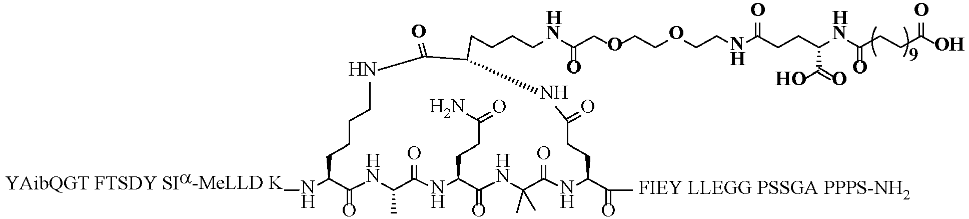

Referring to the synthesis of polypeptide in Example 3, the polypeptide as shown in Example 10 was obtained. The molecular weight of the polypeptide was confirmed by ESI-MS, with the [M+4H]/4 calculated value of 1225.9 and the detected value of 1224.2.

### Example 11

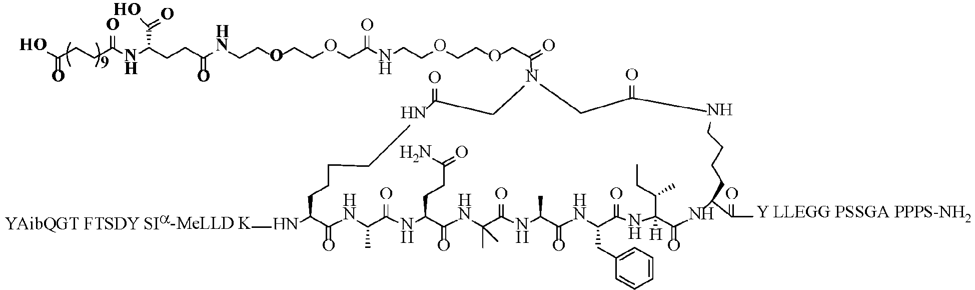

Referring to the synthesis of polypeptide in Example 1, the polypeptide as shown in Example 11 was obtained. The molecular weight of the polypeptide was confirmed by ESI-MS, with the [M+4H]/4 calculated value of 1244.2 and the detected value of 1242.7.

### Example 12

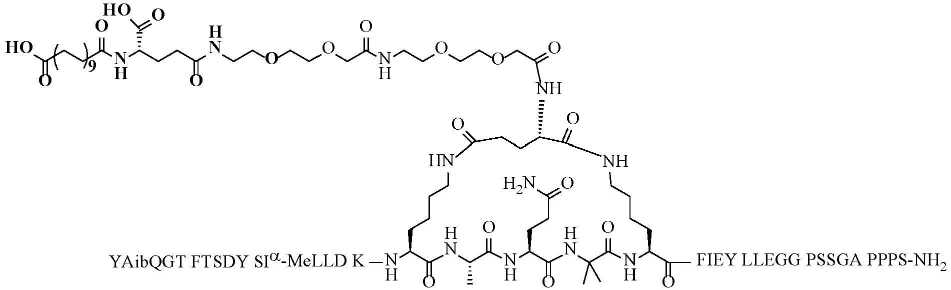

Referring to the synthesis of polypeptide in Example 1, the polypeptide as shown in Example 12 was obtained, where the protecting group-containing amino acids used in the coupling of branched chain were sequentially Fmoc-Glu-OAll, Fmoc-AEEA-OH, Fmoc-AEEA-OH, Fmoc-Glu-OtBu, and 20-(tert-butoxy)-20-oxoicosanoic acid. The molecular weight of the polypeptide was confirmed by ESI-MS, with the [M+4H]/4 calculated value of 1262.2 and the detected value of 1262.2.

### Example 13

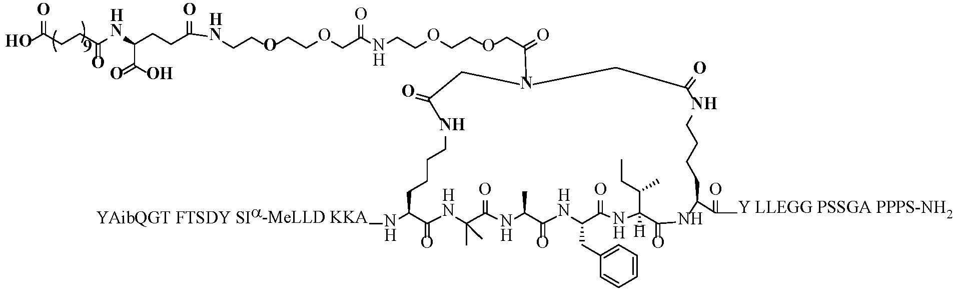

Referring to the synthesis of polypeptide in Example 1, the polypeptide as shown in Example 13 was obtained. The molecular weight of the polypeptide was confirmed by ESI-MS, with the [M+4H]/4 calculated value of 1244.2 and the detected value of 1244.2.

### Example 14

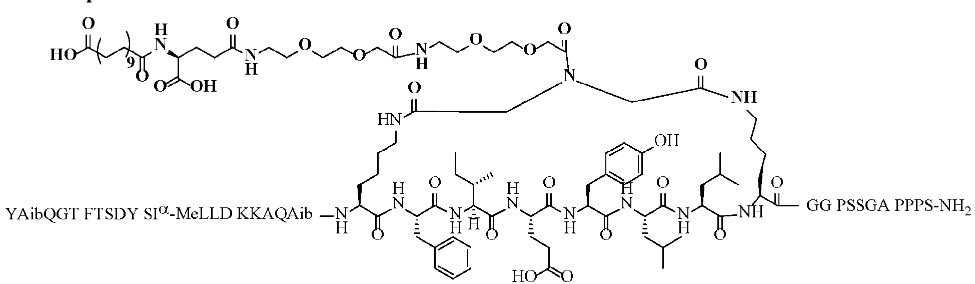

Referring to the synthesis of polypeptide in Example 1, the polypeptide as shown in Example 14 was obtained. The molecular weight of the polypeptide was confirmed by ESI-MS, with the [M+4H]/4 calculated value of 1258.5 and the detected value of 1258.4.

### Example 15

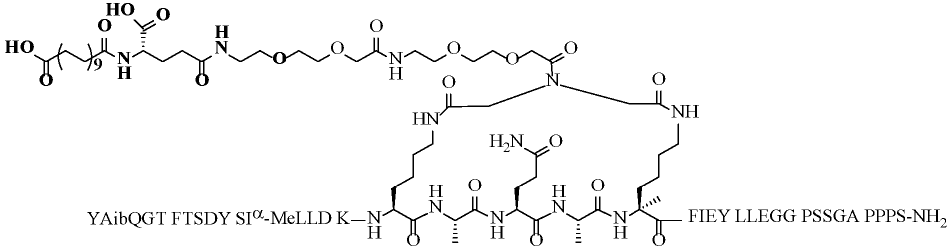

Referring to the synthesis of polypeptide in Example 1, the polypeptide as shown in Example 15 was obtained. The molecular weight of the polypeptide was confirmed by ESI-MS, with the [M+4H]/4 calculated value of 1258.7 and the detected value of 1258.6.

### Example 16

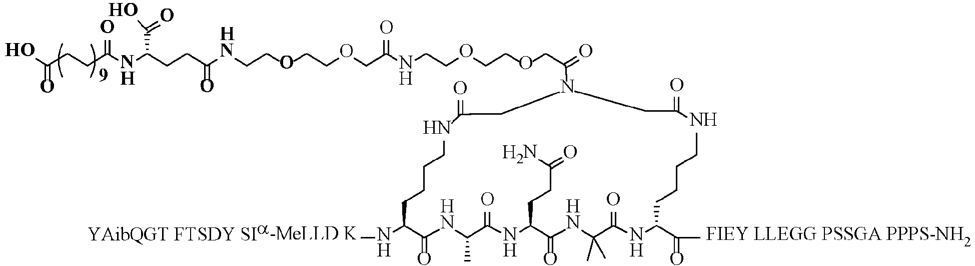

Referring to the synthesis of polypeptide in Example 1, the polypeptide as shown in Example 16 was obtained. The molecular weight of the polypeptide was confirmed by ESI-MS, with the [M+4H]/4 calculated value of 1258.7 and the detected value of 1258.6.

### Example 17

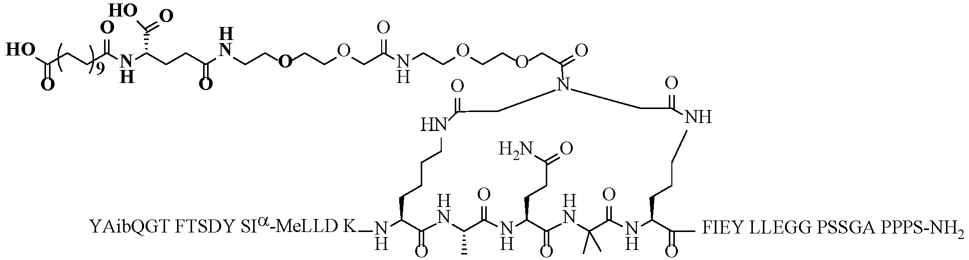

Referring to the synthesis of polypeptide in Example 1, the polypeptide as shown in Example 17 was obtained. The molecular weight of the polypeptide was confirmed by ESI-MS, with the [M+4H]/4 calculated value of 1255.2 and the detected value of 1255.1.

### Example 18

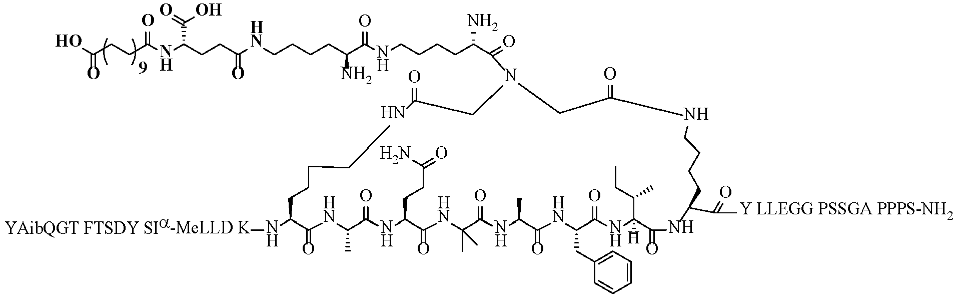

Referring to the synthesis of polypeptide in Example 1, the polypeptide as shown in Example 18 was obtained, where the protecting group-containing amino acids used in the coupling of branched chain were sequentially Fmoc-Ida (OAll)-OH, N-Boc-N'-Fmoc-Lys-OH, N-Boc-N'-Fmoc-Lys-OH, Fmoc-Glu-OtBu, and 20-(tert-butoxy)-20-oxoicosanoic acid. The molecular weight of the polypeptide was confirmed by ESI-MS, with the [M+4H]/4 calculated value of 1235.7 and the detected value of 1234.7.

### Example 19

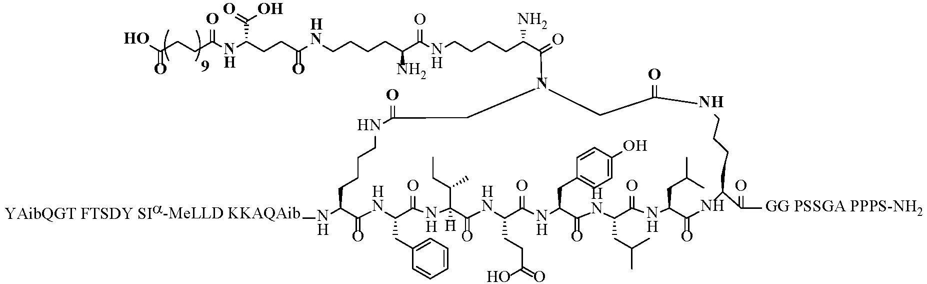

Referring to the synthesis of polypeptide in Example 1, the polypeptide as shown in Example 19 was obtained, where the protecting group-containing amino acids used in the coupling of branched chain were sequentially Fmoc-Ida (OAll)-OH, N-Boc-N'-Fmoc- Lys-OH, N-Boc-N'-Fmoc- Lys-OH, Fmoc-Glu-OtBu, and 20-(tert-butoxy)-20-oxoicosanoic acid. The molecular weight of the polypeptide was confirmed by ESI-MS, with the [M+4H]/4 calculated value of 1250.0 and the detected value of 1249.0.

### Example 20

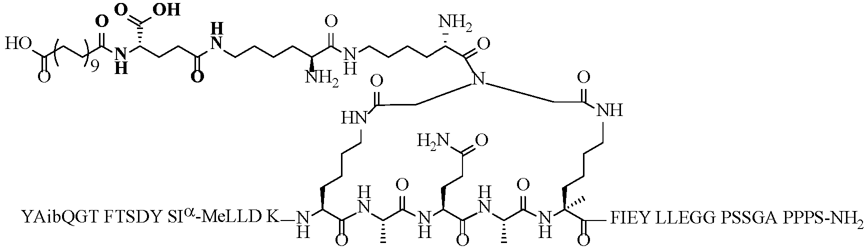

Referring to the synthesis of polypeptide in Example 1, the polypeptide as shown in Example 20 was obtained, where the protecting group-containing amino acids used in the coupling of branched chain were sequentially Fmoc-Ida (OAll)-OH, N-Boc-N'-Fmoc-Lys-OH, N-Boc-N'-Fmoc-Lys-OH, Fmoc-Glu-OtBu, and 20-(tert-butoxy)-20-oxoicosanoic acid. The molecular weight of the polypeptide was confirmed by ESI-MS, with the [M+4H]/4 calculated value of 1250.2 and the detected value of 1249.2.

### Example 21

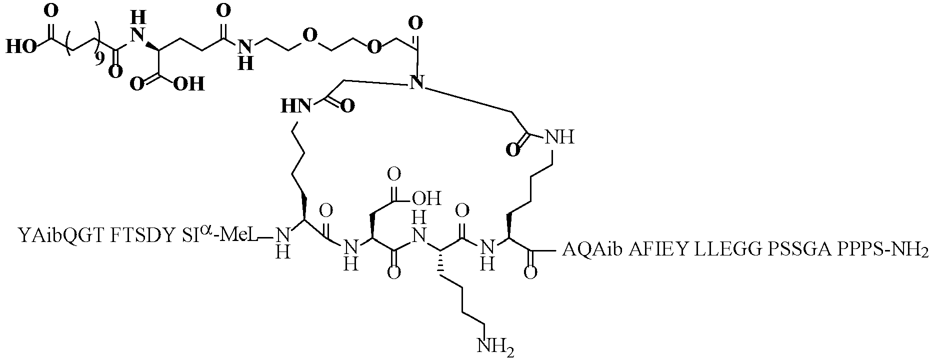

The synthesis of peptide resin was conducted by a conventional solid-phase synthesis process, using a solid-phase reaction column equipped with a sintered filter at the bottom for nitrogen bubbling during the reaction. MBHA Resin was used as the starting resin, and conventional Fmoc-protected amino acids were used as the feedings. The condensing reagent used was a system of DIC/HOBt, with DMF as the main reaction solvent, and a 20% PIP-DMF solution as the Fmoc removal solvent. The peptide backbone was synthesized from the C-terminus to the N-terminus by condensing amino acids one by one. After the backbone synthesis was completed, the ε-amino group of Fmoc-Lys(Mtt)-OH at position A-26 was deprotected by removing the -Mtt group. Then, positions 1 to 4 of side chain (including (2-2-oxoethyl)-glycine, AEEA, y Glu, and 20-(tert-butoxy)-20-oxoicosanoic acid) were condensed sequentially. After that, the ε-amino protecting group of Lys at position A-23 and the carboxyl protecting group of position 1 of side chain ((2-2-oxoethyl)-glycine) were removed, and the condensing reagent was added to form a lactam ring, yielding the target peptide resin.

### Specific steps were as follows:

### Step 1: Activation and deprotection of resin

1. 15 g (5.1 mmol) of MBHA Resin (S=0.34 mmol/g) was added into the solid-phase reaction column, into which was added 225 ml of DCM and left for 20 min to allow the resin to fully swell. DCM was drained, and 150 ml of DMF was added while bubbling nitrogen to wash the resin. The above steps were repeated once again, and the solvent was then drained. 20% of piperidine/DMF (150 mL) was added into the reaction column for reaction, while nitrogen bubbling, for 5 min. The waste was drained until no liquid flowed out. 20% of piperidine/DMF (150 mL) was added into the reaction column again for further reaction, while nitrogen bubbling, for 15 min. A sample was taken and tested with ninhydrin, with the resin appearing greyish red. The reaction solution was drawn off from the reaction column which was then washed with DMF (150 mL) for 7 times, each time for 1 min, with the pH adjusted to 7, and the waste was drained until no liquid flowed out.

### Step 2: Coupling of Fmoc-Ser(tBu)-OH

1. 5.75 g (3.0 eq) of Fmoc-Ser(tBu)-OH, 2.52 g (4.0 eq) of DIC and 2.03 g (3.0 eq) of HOBt were weighed and dissolved in 80 mL of DMF, and then the mixture was added into the reaction column for reaction, while nitrogen bubbling, where the nitrogen was adjusted to make the resin bulge evenly.
2. After reaction at 25°C for 2.0 h, a sample was taken and tested with ninhydrin, with the resin appearing colorless or transparent.
3. The reaction solution was drawn off from the reaction column which was then washed with DMF (150 mL) for 5 times, each time for 1 min, and the waste was drained until no liquid flowed out.

### Coupling steps of the rest amino acids on the backbone

Referring to the coupling method of Fmoc-Ser(tBu)-OH and the equivalents of the feedings in Step 2, the amino acids were coupled sequentially to synthesize the amino acid sequence. The order and the protecting group-containing amino acids used are listed as follows:

| **Protecting group-containing amino acids** | **Note** |
|---|---|
| Fmoc-Pro-OH | |
| Fmoc-Pro-OH | |
| Fmoc-Pro-OH | |
| Fmoc-Ala-OH | Synthesis detected by HPLC |
| Fmoc-Gly-OH | |
| Fmoc-Ser(tBu)-OH | |
| Fmoc-Ser(tBu)-OH | |
| Fmoc-Pro-OH | |
| Fmoc-Gly-OH | |
| Fmoc-Gly-OH | Synthesis detected by HPLC |

### AA-11 peptide was obtained.

### Step 3: Deprotection of AA-11 peptide

1. Into the synthesized AA-11 peptide was added DMF (80 mL) for activation for 10 min, and drained until no liquid flowed out. 20% of piperidine/DMF (80 mL) was added into the reaction column for reaction, while nitrogen bubbling, for 10 min. The waste was drained until no liquid flowed out. 20% of piperidine/DMF (80 mL) was added into the reaction column again for further reaction, while nitrogen bubbling, for 10 min. A sample was taken and tested with ninhydrin, with the resin appearing inky yellow. The reaction solution was drawn off from the reaction column which was then washed with DMF (100 mL) for 8 times, each time for 1 min, with the pH adjusted to around 7, and the waste was drained until no liquid flowed out.

### Step 4: Coupling of Fmoc-Glu(OtBu)-OH

1. 3.04 g (3.0 eq) of **Fmoc-Glu(OtBu)-OH,** 1.20 g (4.0 eq) of DIC and 0.965 g (3.0 eq) of HOBt were weighed and dissolved in 50 mL of DMF, and then the mixture was added into the reaction column for reaction, while nitrogen bubbling, where the nitrogen was adjusted to make the resin bulge evenly.
2. After reaction at 25°C for 3.0 h, a sample was taken and tested with ninhydrin, with the resin appearing colorless or transparent.
3. The reaction solution was drawn off from the reaction column which was then washed with DMF (80 mL) for 5 times, each time for 1 min, and the waste was drained until no liquid flowed out.

### Coupling steps of the rest amino acids on the backbone

Referring to the coupling method of **Fmoc-Glu(OtBu)-OH** and the order of the feedings in Step 4, the amino acids were coupled sequentially to synthesize the amino acid sequence. The order and the protecting group-containing amino acids used are listed as follows:

| **Protecting group-containing amino acids** | **Note** |
|---|---|
| Fmoc-Glu(OtBu)-OH. | AA-12 peptide |
| Fmoc-Leu-OH | |
| Fmoc-Leu-OH | |
| FmocTyr(tBu)-OH | |
| Fmoc-Glu(OtBu)-OH | |
| Fmoc-Ile-OH | |
| Fmoc-Phe-OH | |
| Fmoc-Ala-OH | |
| Fmoc-Aib-OH | |
| Fmoc-Gln(Trt)-OH | Monitored by HPLC |
| Fmoc-Ala-OH | |
| Fmoc-Lys(Alloc)-OH | |
| Fmoc-Lys(Boc)-OH | |
| Fmoc-Asp(OtBu)-OH | |
| Fmoc-Lys(Mtt)-OH | |
| Fmoc-α-Me-Leu-OH | Monitored by HPLC |
| Fmoc-Ile-OH | Monitored by HPLC |
| Fmoc-Ser(tBu)-OH | |
| Fmoc-Tyr(tBu)-OH | |
| Fmoc-Asp(OtBu)-OH | Monitored by HPLC |
| Fmoc-Ser(tBu)-OH | |
| Fmoc-Thr(tBu)-OH | |
| Fmoc-Phe-OH | |
| Fmoc-Thr(tBu)-OH | |
| Fmoc-Gly-OH | |
| Fmoc-Gln(Trt)-OH | |
| Boc-Tyr(tBu) -Aib-OH | Monitored by HPLC |

### Step 5. Coupling of branched chain

1. 300 mL of DCM was added into the reaction column for washing the resin 4 times. After draining the solvent, 80 mL of 20% hexafluoroisopropanol (HFIP)-DCM solution was added, and the reaction was performed while nitrogen bubbling for 5 times, each time for 30 min.
2. The waste was drained until no liquid flowed out. A sample was taken and tested with ninhydrin, with the resin appearing greyish green.
3. 80 mL of DCM and 80 mL of DMF were sequentially added for cross-washing the resin 4 times. The waste was drained until no liquid flowed out.
4. 4.7 g (5.0 eq) of Fmoc-(2-(allyloxy)-2-oxoethyl)glycine (Fmoc-Ida (OAll)-OH), 1.67 g (6.0eq) of DIC and 1.49 g (5.0 eq) of HOBt were weighed and dissolved in 100 mL of DMF, and then the mixture was added into the reaction column for reaction, while nitrogen bubbling, where the nitrogen was adjusted to make the resin bulge evenly.
5. After reaction at 25°C for 5.0 h, a sample was taken and tested with ninhydrin, with the resin appearing colorless or transparent.
6. The reaction solution was drawn off from the reaction column which was then washed with DMF (80 mL) for 5 times, each time for 1 min, and the waste was drained until no liquid flowed out.

### The rest steps of coupling branched chain

Referring to the coupling method of **Fmoc-Glu(OtBu)-OH** and the equivalents of the feedings in Step 4, the compounds listed in the table below were coupled sequentially:

| **Protecting group-containing amino acids** | **Note** |
|---|---|
| Fmoc-AEEA-OH | Monitored by HPLC |
| Fmoc-Glu-OtBu | |
| 20-(tert-butoxy)-20-oxoicosanoic acid | |

### Step 6. Cyclization

1. 80 mL of DMF and 80 mL of DCM were sequentially added into the reaction column for cross-washing the resin 4 times. The waste was drained until no liquid flowed out.
2. 300 mL of a mixed solvent of DCM: CH₃CN: NMP: DEA at a volume ratio of 3:3:2:2 was prepared and added into the reaction column, and 0.74 g (0.27 eq) of Pd(PPh₃)₄ was then added, and the reaction was performed while nitrogen bubbling for 10 min. The waste was drained until no liquid flowed out.
3. A sample was taken and tested with ninhydrin, with the resin appearing yellowish purple.
4. 80 mL of DMF and 80 mL of DCM were sequentially added for cross-washing the resin 8 times.
5. 1.67 g (6.0 eq) of DIC and 1.49 g (5.0 eq) of HOBt were dissolved in 80 mL of DMF, and the mixture was added into the reaction column, where the reaction was performed while nitrogen bubbling for 22 h. A sample was taken and tested with ninhydrin, with the resin appearing colorless.
6. 80 mL of DMF and 80 mL of DCM were sequentially added into the reaction column for cross-washing the resin 8 times.
7. 80 mL of MeOH and 80 mL of DCM were sequentially added into the reaction column for cross-washing the resin 4 times.
8. The resin was taken out and dried in vacuum until the weight no longer decreased, and weighed, obtaining 19.3 g of peptide resin.

The obtained intermediate (peptide resin) has a structure formula as below:
Boc-Tyr(tBu)-Aib-Gln(Trt)-Gly-Thr(tBu)-Phe-Thr(tBu)-Ser(tBu)-Asp(OtBu)-Tyr(tBu)-Ser(tBu)-Ile-a-MeL eu-Lys¹⁴-Asp(OtBu)-Lys(Boc)-Lys¹⁷-Ala-Gln(Trt)-Aib-Ala-Phe-Ile-Glu(OtBu)-Tyr(tBu)-Leu-Leu-Glu(OtBu)-G ly-Gly-Pro-Ser(tBu)-Ser(tBu)-Gly-Ala-Pro-Pro-Pro-Ser(tBu)-Rink Linker -MBHA Resin (14,17(Nε(HOOC-(CH₂)₁₈-COOtBu) -Glu(ε-OtBu)-AEEA)-(2-2-oxoethyl)glycine))

### Step 7. Cleavage of peptide resin

289 mL of a cleavage reagent was formulated with trifluoroacetic acid (TFA), purified water (H₂O), triisopropylsilane (TIS), 3-(tritylthio)propionic acid (Mpa(Trt)-OH, MRP), and phenol (ArOH) at a volume ratio of TFA: H₂O: Mpa(Trt)-OH: TIS: ArOH=82.5:2.5:5:5:5, and then pre-cooled to 10±3°C. The resin was slowly added into the cleavage reagent while stirring until the temperature of the reaction system was stable, and the reaction proceeded with stirring for 4 h while controlling the temperature at 18±3°C. The cleavage solution was filtered, concentrated to thick, and precipitated with a 12-15-fold concentrate volume of methyl tert-butyl ether. The precipitate was filtered, washed, and then dried at room temperature under reduced pressure to obtain 7.5 g of crude cyclopeptide.

### Step 8. Purification by chromatography

2.1 g of the crude cyclopeptide was dissolved in 5% acetonitrile and 2% aqueous ammonia solution and purified by high-performance liquid chromatography using C8 silica gel matrix filler, and the fractions with a purity of >90% were collected and combined. The purification chromatography systems are listed as follows:

| Procedures | Aqueous phase | Organic phase | Gradient |
|---|---|---|---|
| First purification | 0.2% TFA | Acetonitrile | B% (0-50 min): 41%-55% |
| Second purification | 0.2% TFA | Acetonitrile | B% (0-50 min): 41%-55% |
| Third purification | 0.2% TFA | Acetonitrile | B% (0-50 min): 41%-55% |

### Step 9. Concentration and lyophilization

The combined fraction obtained from the above step was concentrated to remove acetonitrile, and filtered through a 0.22 µm millipore filter membrane. The filtered solution was loaded into a vial lyophilizer and lyophilized to obtain 0.133 g of target polypeptide product (with a purity of 94.2%). The molecular weight of the polypeptide was confirmed by ESI-MS, with the [M+4H]/4 calculated value of 1211.9 and the detected value of 1210.9.

### Example 22

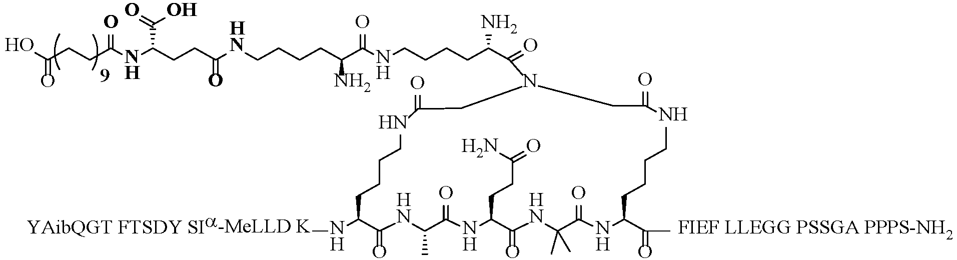

Referring to the synthesis of polypeptide in Example 1, the polypeptide as shown in Example 22 was obtained, where the protecting group-containing amino acids used in the coupling of branched chain were sequentially Fmoc-Ida (OAll)-OH, N-Boc-N'-Fmoc- Lys-OH, N-Boc-N'-Fmoc- Lys-OH, Fmoc-Glu-OtBu, and 20-(tert-butoxy)-20-oxoicosanoic acid. The molecular weight of the polypeptide was confirmed by ESI-MS, with the [M+4H]/4 calculated value of 1246.2 and the detected value of 1245.1.

### Example 23

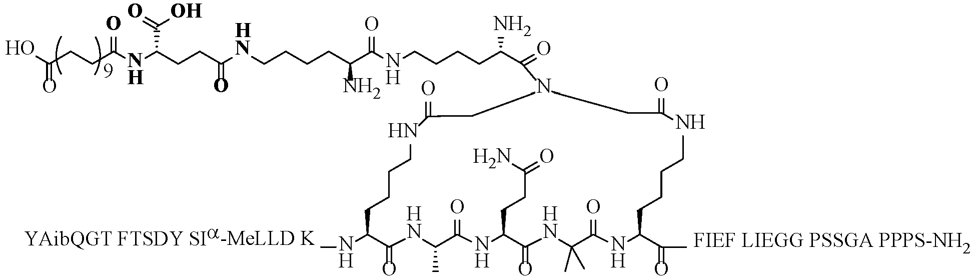

Referring to the synthesis of polypeptide in Example 1, the polypeptide as shown in Example 23 was obtained, where the protecting group-containing amino acids used in the coupling of branched chain were sequentially Fmoc-Ida (OAll)-OH, N-Boc-N'-Fmoc-Lys-OH, N-Boc-N'-Fmoc-Lys-OH, Fmoc-Glu-OtBu, and 20-(tert-butoxy)-20-oxoicosanoic acid. The molecular weight of the polypeptide was confirmed by ESI-MS, with the [M+4H]/4 calculated value of 1246.2 and the detected value of 1245.2.

### Example 24

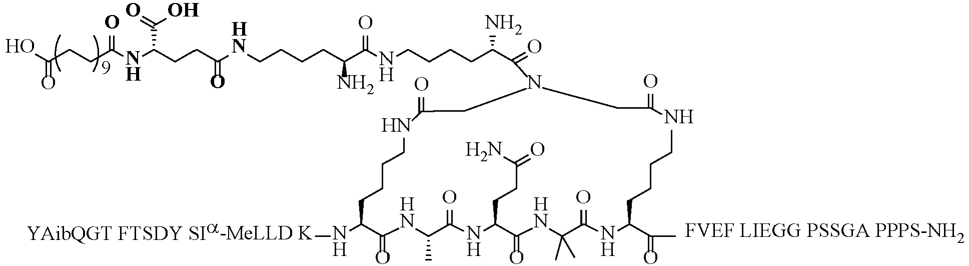

Referring to the synthesis of polypeptide in Example 1, the polypeptide shown in Example 24 was obtained, where the protecting group-containing amino acids used in the coupling of branched chain were sequentially Fmoc-Ida (OAll)-OH, N-Boc-N'-Fmoc-Lys-OH, N-Boc-N'-Fmoc-Lys-OH, Fmoc-Glu-OtBu, and 20-(tert-butoxy)-20-oxoicosanoic acid. The molecular weight of the polypeptide was confirmed by ESI-MS, with the [M+4H]/4 calculated value of 1242.7 and the detected value of 1241.6.

### Example 25

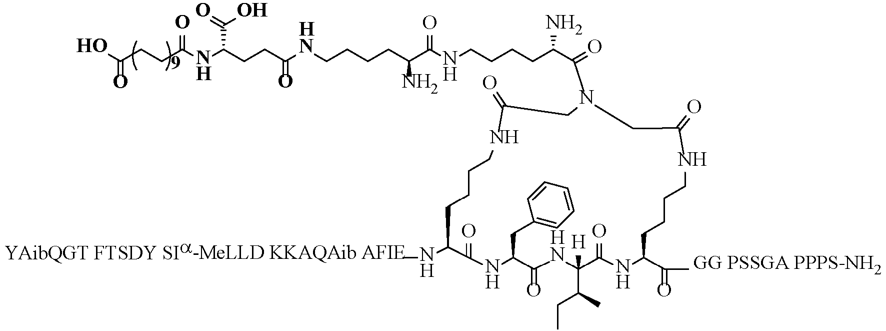

Referring to the synthesis of polypeptide in Example 1, the polypeptide shown in Example 25 was obtained, where the protecting group-containing amino acids used in the coupling of branched chain were sequentially Fmoc-Ida (OAll)-OH, N-Boc-N'-Fmoc-Lys-OH, N-Boc-N'-Fmoc-Lys-OH, Fmoc-Glu-OtBu, and 20-(tert-butoxy)-20-oxoicosanoic acid. The molecular weight of the polypeptide was confirmed by ESI-MS, with the [M+4H]/4 calculated value of 1235.4 and the detected value of 1234.3.

### Example 26

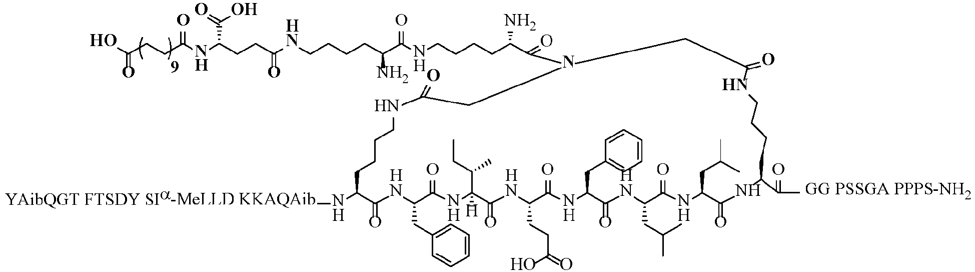

Referring to the synthesis of polypeptide in Example 1, the polypeptide as shown in Example 26 was obtained, where the protecting group-containing amino acids used in the coupling of branched chain were sequentially Fmoc-Ida (OAll)-OH, N-Boc-N'-Fmoc- Lys-OH, N-Boc-N'-Fmoc-Lys-OH, Fmoc-Glu-OtBu, and 20-(tert-butoxy)-20-oxoicosanoic acid. The molecular weight of the polypeptide was confirmed by ESI-MS, with the [M+4H]/4 calculated value of 1246.0 and the detected value of 1250.0.

### Example 27

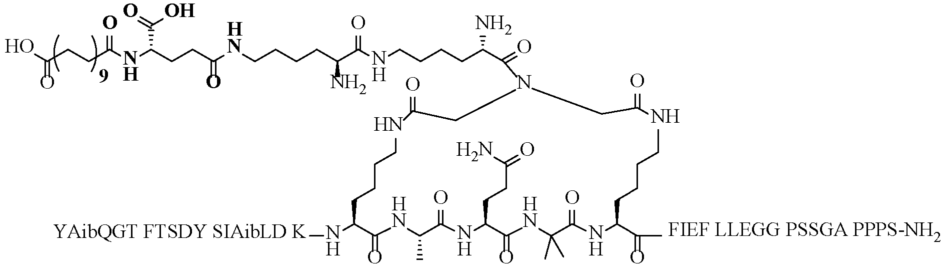

Referring to the synthesis of polypeptide in Example 1, the polypeptide as shown in Example 27 was obtained, where the protecting group-containing amino acids used in the coupling of branched chain were sequentially Fmoc-Ida (OAll)-OH, N-Boc-N'-Fmoc- Lys-OH, N-Boc-N'-Fmoc- Lys-OH, Fmoc-Glu-OtBu, and 20-(tert-butoxy)-20-oxoicosanoic acid. The molecular weight of the polypeptide was confirmed by ESI-MS, with the [M+4H]/4 calculated value of 1235.7 and the detected value of 1234.6.

### Example 28

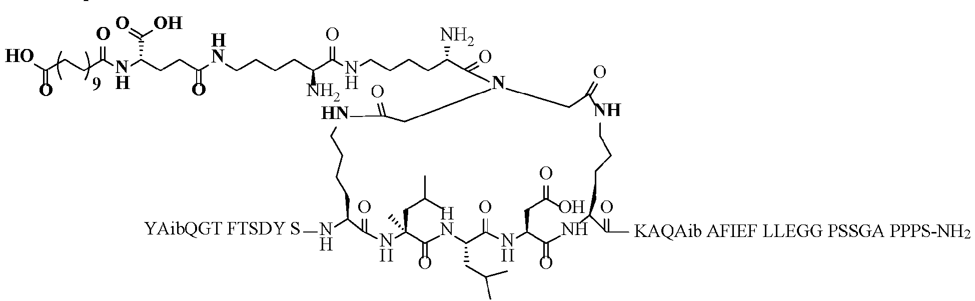

Referring to the synthesis of polypeptide in Example 1, the polypeptide shown in Example 28 was obtained, where the protecting group-containing amino acids used in the coupling of branched chain were sequentially Fmoc-Ida (OAll)-OH, N-Boc-N'-Fmoc- Lys-OH, N-Boc-N'-Fmoc- Lys-OH, Fmoc-Glu-OtBu, and 20-(tert-butoxy)-20-oxoicosanoic acid. The molecular weight of the polypeptide was confirmed by ESI-MS, with the [M+4H]/4 calculated value of 1235.7 and the detected value of 1238.6.

### Example 29

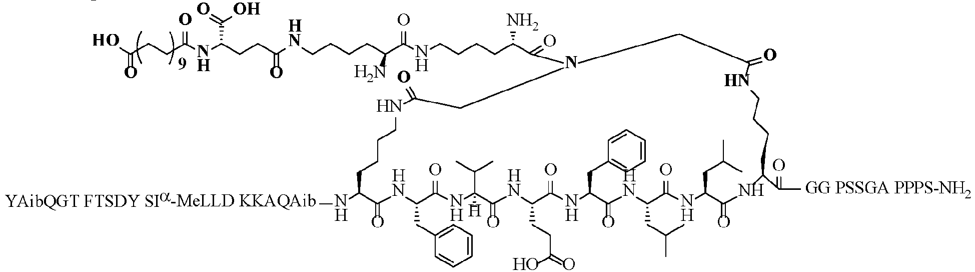

Referring to the synthesis of polypeptide in Example 1, the polypeptide shown in Example 29 was obtained, where the protecting group-containing amino acids used in the coupling of branched chain were sequentially Fmoc-Ida (OAll)-OH, N-Boc-N'-Fmoc-Lys-OH, N-Boc-N'-Fmoc-Lys-OH, Fmoc-Glu-OtBu, and 20-(tert-butoxy)-20-oxoicosanoic acid. The molecular weight of the polypeptide was confirmed by ESI-MS, with the [M+4H]/4 calculated value of 1242.5 and the detected value of 1241.5.

### Example 30

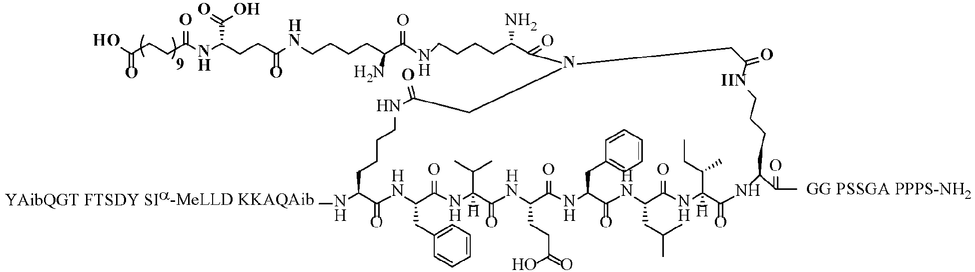

Referring to the synthesis of polypeptide in Example 1, the polypeptide as shown in Example 30 was obtained, where the protecting group-containing amino acids used in the coupling of branched chain were sequentially Fmoc-Ida (OAll)-OH, N-Boc-N'-Fmoc-Lys-OH, N-Boc-N'-Fmoc-Lys-OH, Fmoc-Glu-OtBu, and 20-(tert-butoxy)-20-oxoicosanoic acid. The molecular weight of the polypeptide was confirmed by ESI-MS, with the [M+4H]/4 calculated value of 1242.5 and the detected value of 1241.6.

### Example 31

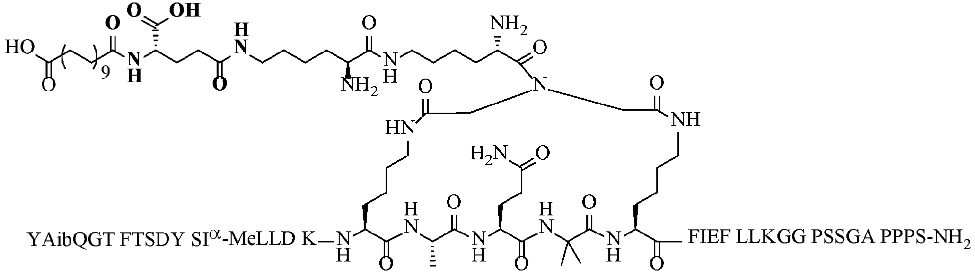

Referring to the synthesis of polypeptide in Example 1, the polypeptide shown in Example 31 was obtained, where the protecting group-containing amino acids used in the coupling of branched chain were sequentially Fmoc-Ida (OAll)-OH, N-Boc-N'-Fmoc- Lys-OH, N-Boc-N'-Fmoc-Lys-OH, Fmoc-Glu-OtBu, and 20-(tert-butoxy)-20-oxoicosanoic acid. The molecular weight of the polypeptide was confirmed by ESI-MS, with the [M+4H]/4 calculated value of 1246.0 and the detected value of 1244.9.

### Example 32

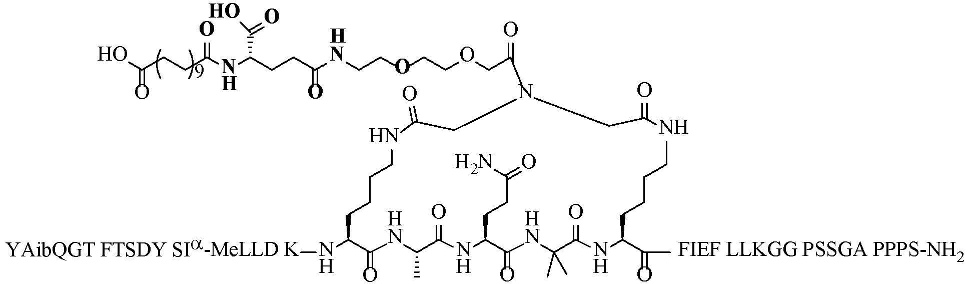

Referring to the synthesis of polypeptide in Example 1, the polypeptide as shown in Example 32 was obtained. The molecular weight of the polypeptide was confirmed by ESI-MS, with the [M+4H]/4 calculated value of 1218.2 and the detected value of 1217.4.

### Example 33

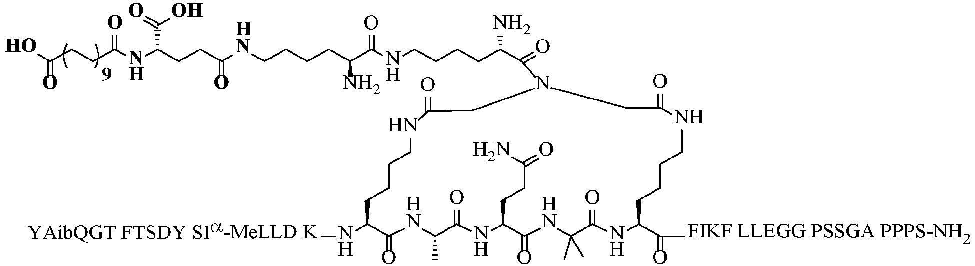

Referring to the synthesis of polypeptide in Example 1, the polypeptide shown in Example 33 was obtained, where the protecting group-containing amino acids used in the coupling of branched chain were sequentially Fmoc-Ida (OAll)-OH, N-Boc-N'-Fmoc-Lys-OH, N-Boc-N'-Fmoc-Lys-OH, Fmoc-Glu-OtBu, and 20-(tert-butoxy)-20-oxoicosanoic acid. The molecular weight of the polypeptide was confirmed by ESI-MS, with the [M+4H]/4 calculated value of 1246.0 and the detected value of 1245.9.

### Example 34

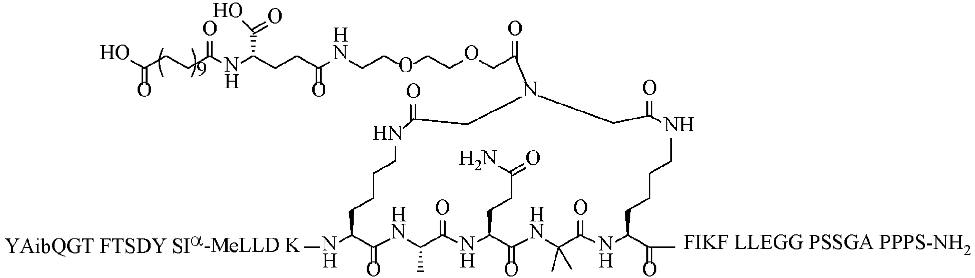

Referring to the synthesis of polypeptide in Example 1, the polypeptide as shown in Example 34 was obtained. The molecular weight of the polypeptide was confirmed by ESI-MS, with the [M+4H]/4 calculated value of 1218.2 and the detected value of 1218.1.

### Example 35

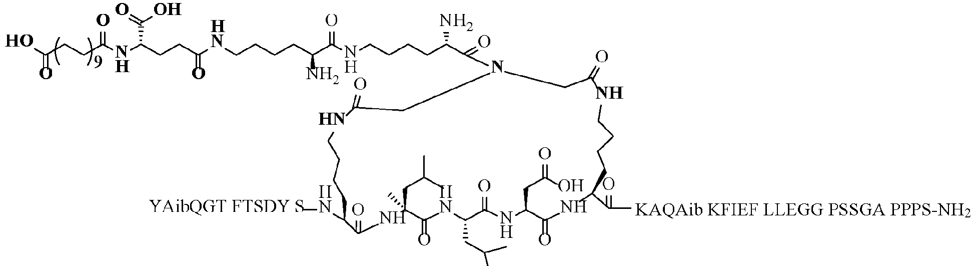

Referring to the synthesis of polypeptide in Example 1, the polypeptide shown in Example 35 was obtained, where the protecting group-containing amino acids used in the coupling of branched chain were sequentially Fmoc-Ida (OAll)-OH, N-Boc-N'-Fmoc-Lys-OH, N-Boc-N'-Fmoc-Lys-OH, Fmoc-Glu-OtBu, and 20-(tert-butoxy)-20-oxoicosanoic acid. The molecular weight of the polypeptide was confirmed by ESI-MS, with the [M+4H]/4 calculated value of 1250.0 and the detected value of 1250.0.

### Example 36

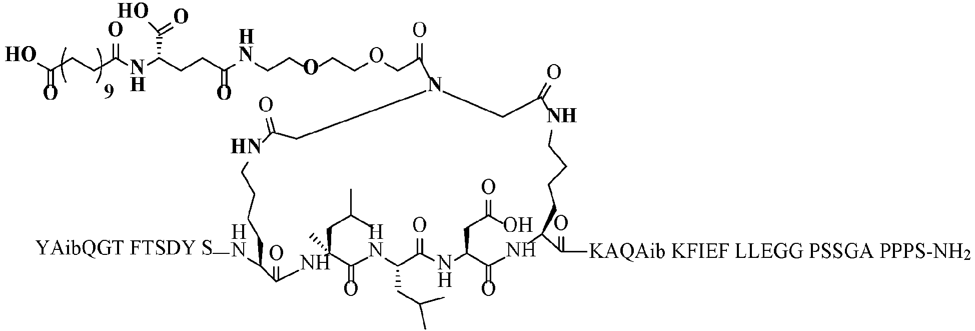

Referring to the synthesis of polypeptide in Example 1, the polypeptide as shown in Example 36 was obtained. The molecular weight of the polypeptide was confirmed by ESI-MS, with the [M+4H]/4 calculated value of 1222.2 and the detected value of 1222.1.

### Example 37

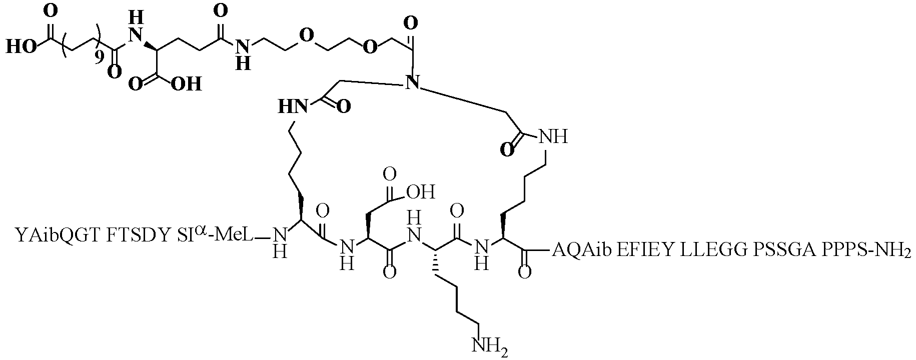

Referring to the synthesis of polypeptide in Example 1, the polypeptide as shown in Example 37 was obtained. The molecular weight of the polypeptide was confirmed by ESI-MS, with the [M+4H]/4 calculated value of 1226.4 and the detected value of 1226.3.

### Example 38

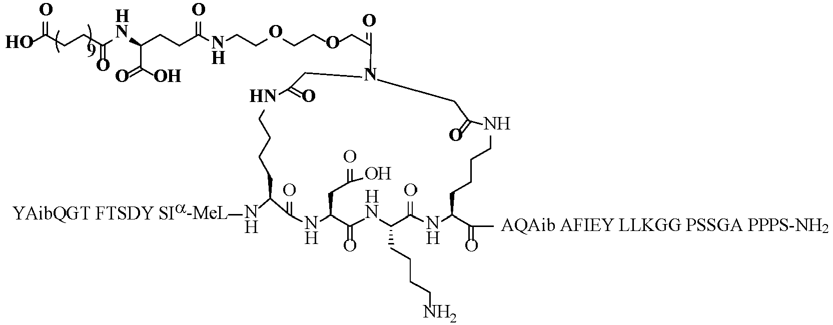

A synthesis process similar to the polypeptide synthesis of Example 21 above was employed. MBHA Resin was used as the starting resin, and conventional Fmoc-protected amino acids were used as the feedings. The condensing reagent used was a system of DIC/HOBt, with DMF as the main reaction solvent, and a 20% PIP-DMF solution as the Fmoc removal solvent. The peptide backbone was synthesized from the C-terminus to the N-terminus by condensing amino acids one by one. After the backbone synthesis was completed, the ε-amino group of Fmoc-Lys(Mtt)-OH at position A-26 was deprotected by removing the -Mtt group. Then, positions 1 to 4 of side chain ((2-2-oxoethyl)-glycine, AEEA, y Glu, and 20-(tert-butoxy)-20-oxoicosanoic acid) were condensed sequentially. After that, the ε-amino protecting group of Lys at position A-23 and the carboxyl protecting group of position 1 of side chain ((2-2-oxoethyl)-glycine) were removed, and the condensing reagent was added to form a lactam ring, yielding the target peptide resin.

### Specific steps were as follows:

### Step 1: Activation and deprotection of resin

1. 15 g (5.1 mmol) of MBHA Resin (S=0.34 mmol/g) was added into the solid-phase reaction column, into which was added 225 mL of DCM and left for 20 min to allow the resin to fully swell. DCM was drained, and 150 mL of DMF was added while bubbling nitrogen to wash the resin. The above steps were repeated once again, and the solvent was then drained. 20% of piperidine/DMF (150 mL) was added into the reaction column for reaction, while nitrogen bubbling, for 10 min. The waste was drained until no liquid flowed out. 20% of piperidine/DMF (150 mL) was added into the reaction column again for further reaction, while nitrogen bubbling, for 10 min. A sample was taken and tested with ninhydrin, with the resin appearing greyish red. The reaction solution was drawn off from the reaction column which was then washed with DMF (150 mL) for 8 times, each time for 1 min, with the pH adjusted to 7, and the waste was drained until no liquid flowed out.

### Step 2: Coupling of Fmoc-Ser(tBu)-OH

1. 5.86 g (3.0 eq) of Fmoc-Ser(tBu)-OH, 2.57 g (4.0 eq) of DIC and 2.07 g (3.0 eq) of HOBt were weighed and dissolved in 80 mL of DMF, and then the mixture was added into the reaction column for reaction, while nitrogen bubbling, where the nitrogen was adjusted to make the resin bulge evenly.
2. After reaction at 25°C for 2.0 h, a sample was taken and tested with ninhydrin, with the resin appearing colorless or transparent.
3. The reaction solution was drawn off from the reaction column which was then washed with DMF (150 mL) for 5 times, each time for 1 min, and the waste was drained until no liquid flowed out.

### Coupling steps of the rest amino acids on the backbone

Referring to the coupling method of Fmoc-Ser(tBu)-OH and the equivalents of the feedings in Step 2, the amino acids were coupled sequentially to synthesize the amino acid sequence. The order and the protecting group-containing amino acids used are listed as follows:

| **Protecting group-containing amino acids** | **Note** |
|---|---|
| Fmoc-Pro-OH | |
| Fmoc-Pro-OH | |
| Fmoc-Pro-OH | |
| Fmoc-Ala-OH | |
| Fmoc-Gly-OH | |
| Fmoc-Ser(tBu)-OH | |
| Fmoc-Ser(tBu)-OH | |
| Fmoc-Pro-OH | |
| Fmoc-Gly-OH | |
| Fmoc-Gly-OH | Synthesis detected by HPLC |

AA-11 peptide was obtained.

### Step 3: Deprotection of AA-11 peptide

1. Into the synthesized AA-11 peptide was added DMF (150 mL) for activation for 10 min, and drained until no liquid flowed out. 20% of piperidine/DMF (150 mL) was added into the reaction column for reaction, while nitrogen bubbling, for 10 min. The waste was drained until no liquid flowed out. 20% of piperidine/DMF (150 mL) was added into the reaction column again for further reaction, while nitrogen bubbling, for 10 min. A sample was taken and tested with ninhydrin, with the resin appearing dark yellow. The reaction solution was drawn off from the reaction column which was then washed with DMF (150 mL) for 8 times, each time for 1 min, with the pH adjusted to around 7, and the waste was drained until no liquid flowed out.

### Step 4: Coupling of Fmoc-Lys(Boc)-OH

1. 7.17 g (3.0 eq) of Fmoc-Lys(Boc)-OH, 2.57 g (4.0 eq) of DIC and 2.07 g (3.0 eq) of HOBt were weighed and dissolved in 150 mL of DMF, and then the mixture was added into the reaction column for reaction, while nitrogen bubbling, where the nitrogen was adjusted to make the resin bulge evenly.
2. After reaction at 25°C for 3.0 h, a sample was taken and tested with ninhydrin, with the resin appearing colorless or transparent.
3. The reaction solution was drawn off from the reaction column which was then washed with DMF (150 mL) for 5 times, each time for 1 min, and the waste was drained until no liquid flowed out.

### Coupling steps of the rest amino acids on the backbone

Referring to the coupling method of Fmoc-Lys(Boc)-OH and the order of the feedings in Step 4, the amino acids were coupled sequentially to synthesize the amino acid sequence. The order and the protecting group-containing amino acids used are listed as follows:

| **Protecting group-containing amino acids** | **Note** |
|---|---|
| Fmoc-Leu-OH | AA-13 peptide |
| Fmoc-Leu-OH | |
| Fmoc-Tyr(tBu)-OH | |
| Fmoc-Glu(OtBu)-OH | |
| Fmoc-Ile-OH | |
| Fmoc-Phe-OH | |
| Fmoc-Ala-OH | |
| Fmoc-Aib-OH | |
| Fmoc-Gln(Trt)-OH | Monitored by HPLC |
| Fmoc-Ala-OH | |
| Fmoc-Lys(Alloc)-OH | |
| Fmoc-Lys(Boc)-OH | |
| Fmoc-Asp(OtBu)-OH | |
| Fmoc-Lys(Mtt)-OH | |
| Fmoc-α-Me-Leu-OH | Monitored by HPLC |
| Fmoc-Ile-OH | Monitored by HPLC |
| Fmoc-Ser(tBu)-OH | |
| Fmoc-Tyr(tBu)-OH | |
| Fmoc-Asp(OtBu)-OH | |
| Fmoc-Ser(tBu)-OH | |
| Fmoc-Thr(tBu)-OH | |
| Fmoc-Phe-OH | |
| Fmoc-Thr(tBu)-OH | |
| Fmoc-Gly-OH | |
| Fmoc-Gln(Trt)-OH | |
| Boc-Tyr(tBu)-Aib-OH | Monitored by HPLC |

### Step 5. Coupling of branched chain

1. 300 mL of DCM was added into the reaction column for washing the resin 4 times. After draining the solvent, 150 mL of 20% hexafluoroisopropanol (HFIP)-DCM solution was added, and the reaction was performed while nitrogen bubbling for 5 times, each time for 30 min.
2. The waste was drained until no liquid flowed out. A sample was taken and tested with ninhydrin, with the resin appearing greyish green.
3. 150 mL of DCM and 150 mL of DMF were sequentially added for cross-washing the resin 4 times. The waste was drained until no liquid flowed out.
4. 10.08 g (5.0 eq) of Fmoc-Ida(Oall)-OH, 3.86 g (6.0 eq) of DIC and 3.45 g (5.0 eq) of HOBt were weighed and dissolved in 150 mL of DMF, and then the mixture was added into the reaction column for reaction, while nitrogen bubbling, where the nitrogen was adjusted to make the resin bulge evenly.
5. After reaction at 25°C for 3.0 h, a sample was taken and tested with ninhydrin, with the resin appearing colorless or transparent.
6. The reaction solution was drawn off from the reaction column which was then washed with DMF (180 mL) for 5 times, each time for 1 min, and the waste was drained until no liquid flowed out.

### The rest steps of coupling branched chain

Referring to the coupling method of Fmoc-Ida(Oall)-OH and the equivalents of the feedings in Step 5, the compounds listed in the table below were coupled sequentially:

| **Protecting group-containing amino acids** | **Note** |
|---|---|
| Fmoc-AEEA-OH | |
| Fmoc-Glu-OtBu | |
| 20-(tert-butoxy)-20-oxoicosanoic acid | Monitored by HPLC |

### Step 6. Cyclization

1. 250 mL of DMF and 250 mL of DCM were sequentially added into the reaction column for cross-washing the resin 4 times. The waste was drained until no liquid flowed out.
2. 300 mL of a mixed solvent of DCM: CH₃CN: NMP: DEA at a volume ratio of 3:3:2:2 was prepared and added into the reaction column, and 1.59 g (0.27 eq) of Pd(PPh₃)₄ was then added, and the reaction was performed while nitrogen bubbling for 30 min. The waste was drained until no liquid flowed out.
3. A sample was taken and tested with ninhydrin, with the resin appearing fuchsia.
4. 250 mL of DMF and 250 mL of DCM were sequentially added for cross-washing the resin 8 times.
5. 3.86 g (6.0 eq) of DIC and 3.45 g (5.0 eq) of HOBt were dissolved in 250 mL of DMF, and the mixture was added into the reaction column, where the reaction was performed while nitrogen bubbling for 22 h. A sample was taken and tested with ninhydrin, with the resin appearing very faint purple.
6. 250 mL of DMF and 250 mL of DCM were sequentially added for cross-washing the resin 8 times.
7. 200 mL of MeOH and 200 mL of DCM were sequentially added for cross-washing the resin 4 times.
8. The resin was taken out and dried in vacuum until the weight no longer decreased, and weighed, obtaining 32.5 g of peptide resin.

The obtained intermediate (peptide resin) has a structure formula as below:
Nε(Boc-Tyr(tBu)-Aib-Gln(Trt)-Gly-Thr(tBu)-Phe-Thr(tBu)-Ser(tBu)-Asp(OtBu)-Tyr(tBu)-Ser(tBu)-Ile-a-MeLeu-Lys¹⁴-Asp(OtBu)-Lys(Boc)-Lys¹⁷-Ala-Gln(Trt)-Aib-Ala-Phe-Ile-Glu(OtBu)-Tyr(tBu)-Leu-Leu-Lys(Boc)-Gly-Gly-Pro-Ser(tBu)-Ser(tBu)-Gly-Ala-Pro-Pro-Pro-Ser(tBu)-Rink Linker -MBHA Resin (14,17(Nε(HOOC-(CH₂)₁₈-COOtBu) -Glu(ε-OtBu)- (AEEA)-(2-2-oxoethyl)glycine))

### Step 7. Cleavage of peptide resin

500 mL of a cleavage reagent was formulated with trifluoroacetic acid (TFA), purified water (H₂O), triisopropylsilane (TIS), 3-(tritylthio)propionic acid (Mpa(Trt)-OH), and phenol (ArOH) at a volume ratio of TFA: H₂O: Mpa(Trt)-OH: TIS: ArOH=82.5:2.5:5:5:5, and then pre-cooled to 10±3°C. The resin was slowly added into the cleavage reagent while stirring until the temperature of the reaction system was stable, and the reaction proceeded with stirring for 4 h while controlling the temperature at 18±3°C.

The cleavage solution was filtered, concentrated to thick, and precipitated with a 12-15-fold concentrate volume of methyl tert-butyl ether. The precipitate was filtered, washed, and then dried at room temperature under reduced pressure to obtain 9.8 g of crude cyclopeptide.

### Step 8. Purification by chromatography

1/6 of the 9.8 g of the crude cyclopeptide was dissolved in 5% acetonitrile and 2% aqueous ammonia solution and purified by high-performance liquid chromatography using C18 silica gel matrix filler, and the fractions with a purity of >90% were collected and combined. The purification chromatography systems are listed as follows:

| Procedures | Aqueous phase | Organic phase | Gradient |
|---|---|---|---|
| First purification | 0.2% TFA | Acetonitrile | B% (0-50 min): 30%-55% |
| Second purification | 50 mmol/L diammonium hydrogen phosphate | Acetonitrile | B% (0-50 min): 31%-65% |
| Third purification | 0.2 mmol/L sodium hydroxide | Acetonitrile | B% (0-50 min): 41%-55% |

### Step 9. Concentration and lyophilization

The combined fraction obtained from the above step was concentrated to remove acetonitrile, and filtered through a 0.22 µm millipore filter membrane. The filtered solution was loaded into a vial lyophilizer and lyophilized to obtain 144 mg of target polypeptide product (with a purity of 96.69%). The molecular weight of the polypeptide was confirmed by ESI-MS, with the [M+4H]/4 calculated value of 1211.6 and the detected value of 1211.5.

### Example 39

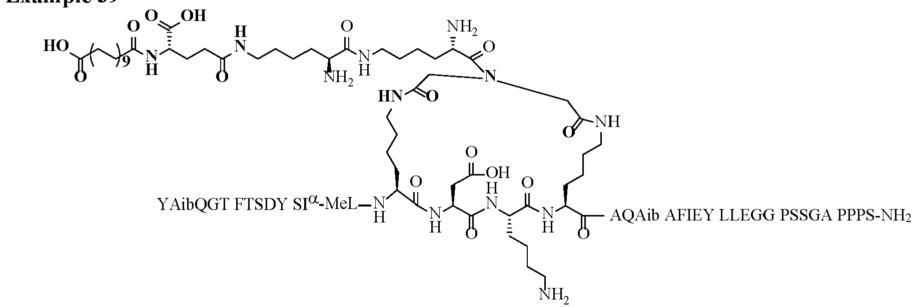

Referring to the synthesis of polypeptide in Example 21, the polypeptide shown in Example 39 was obtained, where the protecting group-containing amino acids used in the coupling of branched chain were sequentially Fmoc-Ida (OAll)-OH, N-Boc-N'-Fmoc- Lys-OH, N-Boc-N'-Fmoc- Lys-OH, Fmoc-Glu-OtBu, and 20-(tert-butoxy)-20-oxoicosanoic acid. The molecular weight of the polypeptide was confirmed by ESI-MS, with the [M+4H]/4 calculated value of 1239.7 and the detected value of 1239.7.

### Example 40

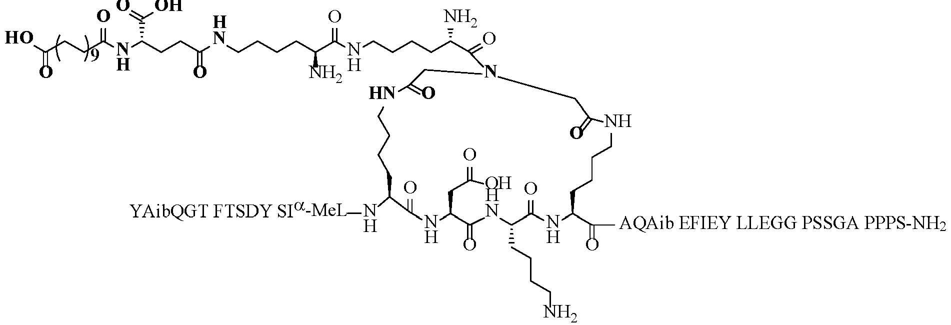

Referring to the synthesis of polypeptide in Example 21, the polypeptide shown in Example 40 was obtained, where the protecting group-containing amino acids used in the coupling of branched chain were sequentially Fmoc-Ida (OAll)-OH, N-Boc-N'-Fmoc-Lys-OH, N-Boc-N'-Fmoc-Lys-OH, Fmoc-Glu-OtBu, and 20-(tert-butoxy)-20-oxoicosanoic acid. The molecular weight of the polypeptide was confirmed by ESI-MS, with the [M+4H]/4 calculated value of 1254.2 and the detected value of 1254.1.

### Example 41

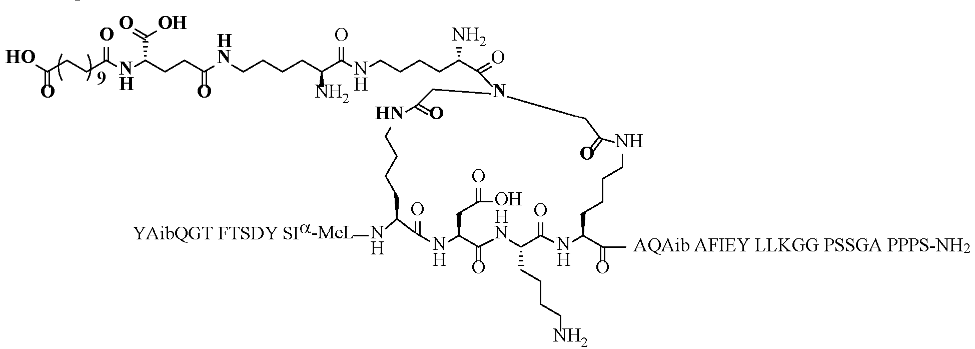

Referring to the synthesis of polypeptide in Example 38, the polypeptide as shown in Example 41 was obtained, where the protecting group-containing amino acids used in the coupling of branched chain were sequentially Fmoc-Ida (OAll)-OH, N-Boc-N'-Fmoc-Lys-OH, N-Boc-N'-Fmoc-Lys-OH, Fmoc-Glu-OtBu, and 20-(tert-butoxy)-20-oxoicosanoic acid. The molecular weight of the polypeptide was confirmed by ESI-MS, with the [M+4H]/4 calculated value of 1239.4 and the detected value of 1239.3.

### Example 42

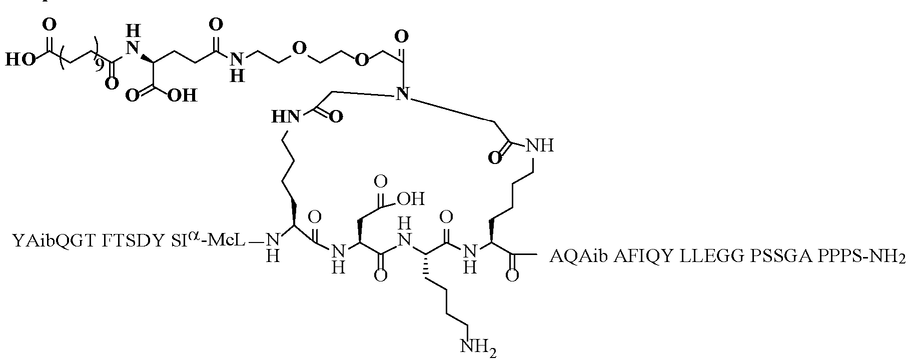

Referring to the synthesis of polypeptide in Example 21, the polypeptide as shown in Example 42 was obtained. The molecular weight of the polypeptide was confirmed by ESI-MS, with the [M+4H]/4 calculated value of 1211.6 and the detected value of 1211.5.

### Example 43

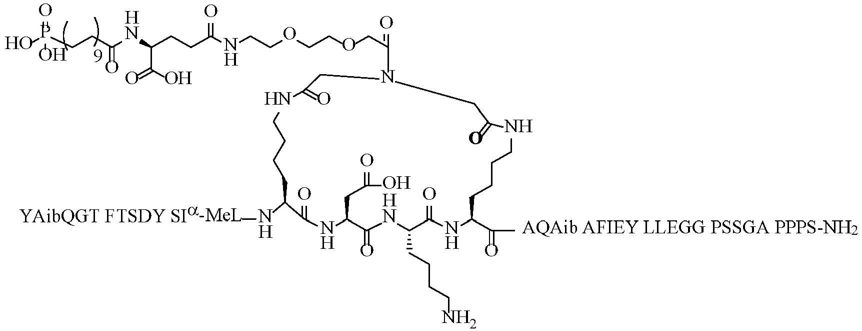

MBHA Resin was used as the starting resin, and conventional Fmoc-protected amino acids were used as the feedings. The condensing reagent used was a system of DIC/HOBt, with DMF as the main reaction solvent, and a 20% PIP-DMF solution as the Fmoc removal solvent. The peptide backbone was synthesized from the C-terminus to the N-terminus by condensing amino acids one by one. After the backbone synthesis was completed, the ε-amino group of Fmoc-Lys(Mtt)-OH at position A-26 was deprotected by removing the -Mtt group. Then, positions 1 to 4 of side chain ((2-2-oxoethyl)-glycine, AEEA, y Glu, and 19-(bis(benzyloxy)phosphoryl)nonadecanoic acid) were condensed sequentially. After that, the ε-amino protecting group of Lys at position A-23 and the carboxyl protecting group of position 1 of side chain ((2-2-oxoethyl)-glycine) were removed, and the condensing reagent was added to form a lactam ring, yielding the target peptide resin.

### Specific steps were as follows:

### Step 1: Activation and deprotection of resin

1. 15 g (5.1 mmol) of MBHA Resin (S=0.34 mmol/g) was added into the solid-phase reaction column, into which was added 225 mL of DCM and left for 20 min to allow the resin to fully swell. DCM was drained, and 150 mL of DMF was added while bubbling nitrogen to wash the resin. The above steps were repeated once again, and the solvent was then drained. 20% of piperidine/DMF (150 mL) was added into the reaction column for reaction, while nitrogen bubbling, for 10 min. The waste was drained until no liquid flowed out. 20% of piperidine/DMF (150 mL) was added into the reaction column again for further reaction, while nitrogen bubbling, for 10 min. A sample was taken and tested with ninhydrin, with the resin appearing greyish red. The reaction solution was drawn off from the reaction column which was then washed with DMF (150 mL) for 8 times, each time for 1 min, with the pH adjusted to 7, and the waste was drained until no liquid flowed out.

### Step 2: Coupling of Fmoc-Ser(tBu)-OH

1. 5.86 g (3.0 eq) of Fmoc-Ser(tBu)-OH, 2.57 g (4.0 eq) of DIC and 2.07 g (3.0 eq) of HOBt were weighed and dissolved in 80 mL of DMF, and then the mixture was added into the reaction column for reaction, while nitrogen bubbling, where the nitrogen was adjusted to make the resin bulge evenly.
2. After reaction at 25°C for 2.0 h, a sample was taken and tested with ninhydrin, with the resin appearing colorless or transparent.
3. The reaction solution was drawn off from the reaction column which was then washed with DMF (150 mL) for 5 times, each time for 1 min, and the waste was drained until no liquid flowed out.

### Coupling steps of the rest amino acids on the backbone

Referring to the coupling method of Fmoc-Ser(tBu)-OH and the equivalents of the feedings in Step 2, the amino acids were coupled sequentially to synthesize the amino acid sequence. The order and the protecting group-containing amino acids used are listed as follows:

| **Protecting group-containing amino acids** | **Note** |
|---|---|
| Fmoc-Pro-OH | |
| Fmoc-Pro-OH | |
| Fmoc-Pro-OH | |
| Fmoc-Ala-OH | |
| Fmoc-Gly-OH | |
| Fmoc-Ser(tBu)-OH | |
| Fmoc-Ser(tBu)-OH | |
| Fmoc-Pro-OH | |
| Fmoc-Gly-OH | |
| Fmoc-Gly-OH | Synthesis detected by HPLC |

AA-11 peptide was obtained.

### Step 3: Deprotection of AA-11 peptide

1. The synthesized AA-11 peptide was added into DMF (150 mL) for activation for 10 min, and drained until no liquid flowed out. 20% of piperidine/DMF (150 mL) was added into the reaction column for reaction, while nitrogen bubbling, for 10 min. The waste was drained until no liquid flowed out. 20% of piperidine/DMF (150 mL) was added into the reaction column again for further reaction, while nitrogen bubbling, for 10 min. A sample was taken and tested with ninhydrin, with the resin appearing dark yellow. The reaction solution was drawn off from the reaction column which was then washed with DMF (150 mL) for 8 times, each time for 1 min, with the pH adjusted to around 7, and the waste was drained until no liquid flowed out.

### Step 4: Coupling of Fmoc-Glu(tBu)-OH

1. 6.51 g (3.0 eq) of **Fmoc-Glu(tBu)-OH,** 2.57 g (4.0 eq) of DIC and 2.07 g (3.0 eq) of HOBt were weighed and dissolved in 150 mL of DMF, and then the mixture was added into the reaction column for reaction, while nitrogen bubbling, where the nitrogen was adjusted to make the resin bulge evenly.
2. After reaction at 25°C for 3.0 h, a sample was taken and tested with ninhydrin, with the resin appearing colorless or transparent.
3. The reaction solution was drawn off from the reaction column which was then washed with DMF (150 mL) for 5 times, each time for 1 min, and the waste was drained until no liquid flowed out.

### Coupling steps of the rest amino acids on the backbone

Referring to the coupling method of **Fmoc-Glu(tBu)-OH** and the order of the feedings in Step 4, the amino acids were coupled sequentially to synthesize the amino acid sequence. The order and the protecting group-containing amino acids used are listed as follows:

| **Protecting group-containing amino acids** | **Note** |
|---|---|
| Fmoc-Leu-OH | AA-13 peptide |
| Fmoc-Leu-OH | |
| Fmoc-Tyr(tBu)-OH | |
| Fmoc-Glu(OtBu)-OH | |
| Fmoc-Ile-OH | |
| Fmoc-Phe-OH | |
| Fmoc-Ala-OH | |
| Fmoc-Aib-OH | |
| Fmoc-Gln(Trt)-OH | Monitored by HPLC |
| Fmoc-Ala-OH | |
| Fmoc-Lys(Alloc)-OH | |
| Fmoc-Lys(Boc)-OH | |
| Fmoc-Asp(OtBu)-OH | |
| Fmoc-Lys(Mtt)-OH | |
| Fmoc-α-Me-Leu-OH | Monitored by HPLC |
| Fmoc-Ile-OH | Monitored by HPLC |
| Fmoc-Ser(tBu)-OH | |
| Fmoc-Tyr(tBu)-OH | |
| Fmoc-Asp(OtBu)-OH | |
| Fmoc-Ser(tBu)-OH | |
| Fmoc-Thr(tBu)-OH | |
| Fmoc-Phe-OH | |
| Fmoc-Thr(tBu)-OH | |
| Fmoc-Gly-OH | |
| Fmoc-Gln(Trt)-OH | |
| Boc-Tyr(tBu)-Aib-OH | Monitored by HPLC |

### Step 5. Coupling of branched chain

1. 300 mL of DCM was added into the reaction column for washing the resin 4 times. After draining the solvent, 150 mL of 20% hexafluoroisopropanol (HFIP)-DCM solution was added, and the reaction was performed while nitrogen bubbling for 5 times, each time for 30 min.
2. The waste was drained until no liquid flowed out. A sample was taken and tested with ninhydrin, with the resin appearing greyish green.
3. 150 mL of DCM and 150 mL of DMF were sequentially added for cross-washing the resin 4 times. The waste was drained until no liquid flowed out.
4. 10.08 g (5.0 eq) of Fmoc-Ida(Oall)-OH, 3.86 g (6.0 eq) of DIC and 3.45 g (5.0 eq) of HOBt were weighed and dissolved in 150 mL of DMF, and then the mixture was added into the reaction column for reaction, while nitrogen bubbling, where the nitrogen was adjusted to make the resin bulge evenly.
5. After reaction at 25°C for 3.0 h, a sample was taken and tested with ninhydrin, with the resin appearing colorless or transparent.
6. The reaction solution was drawn off from the reaction column which was then washed with DMF (180 mL) for 5 times, each time for 1 min, and the waste was drained until no liquid flowed out.

### The rest steps of coupling branched chain

Referring to the coupling method of Fmoc-Ida(Oall)-OH and the equivalents of the feedings in Step 5, the compounds listed in the table below were coupled sequentially:

| **Protecting group-containing amino acids** | **Note** |
|---|---|
| Fmoc-AEEA-OH | |
| Fmoc-Glu-OtBu | |
| 19-(bis(benzyloxy)phosphoryl)nonadecanoic acid | Monitored by HPLC |

### Step 6. Cyclization

1. 250 mL of DMF and 250 mL of DCM were sequentially added into the reaction column for cross-washing the resin 4 times. The waste was drained until no liquid flowed out.
2. 300 mL of a mixed solvent of DCM: CH₃CN: NMP: DEA at a volume ratio of 3:3:2:2 was prepared and added into the reaction column, and 1.59 g (0.27 eq) of Pd(PPh₃)₄ was then added, and the reaction was performed while nitrogen bubbling for 30 min. The waste was drained until no liquid flowed out.
3. A sample was taken and tested with ninhydrin, with the resin appearing yellowish purple.
4. 250 mL of DMF and 250 mL of DCM were sequentially added for cross-washing the resin 8 times.
5. 3.86 g (6.0 eq) of DIC and 3.45 g (5.0 eq) of HOBt were dissolved in 250 mL of DMF, and the mixture was added into the reaction column, where the reaction was performed while nitrogen bubbling for 23 h. A sample was taken and tested with ninhydrin, with the resin appearing colorless.
6. 250 mL of DMF and 250 mL of DCM were sequentially added for cross-washing the resin 8 times.
7. 200 mL of MeOH and 200 mL of DCM were sequentially added for cross-washing the resin 4 times.
8. The resin was taken out and dried in vacuum until the weight no longer decreased, and weighed, obtaining 33 g of peptide resin.

The obtained intermediate (peptide resin) has a structure formula as below:
Nε(Tyr(tBu)-Aib-Gln(Trt)-Gly-Thr(tBu)-Phe-Thr(tBu)-Ser(tBu)-Asp(OtBu)-Tyr(tBu)-Ser(tBu)-Ile-a-MeLe u-Lys¹⁴-Asp(OtBu)-Lys(Boc)-Lys¹⁷-Ala-Gln(Trt)-Aib-Ala-Phe-Ile-Glu(OtBu)-Tyr(tBu)-Leu-Leu-Glu(OtBu)-Gl y-Gly-Pro-Ser(tBu)-Ser(tBu)-Gly-Ala-Pro-Pro-Pro-Ser(tBu)-Rink Linker -MBHA Resin (14,17(Nε(HOOC-(CH₂)₁₈-PO(OBn)₂) -Glu(ε-OtBu)- (AEEA)-(2-2-oxoethyl)glycine))

### Step 7. Cleavage of peptide resin

500 mL of a cleavage reagent was formulated with trifluoroacetic acid (TFA), purified water (H₂O), triisopropylsilane (TIS), 3-(tritylthio)propionic acid (Mpa(Trt)-OH), and phenol (ArOH) at a volume ratio of TFA: H₂O: Mpa(Trt)-OH: TIS: ArOH=82.5:2.5:5:5:5, and then pre-cooled to 10±3°C. The resin was slowly added into the cleavage reagent while stirring until the temperature of the reaction system was stable, and the reaction proceeded with stirring for 8 h while controlling the temperature at 18±3°C. The cleavage solution was filtered, concentrated to thick, and precipitated with a 12-15-fold concentrate volume of methyl tert-butyl ether. The precipitate was filtered, washed, and then dried at room temperature under reduced pressure to obtain 8.8 g of crude cyclopeptide.

### Step 8. Purification by chromatography

2.0 g of the crude cyclopeptide was dissolved in 5% acetonitrile and 2% aqueous ammonia solution and purified by high-performance liquid chromatography using C18 silica gel matrix filler, and the fractions with a purity of >90% were collected and combined. The purification chromatography systems are listed as follows:

| Procedures | Aqueous phase | Organic phase | Gradient |
|---|---|---|---|
| First purification | 0.2% TFA | Acetonitrile | B% (0-50 min): 30%-55% |
| Second purification | 50 mmol/L diammonium hydrogen phosphate | Acetonitrile | B% (0-50 min): 31%-65% |
| Third purification | 0.2 mmol/L sodium hydroxide | Acetonitrile | B% (0-50 min): 41%-55% |

### Step 9. Concentration and lyophilization

The combined fraction obtained from the above step was concentrated to remove acetonitrile, and filtered through a 0.22 µm millipore filter membrane. The filtered solution was loaded into a vial lyophilizer and lyophilized to obtain 28 mg of target polypeptide product (with a purity of 97.67%). The molecular weight of the polypeptide was confirmed by ESI-MS, with the [M] calculated value of 1220.9 and the [M+H] detected value of 1219.8.

### Example 44

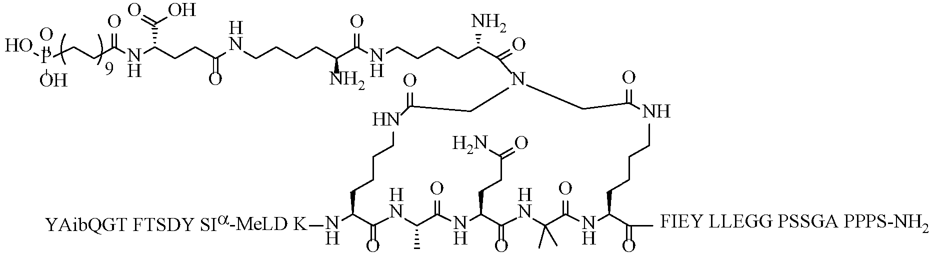

Referring to the synthesis of polypeptide in Example 1, the polypeptide shown in Example 44 was obtained. The molecular weight of the polypeptide was confirmed by ESI-MS, with the [M+4H]/4 calculated value of 1259.2 and the detected value of 1258.0.

### Example 45

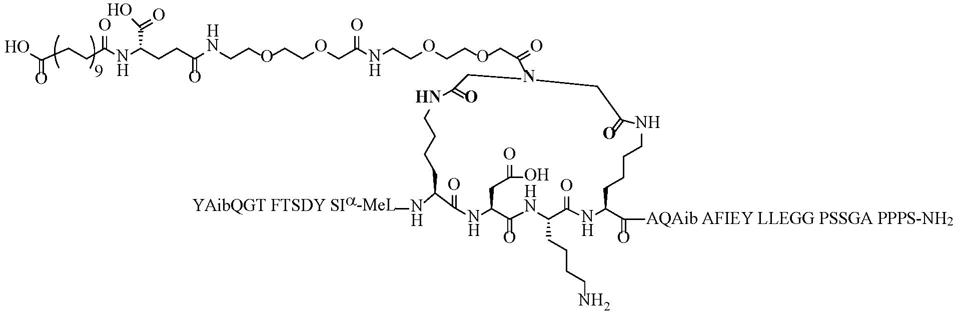

Referring to the synthesis of polypeptide in Example 21, the polypeptide shown in Example 45 was obtained. The molecular weight of the polypeptide was confirmed by ESI-MS, with the [M+4H]/4 calculated value of 1248.2 and the detected value of 1248.1.

### Example 46

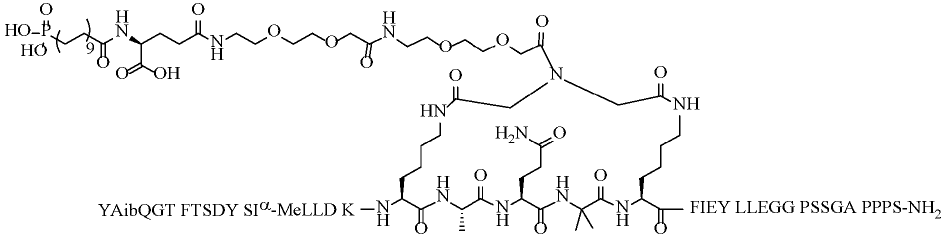

Referring to the synthesis of polypeptide in Example 1, the polypeptide as shown in Example 46 was obtained, where the protecting group-containing amino acids used in the coupling of branched chain were sequentially Fmoc-Ida(Oall)-OH, Fmoc-AEEA-OH, Fmoc-AEEA-OH, Fmoc-Glu-OtBu, and 19-(bis(benzyloxy)phosphoryl)nonadecanoic acid. The molecular weight of the polypeptide was confirmed by ESI-MS, with the [M] calculated value of 5067.61 and the detected value of 5066.61.

### Example 47

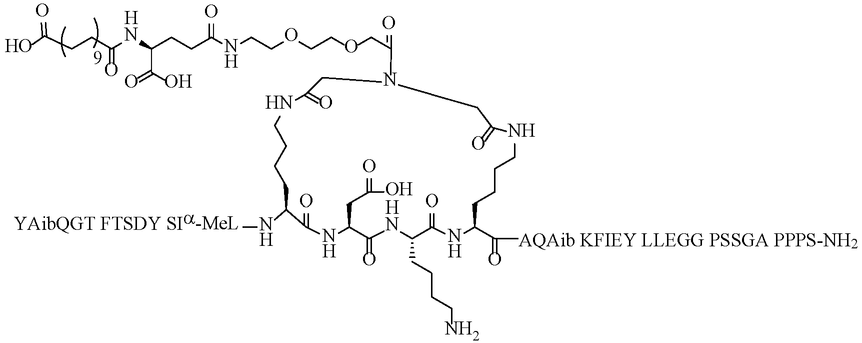

Referring to the synthesis of polypeptide in Example 21, the polypeptide as shown in Example 47 was obtained. The molecular weight of the polypeptide was confirmed by ESI-MS, with the [M] calculated value of 4900.56 and the detected value of 4900.62.

### Example 48

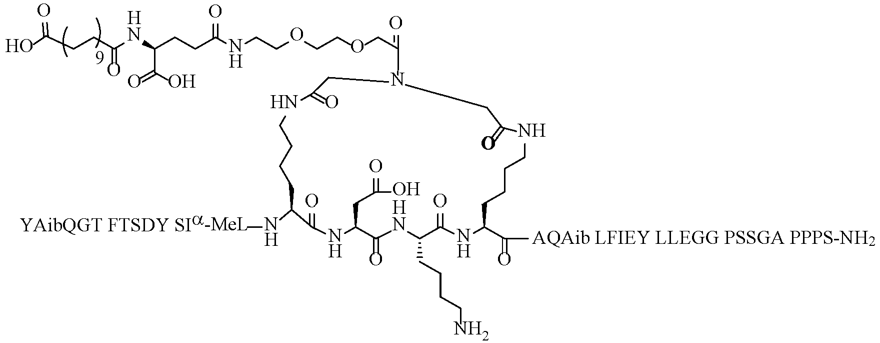

Referring to the synthesis of polypeptide in Example 21, the polypeptide as shown in Example 48 was obtained. The molecular weight of the polypeptide was confirmed by ESI-MS, with the [M] calculated value of 4885.56 and the detected value of 4885.50.

### Example 49

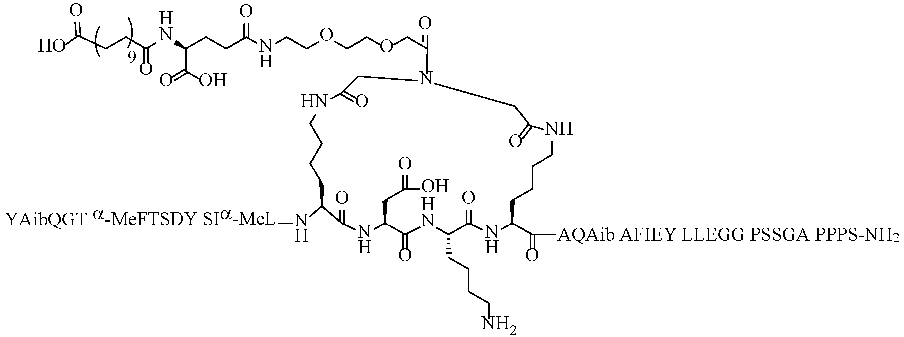

Referring to the synthesis of polypeptide in Example 21, the polypeptide as shown in Example 49 was obtained. The molecular weight of the polypeptide was confirmed by ESI-MS, with the [M] calculated value of 4857.51 and the detected value of 4857.55.

### Example 50

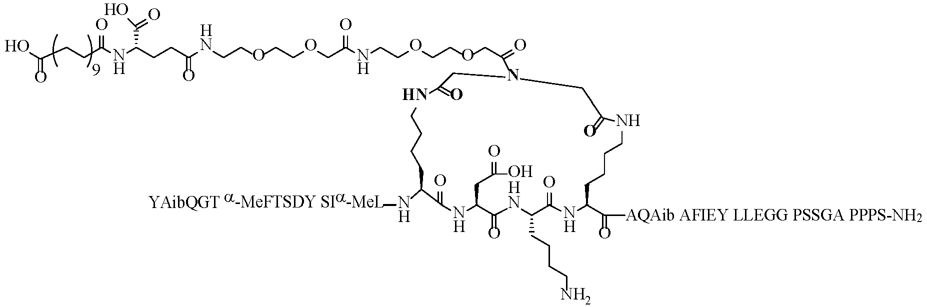

Referring to the synthesis of polypeptide in Example 21, the polypeptide as shown in Example 50 was obtained. The molecular weight of the polypeptide was confirmed by ESI-MS, with the [M] calculated value of 5002.59 and the detected value of 5002.62.

### Example 51

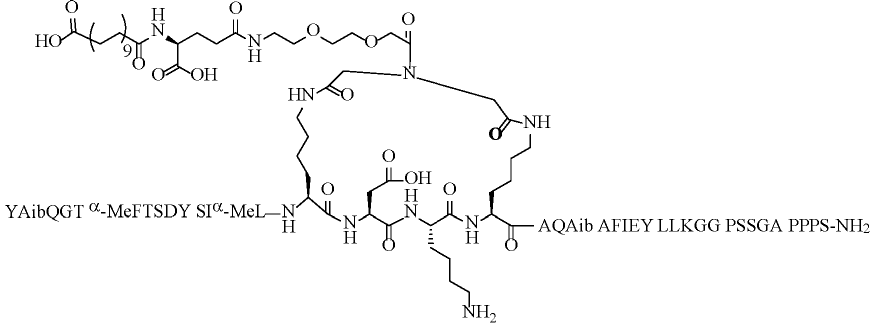

Referring to the synthesis of polypeptide in Example 21, the polypeptide as shown in Example 51 was obtained. The molecular weight of the polypeptide was confirmed by ESI-MS, with the [M] calculated value of 4856.57 and the detected value of 4856.48.

### Example 52

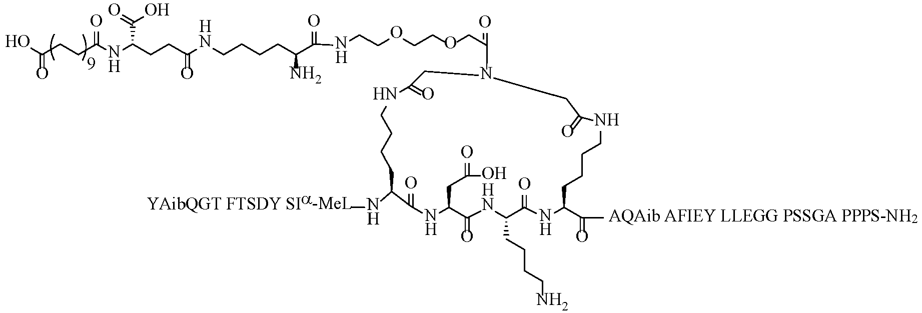

MBHA Resin was used as the starting resin, and conventional Fmoc-protected amino acids were used as the feedings. The condensing reagent used was a system of DIC/HOBt, with DMF as the main reaction solvent, and a 20% PIP-DMF solution as the Fmoc removal solvent. The peptide backbone was synthesized from the C-terminus to the N-terminus by condensing amino acids one by one. After the backbone synthesis was completed, the ε-amino group of Fmoc-Lys(Mtt)-OH at position A-26 was deprotected by removing the -Mtt group. Then, positions 1 to 5 of side chain ((2-2-oxoethyl)-glycine, AEEA, N-Boc-N'-Fmoc-Lys-OH, γ Glu, and 20-(tert-butoxy)-20-oxoicosanoic acid) were condensed sequentially. After that, the ε-amino protecting group of Lys at position A-23 and the carboxyl protecting group of position 1 of side chain ((2-2-oxoethyl)-glycine) were removed, and the condensing reagent was added to form a lactam ring, yielding the target peptide resin.

### Specific steps were as follows:

### Step 1: Activation and deprotection of resin

1. 15 g (5.1 mmol) of MBHA Resin (S=0.34 mmol/g) was added into the solid-phase reaction column, into which was added 225 mL of DCM and left for 20 min to allow the resin to fully swell. DCM was drained, and 150 mL of DMF was added while bubbling nitrogen to wash the resin. The above steps were repeated once again, and the solvent was then drained. 20% of piperidine/DMF (150 mL) was added into the reaction column for reaction, while nitrogen bubbling, for 10 min. The waste was drained until no liquid flowed out. 20% of piperidine/DMF (150 mL) was added into the reaction column again for further reaction, while nitrogen bubbling, for 10 min. A sample was taken and tested with ninhydrin, with the resin appearing greyish red. The reaction solution was drawn off from the reaction column which was then washed with DMF (150 mL) for 8 times, each time for 1 min, with the pH adjusted to 7, and the waste was drained until no liquid flowed out.

### Step 2: Coupling of Fmoc-Ser(tBu)-OH

1. 5.86 g (3.0 eq) of Fmoc-Ser(tBu)-OH, 2.57 g (4.0 eq) of DIC and 2.07 g (3.0 eq) of HOBt were weighed and dissolved in 80 mL of DMF, and then the mixture was added into the reaction column for reaction, while nitrogen bubbling, where the nitrogen was adjusted to make the resin bulge evenly.
2. After reaction at 25°C for 2.0 h, a sample was taken and tested with ninhydrin, with the resin appearing colorless or transparent.
3. The reaction solution was drawn off from the reaction column which was then washed with DMF (150 mL) for 5 times, each time for 1 min, and the waste was drained until no liquid flowed out.

### Coupling steps of the rest amino acids on the backbone

Referring to the coupling method of Fmoc-Ser(tBu)-OH and the equivalents of the feedings in Step 2, the amino acids were coupled sequentially to synthesize the amino acid sequence. The order and the protecting group-containing amino acids used are listed as follows:

| **Protecting group-containing amino acids** | **Note** |
|---|---|
| Fmoc-Pro-OH | |
| Fmoc-Pro-OH | |
| Fmoc-Pro-OH | |
| Fmoc-Ala-OH | |
| Fmoc-Gly-OH | |
| Fmoc-Ser(tBu)-OH | |
| Fmoc-Ser(tBu)-OH | |
| Fmoc-Pro-OH | |
| Fmoc-Gly-OH | |
| Fmoc-Gly-OH | Synthesis detected by HPLC |

AA-11 peptide was obtained.

### Step 3: Deprotection of AA-11 peptide

1. Into the synthesized AA-11 peptide was added DMF (150 mL) for activation for 10 min, and drained until no liquid flowed out. 20% of piperidine/DMF (150 mL) was added into the reaction column for reaction, while nitrogen bubbling, for 10 min. The waste was drained until no liquid flowed out. 20% of piperidine/DMF (150 mL) was added into the reaction column again for further reaction, while nitrogen bubbling, for 10 min. A sample was taken and tested with ninhydrin, with the resin appearing dark yellow. The reaction solution was drawn off from the reaction column which was then washed with DMF (150 mL) for 8 times, each time for 1 min, with the pH adjusted to around 7, and the waste was drained until no liquid flowed out.

### Step 4: Coupling of Fmoc-Glu(tBu)-OH

1. 6.51 g (3.0 eq) of **Fmoc-Glu(tBu)-OH,** 2.57 g (4.0 eq) of DIC and 2.07 g (3.0 eq) of HOBt were weighed and dissolved in 150 mL of DMF, and then the mixture was added into the reaction column for reaction, while nitrogen bubbling, where the nitrogen was adjusted to make the resin bulge evenly.
2. After reaction at 25°C for 3.0 h, a sample was taken and tested with ninhydrin, with the resin appearing colorless or transparent.
3. The reaction solution was drawn off from the reaction column which was then washed with DMF (150 mL) for 5 times, each time for 1 min, and the waste was drained until no liquid flowed out.

### Coupling steps of the rest amino acids on the backbone

Referring to the coupling method of **Fmoc-Glu(tBu)-OH** and the order of the feedings in Step 4, the amino acids were coupled sequentially to synthesize the amino acid sequence. The order and the protecting group-containing amino acids used are listed as follows:

| **Protecting group-containing amino acids** | **Note** |
|---|---|
| Fmoc-Leu-OH | AA-13 peptide |
| Fmoc-Leu-OH | |
| Fmoc-Tyr(tBu)-OH | |
| Fmoc-Glu(OtBu)-OH. | |
| Fmoc-Ile-OH | |
| Fmoc-Phe-OH | |
| Fmoc-Ala-OH | |
| Fmoc-Aib-OH | |
| Fmoc-Gln(Trt)-OH | Monitored by HPLC |
| Fmoc-Ala-OH | |
| Fmoc-Lys(Alloc)-OH | |
| Fmoc-Lys(Boc)-OH | |
| Fmoc-Asp(OtBu)-OH | |
| Fmoc-Lys(Mtt)-OH | |
| Fmoc-α-Me-Leu-OH | Monitored by HPLC |
| Fmoc-Ile-OH | Monitored by HPLC |
| Fmoc-Ser(tBu)-OH | |
| Fmoc-Tyr(tBu)-OH | |
| Fmoc-Asp(OtBu)-OH | |
| Fmoc-Ser(tBu)-OH | |
| Fmoc-Thr(tBu)-OH | |
| Fmoc-Phe-OH | |
| Fmoc-Thr(tBu)-OH | |
| Fmoc-Gly-OH | |
| Fmoc-Gln(Trt)-OH | |
| Boc-Tyr(tBu)-Aib-OH | Monitored by HPLC |

### Step 5. Coupling of branched chain

1. 300 mL of DCM was added into the reaction column for washing the resin 4 times. After draining the solvent, 150 mL of 20% hexafluoroisopropanol (HFIP)-DCM solution was added, and the reaction was performed while nitrogen bubbling for 5 times, each time for 30 min.
2. The waste was drained until no liquid flowed out. A sample was taken and tested with ninhydrin, with the resin appearing greyish green.
3. 150 mL of DCM and 150 mL of DMF were sequentially added for cross-washing the resin 4 times. The waste was drained until no liquid flowed out.
4. 10.08 g (5.0 eq) of Fmoc-Ida(Oall)-OH, 3.86 g (6.0 eq) of DIC and 3.45 g (5.0 eq) of HOBt were weighed and dissolved in 150 mL of DMF, and then the mixture was added into the reaction column for reaction, while nitrogen bubbling, where the nitrogen was adjusted to make the resin bulge evenly.
5. After reaction at 25°C for 3.0 h, a sample was taken and tested with ninhydrin, with the resin appearing colorless or transparent.
6. The reaction solution was drawn off from the reaction column which was then washed with DMF (180 mL) for 5 times, each time for 1 min, and the waste was drained until no liquid flowed out.

### The rest steps of coupling branched chain

Referring to the coupling method of Fmoc-Ida(Oall)-OH and the equivalents of the feedings in Step 5, the compounds listed in the table below were coupled sequentially:

| **Protecting group-containing amino acids** | **Note** |
|---|---|
| Fmoc-AEEA-OH | |
| N-Boc-N'-Fmoc-Lys-OH | |
| Fmoc-Glu-OtBu | |
| 20-(tert-butoxy)-20-oxoicosanoic acid | Monitored by HPLC |

### Step 6. Cyclization

1. 250 mL of DMF and 250 mL of DCM were sequentially added into the reaction column for cross-washing the resin 4 times. The waste was drained until no liquid flowed out.
2. 300 mL of a mixed solvent of DCM: CH₃CN: NMP: DEA at a volume ratio of 3:3:2:2 was prepared and added into the reaction column, and 1.59 g (0.27 eq) of Pd(PPh₃)₄ was then added, and the reaction was performed while nitrogen bubbling for 30 min. The waste was drained until no liquid flowed out.
3. A sample was taken and tested with ninhydrin, with the resin appearing yellowish purple.
4. 250 mL of DMF and 250 mL of DCM were sequentially added for cross-washing the resin 8 times.
5. 3.86 g (6.0 eq) of DIC and 3.45 g (5.0 eq) of HOBt were dissolved in 250 mL of DMF, and the mixture was added into the reaction column, where the reaction was performed while nitrogen bubbling for 22 h. A sample was taken and tested with ninhydrin, with the resin appearing colorless.
6. 250 mL of DMF and 250 mL of DCM were sequentially added for cross-washing the resin 8 times.
7. 200 mL of MeOH and 200 mL of DCM were sequentially added for cross-washing the resin 4 times.
8. The resin was taken out and dried in vacuum until the weight no longer decreased, and weighed, obtaining 25.5 g of peptide resin.

The obtained intermediate (peptide resin) has a structure formula as below:
Nε(Boc-Tyr(tBu)-Aib-Gln(Trt)-Gly-Thr(tBu)-Phe-Thr(tBu)-Ser(tBu)-Asp(OtBu)-Tyr(tBu)-Ser(tBu)-Ile-a-MeLeu-Lys¹⁴-Asp(OtBu)-Lys(Boc)-Lys¹⁷-Ala-Gln(Trt)-Aib-Ala-Phe-Ile-Glu(OtBu)-Tyr(tBu)-Leu-Leu-Glu(OtB u)-Gly-Gly-Pro-Ser(tBu)-Ser(tBu)-Gly-Ala-Pro-Pro-Pro-Ser(tBu)-Rink Linker -MBHA Resin (14,17(Nε(HOOC-(CH₂)₁₈-COOtBu) -Boc(Lys)-Glu(ε-OtBu)-(AEEA)-(2-2-oxoethyl)glycine))

### Step 7. Cleavage of peptide resin

500 mL of a cleavage reagent was formulated with trifluoroacetic acid (TFA), purified water (H₂O), triisopropylsilane (TIS), 3-(tritylthio)propionic acid (Mpa(Trt)-OH), and phenol (ArOH) at a volume ratio of TFA: HzO: Mpa(Trt)-OH: TIS: ArOH=82.5:2.5:5:5:5, and then pre-cooled to 10±3°C. The resin was slowly added into the cleavage reagent while stirring until the temperature of the reaction system was stable, and the reaction proceeded with stirring for 4 h while controlling the temperature at 18±3°C. The cleavage solution was filtered, concentrated to thick, and precipitated with a 12-15-fold concentrate volume of methyl tert-butyl ether. The precipitate was filtered, washed, and then dried at room temperature under reduced pressure to obtain 8.6 g of crude cyclopeptide.

### Step 8. Purification by chromatography

8.6 g of the crude cyclopeptide was dissolved in 5% acetonitrile and 2% aqueous ammonia solution and purified by high-performance liquid chromatography using C18 silica gel matrix filler, and the fractions with a purity of >90% were collected and combined. The purification chromatography systems are listed as follows:

| Procedures | Aqueous phase | Organic phase | Gradient |
|---|---|---|---|
| First purification | 0.2% TFA | Acetonitrile | B% (0-50 min): 30%-55% |
| Second purification | 50 mmol/L diammonium hydrogen phosphate | Acetonitrile | B% (0-50 min): 31%-65% |
| Third purification | 0.2 mmol/L sodium hydroxide | Acetonitrile | B% (0-50 min): 41%-55% |

### Step 9. Concentration and lyophilization

The combined fraction obtained from the above step was concentrated to remove acetonitrile, and filtered through a 0.22 µm millipore filter membrane. The filtered solution was loaded into a vial lyophilizer and lyophilized to obtain 1.1 g of target polypeptide product (with a purity of 99.12%). The molecular weight of the polypeptide was confirmed by ESI-MS, with the [M] calculated value of 4972.62 and the detected value of 4971.42.

### Example 53

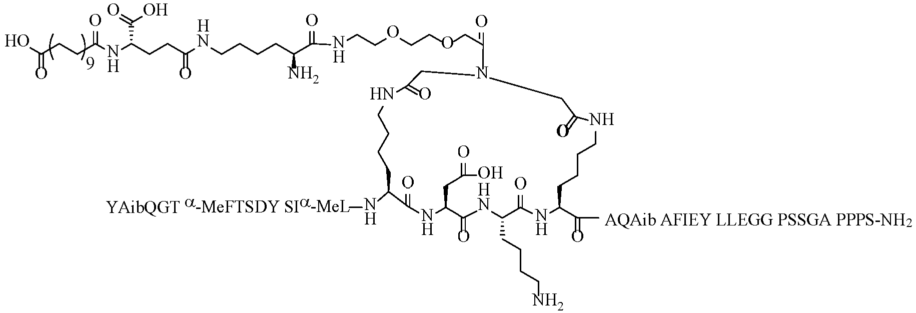

Referring to the synthesis of polypeptide in Example 52, the polypeptide shown in Example 53 was obtained. The molecular weight of the polypeptide was confirmed by ESI-MS, with the [M] calculated value of 4985.61 and the detected value of 4985.42.

### Example 54

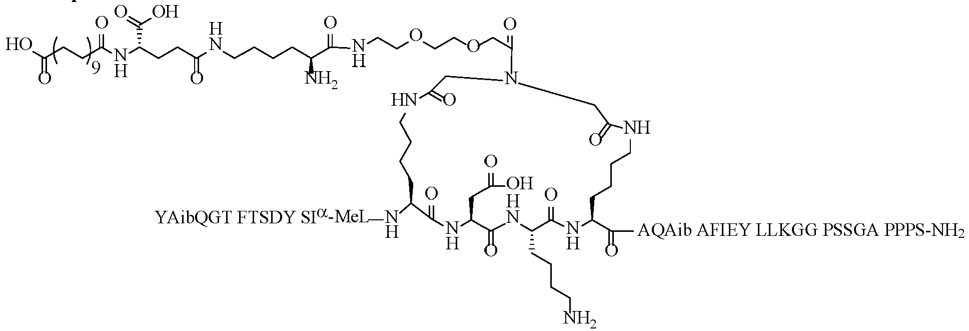

Referring to the synthesis of polypeptide in Example 52, the polypeptide shown in Example 54 was obtained. The molecular weight of the polypeptide was confirmed by ESI-MS, with the [M] calculated value of 4970.65 and the detected value of 4969.65.

### Biological test data

### Test 1: In vitro GLP-1R/GIPR/GCGR agonistic activity test

### Method 1:

### A: Main Materials:

### 1) Cell lines

The cell lines were constructed by Pharmaron Inc. See details in the table below.

| Target | Host cell | Clone |
|---|---|---|
| GLP-1R | HEK293 | N/A |
| GCGR | HEK293 | N/A |
| GIPR | CHO | N/A |

### 2) Reagents and Consumables

| Name | Lot No. | Item No. | Manufacturer |
|---|---|---|---|
| cAMP kit | 3053757 | TRF0263 | Perkin Elmer |
| 1M HEPES | 2458415 | 15630-080 | Gibco |
| Hanks' Balanced Salt Solution (HBSS) | 2193007 | 14025-076 | Gibco |
| Bovine Serum Albumin (BSA) | 2888494 | CR84-100 | Perkin Elmer |
| 3-isobutyl-1-methylxanthine (IBMX) | 102433079 | I5879 | Sigma |
| TrypLE | 2492769 | 12604-021 | Invitrogen |
| 96-well plate | 1363672 | 249944 | Thermo |
| CulturPlate-384 | 8213-22291 | 6007680 | Perkin Elmer |

### 3) Instruments

| Name | Model | Manufacturer |
|---|---|---|
| EnVision | 2015 | Perkin Elmer |
| Automatic fluorescent cell counter | Countess III | Invitrogen |
| Centrifuge | CLT55 | Cence |

### B. Methods

### I) Experimental materials

### Experimental buffers

| **Reagents** | **Storage concentration** | **Volume** | **Final concentration** |
|---|---|---|---|
| Hanks' Balanced Salt Solution | 1× | 38.7 mL | ≈1× |
| HEPES buffer | 1 mol/L | 800 µL | 20 mmol/L |
| 7.5% casein solution (HEPES) | 7.5% | 533 µL | 0.10%/ |
| 3-isobutyl-1-methylxanthine (IBMX) | 1 mol/L | 20 µL | 0.5 mmol/L |

### Preparation of assay reagents

| **Reagents** | **Storage concentration** | **Volume** | **Final concentration** |
|---|---|---|---|
| Cell lysis buffer | 1× | 9.73 mL | ≈1× |
| Eu-cAMP solution | 50× | 200 µL | 1× |
| Ulight-anti-cAMP solution | 150× | 66.7 µL | 1× |

### II) Experimental Methods

### a) Preparation of compound plates

The test compounds were diluted at a 5-fold dilution factor in 10 points, starting from an initial concentration of 200 nM.

### b) Preparation of cell suspension

1) The cell lines were cultured respectively in complete medium at 37°C and 5% CO₂.
2) After digestion with TrypLE, the cells were re-suspended in experimental buffer respectively, and inoculated in a CulturPlate-384 cell culture plate at a density of 2000 cells/well and at a volume of 15 µL/well.

### c) Determination of agonistic activity

1) Eu-cAMP tracer was freeze-thawed and diluted 50 fold with detection buffer; and Ulight-anti-cAMP was freeze-thawed and diluted 150 fold with detection buffer.
2) 5 µL of the test compounds were added respectively into experimental wells of the CulturPlate-384 cell culture plate, which was centrifuged at 200 g for 30 s, and left for stand at 37°C for 30 min.
3) 10 µL of Eu-cAMP tracer was added and then 10 µL of Uligh-anti-cAMP was added into experimental wells respectively.
4) The reaction plate was centrifuged at 200g at room temperature for 30 s and left for stand at 25°C for 1h.
5) Envision 2105 was used to collect data, with excitation light at 340 nm and emission light at 665 nm and 615 nm.

### C. Experimental results

| **Compounds** | **Activity test results** | | |
|---|---|---|---|
| | **GLP-1 EC₅₀ (nM)** | **GIP-1 EC₅₀ (nM)** | **GCG EC₅₀ (nM)** |
| Human GLP-1 (7-37) | 0.001 | NA | NA |
| Human GIP (1-42) | NA | 0.013 | NA |
| Human Glucagon | NA | NA | 0.001 |
| LY3437943 | 0.014 | 0.001 | 0.031 |
| Example 1 | 0.009 | 0.001 | 0.024 |
| Example 2 | 0.009 | 0.001 | 0.279 |
| Example 3 | 0.025 | 0.003 | 1.679 |
| Example 4 | 0.023 | 0.016 | 0.143 |
| Example 5 | 0.029 | 0.007 | 0.012 |
| Example 6 | 0.005 | 0.0005 | 0.017 |
| Example 7 | 0.005 | 0.0003 | 0.061 |
| Example 8 | 0.004 | 0.0004 | 0.012 |
| Example 9 | 0.005 | 0.0004 | 0.022 |
| Example 10 | 0.009 | 0.0003 | 0.057 |
| Example 11 | 0.006 | 0.0003 | 0.129 |
| Example 12 | 0.002 | 0.0001 | 0.007 |
| Example 14 | 0.001 | 0.0001 | 0.028 |
| Example 16 | 0.002 | 0.0001 | 0.042 |
| Example 17 | 0.001 | 0.0001 | 0.016 |
| Example 18 | 0.008 | 0.0004 | 0.052 |
| Example 21 | 0.020 | 0.0008 | 0.039 |
| Example 22 | 0.009 | 0.0004 | 0.034 |
| Example 23 | 0.007 | 0.0004 | 0.096 |
| Example 26 | 0.014 | 0.0005 | 0.110 |
| Example 28 | 0.011 | 0.0008 | 0.110 |
| Example 38 | 0.002 | 0.0002 | 0.018 |
| Example 41 | 0.003 | 0.0003 | 0.015 |
| Example 42 | 0.007 | 0.0005 | 0.025 |
| Example 43 | 0.015 | 0.0009 | 0.042 |
| Example 45 | 0.005 | 0.0001 | 0.029 |
| Example 51 | 0.001 | 0.0002 | 0.025 |
| Example 52 | 0.003 | 0.0001 | 0.025 |
| Example 54 | 0.002 | 0.0004 | 0.02 |

**Conclusion:** The polypeptide compounds of the present disclosure exhibit strong agonistic activity on GLP-1R/GIPR/GCGR, which is overall superior to or not inferior to the activity of LY3437943, particularly, the introduction of the "staple" structure and its position have an effect on the activity of the polypeptide. Furthermore, in the repeated experiments and the tests with other compounds, the experimental results showed the same trend. In a preferred case, compared with the activity of the control LY3437943, the agonistic activity of the compounds of the present disclosure on GLP-1R is 0.05 to 5 times that of LY3437943, the agonistic activity on GIPR is 0.1~5 times that of LY3437943; and the agonistic activity on GCGR is 0.2~5 times that of LY3437943.

In summary, the compounds of the present disclosure have superior in vitro GLP-1R/GIPR/GCGR agonistic activity, which is overall superior to or not inferior to that of the control LY3437943.

### Test 2: Pharmacokinetic properties of the compounds in Cynomolgus macaques

### A. Experimental purpose

To test the pharmacokinetic profiles of the compounds in Cynomolgus macaques.

### B. Experimental procedure

Non-juvenile male cynomolgus macaques were used for experiment, 3 cynomolgus macaques for each compound. Whole blood was collected at time points of 1 h, 2 h, 4 h, 8 h, 16 h, 24 h, 48 h, 72 h, 120 h, 168 h and 240 h after administration, to prepare plasma. The drug concentration was analyzed by LC-MS/MS method, and the pharmacokinetic parameters were calculated using Phoenix WinNonlin software.

### C. Experimental results

The experimental results were as shown in the table below.

**Table: Pharmacokinetic test results in Cynomolgus macaques**

| **Compound No.** | **Cₘₐₓ (nM)** | **T_{1/2} (h)** | **Tₘₐₓ (h)** | **AUC₀₋₂₄₀ₕ (nM.hr)** | **AUC_{INF} (nM.hr)** |
|---|---|---|---|---|---|
| **LY-3437943** | 22.1 | 58.8 | 29.3 | 2699 | 2925 |
| **Example 7** | 24.3 | 84.1 | 29.3 | 3318 | 3956 |
| **Example 9** | 30.1 | 77.4 | 29.3 | 3232 | 3635 |
| **Example 21** | 31.2 | 105 | 40.0 | 4603 | 5937 |
| **Example 38** | 33.9 | 74.9 | 21.3 | 3878 | 4406 |
| **Example 52** | 36.2 | 97.8 | 29.3 | 5233 | 6607 |

**Conclusion:** According to the pharmacokinetic properties in Cynomolgus macaques, the compounds of the present disclosure have a half-life T_{1/2} of greater than 70 h, with a significantly superior half-life T_{1/2} and exposure relative to LY-3437943, and an overall advantage not inferior to that of LY-3437943.

Furthermore, in the repeated experiments, the experimental results obtained from testing other compounds showed the same trend. In a preferred case, compared with the **control** LY3437943, the half-life T_{1/2} and exposure AUC_{INF} of the compounds of the present disclosure are 1.1 to 2.5 times those of LY3437943, and the overall properties are superior to LY3437943.

Additionally, in further studies, the compounds of the present disclosure were pharmacokinetically tested in C57BL/6 mice and SD rats, the obtained experimental results showed the same trend as the pharmacokinetic properties in Cynomolgus macaques, exhibiting excellent pharmacokinetic properties in both mice and rats, and superior or comparable half-life T_{1/2} and exposure relative to the LY-3437943 compound.

### Test 3: Test of metabolic stability in human kidney S9

### A. Experimental purpose

The metabolic stability of the compounds in human kidney was evaluated to determine whether the drugs would be degraded by metabolic enzymes in the kidney to affect the efficacies and pharmacokinetics.

### B. Experimental procedure

1. Relevant solutions were formulated.
2. a) With cofactors (NADPH & UDPGA): NADPH and UDPGA were added during the incubation. The final concentrations of KS9 Fractions, NADPH and UDPGA were 1 mg/mL, 1 mM and 2 mM, respectively. b) Without cofactors (NADPH & UDPGA): H₂O was added into the culture solution. The final concentration of KS9 Fractions was 1 mg/mL. The mixture was pre-heated at 37°C for 10 min.
3. 20 µM of the control compound solution or 100 µM of the test compound solution was added to initiate the reaction. In this study, 7-hydroxy-courmarin and Raloxifene were used as the positive control. The final concentration of the positive control compounds was 0.2 µM, and the final concentration of the test compound was 1 µM. The culture solution was incubated in water bath at 37°C.
4. At 0.5, 5, 15, 30 and 60 min, volume aliquots were taken from the reaction solution and the reaction was stopped by adding acetonitrile. The samples were centrifuged for 30 min. The supernatant was taken and mixed with ultrapure water for subsequent LC-MS/MS analysis.
5. Data analysis: All calculation was conducted using Microsoft Excel. Peak areas were determined and slope values were calculated based on the extracted ion chromatograms.

### C. Experimental results

**Table: Test results of metabolic stability in kidney S9**

| **Compounds** | **Species** | **Assay Format** | ***in vitro* t_{1/2} (min)** | ***in vitro* CLᵢₙₜ (µL/min/mg)** |
|---|---|---|---|---|
| 7-Hydroxy-courmarin | Human | +Cofactors | 101.42 | 6.84 |
| Raloxifene | Human | +Cofactors | 93.37 | 7.77 |
| LY3437943 | Human | +Cofactors | > 184.78 | < 3.75 |
| Example 1 | Human | +Cofactors | > 184.78 | < 3.75 |
| Example 7 | Human | +Cofactors | > 184.78 | < 3.75 |
| Example 8 | Human | +Cofactors | > 184.78 | < 3.75 |
| Example 10 | Human | +Cofactors | > 184.78 | < 3.75 |
| Example 11 | Human | +Cofactors | > 184.78 | < 3.75 |
| Example 21 | Human | +Cofactors | > 184.78 | < 3.75 |

**Conclusion:** The compounds of the present disclosure exhibit excellent *in vitro* pharmacokinetic properties.

### Test 4: Study on pharmacodynamics of compounds in DIO obesity mouse model

Experimental purpose: To evaluate the effects of multiple administration of the test substance (compound) on body weight and glucolipid metabolism.

In this experiment, mice were used as test animals, which were induced with a high-fat diet (Research Diets: D 12492) from 4-5 weeks old for 8 weeks, and the animals were grouped according to their body weights and fasting blood glucose levels, with the average body weight of the model animals being 47.2 ± 0.3 g, the average body weight of the control animals being 27.9 ± 0.3 g, the average fasting blood glucose of the model animals being 8.8 ± 0.2 mmol/L, and the average fasting blood glucose of the control animals being 6.0±0.2 mmol/L. There were six animals in each group. The animals were administered immediately after grouping and were administered by subcutaneous injection every 3 days for 22 consecutive days. Both the normal control and model groups were given with vehicle (20 mM citrate buffer). Body weight and food intake were measured twice a week. Fasting blood glucose was tested on Days 10 and 20, OGTT test was performed on Day 20, and blood was collected on Day 23 to test the liver function indicators (ALT, AST, ALP) and serum lipid metabolism indicators (TC, TG, HDL, LDL). Liver tissues were taken to calculate organ indexes, and liver lipid metabolism indicators (hepatic TC, TG) were detected. The liver tissues were examined pathologically (HE, Oil Red) to evaluate the effects of the test substances on body weight and glucolipid metabolism in a high-fat diet-induced obesity mouse model.

The experimental data of each group of animals was described as mean ± standard error (X ± SEM). P<0.05 indicated the statistically significant difference. All statistical analysis was completed using Graphpad 8.0 software.

**Table: Effect of compounds on body weight change in DIO mice**

| **Compounds** | **Dosage** | **Change rate of body weight on Day 22 (%)** |
|---|---|---|
| Control | - | 3.40±1.08 |
| Model | - | 3.26±1.96 |
| Example 7 | 10 nmol/kg | -35.68±2.00 |
| Example 9 | 10 nmol/kg | -25.85±2.05 |
| Example 21 | 10 nmol/kg | -32.78±2.97 |
| LY3437943 | 10 nmol/kg | -29.19±2.94 |

**Conclusion:** It can be seen from the results in the table that, the compounds of the present disclosure have a significant weight reduction effect, which is comparable or superior to that of LY3437943. Additionally, the experimental results obtained from testing other compounds of the present disclosure also showed a weight reduction effect comparable or superior to that of LY3437943.

**Table: Effect of compounds on fasting blood glucose change in DIO mice**

| Blood glucose (mmol/L) | Control (n=6) | Model (n=6) | Example 7 (n=6) | Example 9 (n=6) | Example 21 (n=6) | LY3437943 (n=6) |
|---|---|---|---|---|---|---|
| Day 0 | 6.4±0.3 | 8.5±0.2 | 8.4±0.4 | 8.6±0.6 | 8.2±0.4 | 8.2±0.3 |
| Day 10 | 7.0±0.2 | 8.4±0.2 | 5.0±0.7 | 6.4±0.3 | 6.5±0.9 | 5.2±0.9 |
| Day 20 | 7.7±0.3 | 10.2±0.8 | 6.0±0.6 | 5.5±0.2 | 5.7±0.4 | 6.2±0.4 |

**Conclusion:** It can be seen from the table that, the compounds of the present disclosure have a significant hypoglycemic effect, which is comparable or superior to that of LY3437943. Additionally, the experimental results obtained from testing other compounds of the present disclosure also showed a hypoglycemic effect comparable or superior to that of LY3437943.

**Table: Effect of compounds on liver-body ratio change in DIO mice**

| / | Control (n=6) | Model (n=6) | Example 7 (n=6) | Example 9 (n=6) | Example 21 (n=6) | LY3437943 (n=6) |
|---|---|---|---|---|---|---|
| Liver-body ratio (g/g) | 0.040±0.001 | 0.037±0.004 | 0.032±0.002 | 0.029±0.001 | 0.032±0.002 | 0.032±0.003 |

**Conclusion:** It can be seen from the results in the table that, the compounds of the present disclosure have a significant effect of improving liver-body ratio, which is comparable or superior to that of LY3437943. Additionally, the experimental results obtained from testing other compounds of the present disclosure also showed an effect of improving liver-body ratio comparable or superior to that of LY3437943.

**Table: Effect of compounds on liver indicator change in DIO mice**

| | Control (n=6) | Model (n=6) | Example 7 (n=6) | Example 9 (n=6) | Example 21 (n=6) | LY3437943 (n=6) |
|---|---|---|---|---|---|---|
| Hepatic triglyceride/TG (mmol/mg) | 11.30±0.67 | 24.68±1.98 | 13.89±1.50 | 14.01.±0.99 | 12.58±1.27 | 14.57±0.98 |
| Hepatic cholesterol/TC (mmol/mg) | 6.74±0.18 | 6.09±0.77 | 8.90±0.46 | 8.85±0.25* | 9.71±0.49 | 8.11±0.67 |

**Conclusion:** It can be seen from the results in the table that, the compounds of the present disclosure have a significant effect of reducing hepatic triglyceride. Additionally, the experimental results obtained from testing other compounds of the present disclosure also showed an effect of reducing hepatic triglyceride which is comparable or superior to that of LY3437943.

Furthermore, following a similar experimental protocol, the compounds of Example 38 and Example 52 were tested, with the effect on body weight change in DIO mice as shown below.

**Table: Effect of compounds on body weight change in DIO mice**

| **Compounds** | **Dosage** | **Change rate of body weight on Day 22 (%)** |
|---|---|---|
| Control | - | -1.90±2.67 |
| Model | - | -0.85±1.75 |
| Example 38 | 10nmol/kg | -29.87±1.30 |
| Example 52 | 10nmol/kg | -30.26±2.80 |
| LY3437943 | 10nmol/kg | -26.32±1.91 |

**Conclusion:** It can be seen from the results in the table that, the compounds of Example 38 and Example 52 also have a significant weight reduction effect, which is comparable or superior to that of LY3437943.

Additionally, further pharmacodynamic studies of the compounds of the present disclosure in the IPGTT test showed that the compounds of the present disclosure all exhibited excellent hypoglycemic ability as compared to the vehicle group, which is comparable or superior to the hypoglycemic effect of LY3437943.

In summary, a series of polypeptide compounds with a modified structure have been newly designed and synthesized according to the present disclosure. Such polypeptides have strong *in vitro* agonistic activity on GLP-1R/GIPR/GCGR, excellent *in vitro* and *in vivo* pharmacokinetic properties, good stability, and significant weight reduction, hypoglycemic, and hypolipidemic effects. The polypeptide compounds of the present disclosure have a prospect for drug development as long-acting GLP-1/GIP/GCG multiple agonists.

## Claims

1. A polypeptide having a modified structure or a pharmaceutically acceptable salt thereof, wherein a sequence of the polypeptide is as shown in Formula Z-3:
X₅X₀X₆GT FTSDY SIX₁X₇X₈ KX₉X₁₀X₁₁X₀ X₄FX₁₂X₁₃X₁₄ LLX₁₅GG PSSGA PPPS₀ Formula Z-3
wherein:
X₅ is selected from the group consisting of tyrosine (Tyr, Y) and histidine (His, H);
X₀ is independently selected from the group consisting of 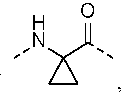 Aib, and alanine (Ala, A);
X₆ is selected from the group consisting of glutamine (Gln, Q) and histidine (His, H);
X₁ is independently selected from the group consisting of α-methyl-substituted leucine (α-MeLeu, α-MeL) and tyrosine (Tyr, Y);
X₇ is selected from the group consisting of leucine (Leu, L) and lysine (Lys, K);
X₈ is selected from the group consisting of aspartic acid (Asp, D) and glutamic acid (Glu, E);
X₉ is selected from the group consisting of lysine (Lys, K), glutamine (Gln, Q) and glutamic acid (Glu, E);
X₁₀ is selected from the group consisting of alanine (Ala, A) and tyrosine (Tyr, Y);
X₁₁ is selected from the group consisting of lysine (Lys, K), glutamine (Gln, Q) and alanine (Ala, A);
X₄ is selected from the group consisting of α-methyl-substituted lysine (α-MeLys, α-MeK), D-lysine (d-K), L-ornithine (L-Orn), alanine (Ala, A), lysine (Lys, K) and glutamic acid (Glu, E);
X₁₂ is selected from the group consisting of valine (Val, V) and isoleucine (Ile, I);
X₁₃ is selected from the group consisting of lysine (Lys, K), glutamine (Gln, Q) and glutamic acid (Glu, E);
X₁₄ is selected from the group consisting of lysine (Lys, K), tryptophan (Trp, W), tyrosine (Tyr, Y), glutamic acid (Glu, E) and phenylalanine (Phe, F);
X₁₅ is selected from the group consisting of lysine (Lys, K) and glutamic acid (Glu, E);
S₀ is selected from the group consisting of 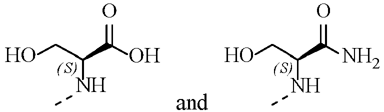 (i.e., a C-terminal amino acid is optionally amidated to a C-terminal primary amide);
and the polypeptide compound has the following modifications:
1) side chains of amino acids at positions i and i+j in the sequence are connected by a modification to form a modified structure similar to a "staple" structure (stapled body), wherein i is independently selected from the group consisting of 14, 17, 19, 21, 24 and 25, and j is independently selected from the group consisting of 3, 4, 5 and 7,
2) the modification is formed by the condensation connection of amino or carboxyl groups on the side chains of two amino acid unit structures to 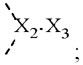
X₂ is selected from the group consisting of 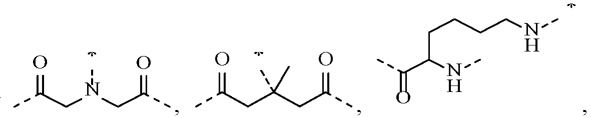 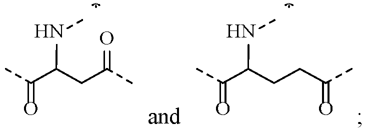 wherein, "*" indicates a position connected to X₃, and two ends are positions connected to the corresponding amino or carboxyl groups in the modified amino acids;
X₃ is selected from the group consisting of 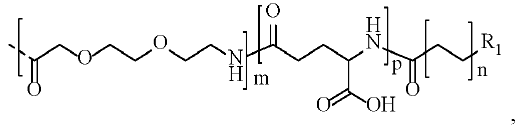 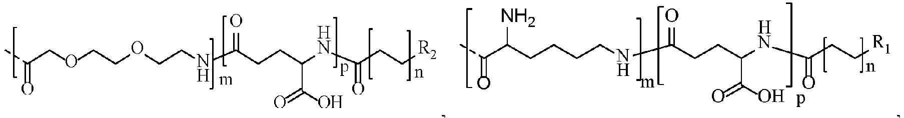 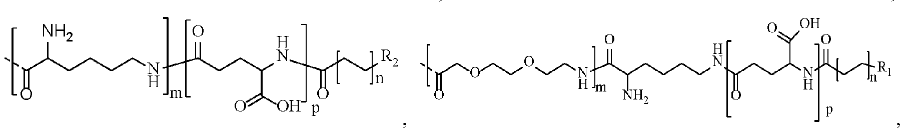 and 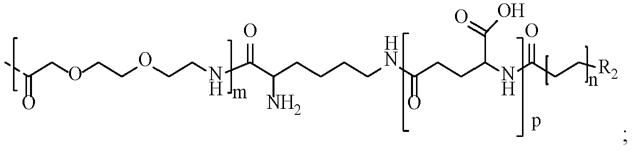
R₁ and R₂ are independently selected from the group consisting of 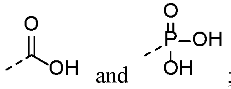 m is 1, 2 or 3; p is 1 or 2; and n is 8, 9 or 10.

2. The polypeptide or a pharmaceutically acceptable salt thereof according to claim 1, wherein the S₀ in the polypeptide sequence is

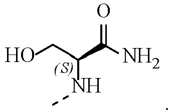

3. The polypeptide or a pharmaceutically acceptable salt thereof according to claim 1, wherein the amino acid X₀ at position 2 in the polypeptide sequence is Aib.

4. The polypeptide or a pharmaceutically acceptable salt thereof according to claim 1, wherein the amino acid X₀ at position 20 in the polypeptide sequence is Aib or A.

5. The polypeptide or a pharmaceutically acceptable salt thereof according to any one of claims 1-2, wherein the polypeptide sequence is selected from the group consisting of
YAibQGT FTSDY SI*α-MeL*LD KKAQAib KFIEY LLEGG PSSGA PPPS-NH₂ (SEQ ID NO: 1),
YAibQGT FTSDY SI*α-MeL*LD KKAQAib AFIKW LLKGG PSSGA PPPS-NH₂ (SEQ ID NO: 2),
YAibQGT FTSDY SI*α-MeL*LD KKAQAib AFIEK LLEGG PSSGA PPPS-NH₂ (SEQ ID NO: 3),
HAibHGT FTSDY SIYLE KKYAAib EFVKW LLKGG PSSGA PPPS-NH₂ (SEQ ID NO: 4),
HAibHGT FTSDY SIYLE KKYAAib KFVQW LLEGG PSSGA PPPS-NH₂ (SEQ ID NO: 5),
YAibQGT FTSDY SI*α-MeL*LD KKAQAib KFIEF LLEGG PSSGA PPPS-NH₂ (SEQ ID NO: 15),
YAibQGT FTSDY SI*α-MeL*LD KKAQAib EFIEY LLEGG PSSGA PPPS-NH₂ (SEQ ID NO: 16),
YAibQGT FTSDY SI*α-MeL*LD KKAQAib AFIKY LLEGG PSSGA PPPS-NH₂ (SEQ ID NO: 17),
YAibQGT FTSDY SI*α-MeL*LD KKAQAib KFIEY LLEGG PSSGA PPPS-NH₂ (SEQ ID NO: 18),
YAibQGT FTSDY SI*α-MeL*LD KKAKAib AFIKY LLEGG PSSGA PPPS-NH₂ (SEQ ID NO: 19),
YAibQGT FTSDY SI*α-MeL*LD KKAQAib KFIEY LLKGG PSSGA PPPS-NH₂ (SEQ ID NO: 20),
YAibQGT FTSDY SI*α-MeL*LD KKAQA *α-MeK*FIEY LLEGG PSSGA PPPS-NH₂ (SEQ ID NO: 21),
YAibQGT FTSDY SI*α-MeL*LD KKAQAib d-KFIEY LLEGG PSSGA PPPS-NH₂ (SEQ ID NO: 22),
YAibQGT FTSDY SI*α-MeL*LD KKAQAib *L-OrnFIEY* LLEGG PSSGA PPPS-NH₂ (SEQ ID NO: 23), and
YAibQGT FTSDY SI*α-MeL*KD KKAQAib AFIEY LLEGG PSSGA PPPS-NH₂ (SEQ ID NO: 24).

6. The polypeptide or a pharmaceutically acceptable salt thereof according to claim 5, wherein i is 14, and j is 3.

7. The polypeptide or a pharmaceutically acceptable salt thereof according to claim 5, wherein i is 17, and j is 4.

8. The polypeptide or a pharmaceutically acceptable salt thereof according to claim 5, wherein i is 17, and j is 7.

9. The polypeptide or a pharmaceutically acceptable salt thereof according to claim 5, wherein i is 19, and j is 5.

10. The polypeptide or a pharmaceutically acceptable salt thereof according to claim 5, wherein i is 21, and j is 7.

11. The polypeptide or a pharmaceutically acceptable salt thereof according to claim 5, wherein i is 24, and j is 4.

12. The polypeptide or a pharmaceutically acceptable salt thereof according to claim 5, wherein i is 25, and j is 4.

13. The polypeptide or a pharmaceutically acceptable salt thereof according to claim 1, wherein m is 1 or 2.

14. The polypeptide or a pharmaceutically acceptable salt thereof according to claim 1, wherein p is 1.

15. The polypeptide or a pharmaceutically acceptable salt thereof according to claim 1, wherein n is 9.

16. The polypeptide or a pharmaceutically acceptable salt thereof according to claim 1, wherein X₃ is selected from the group consisting of 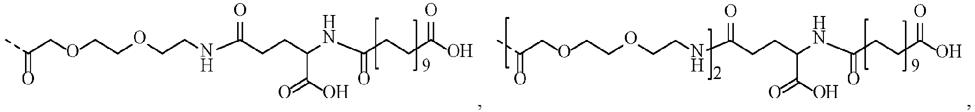 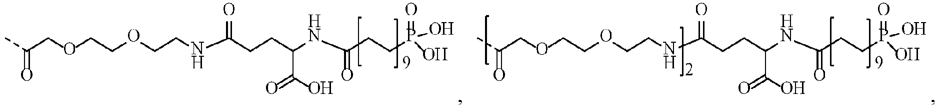 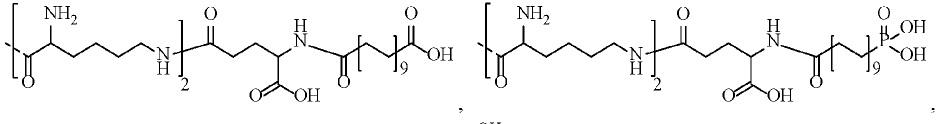 and 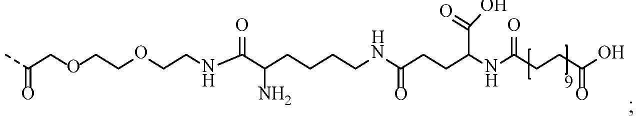 further preferably, X₃ is selected from the group consisting of 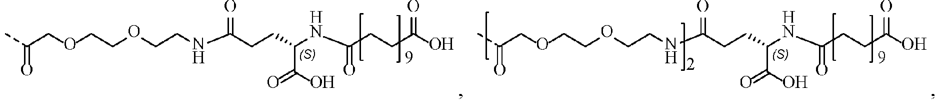 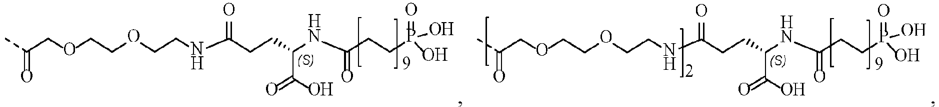 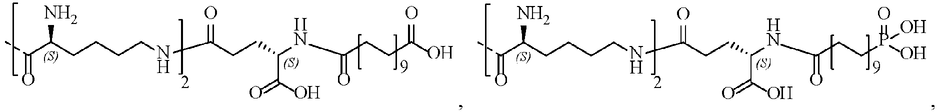 and

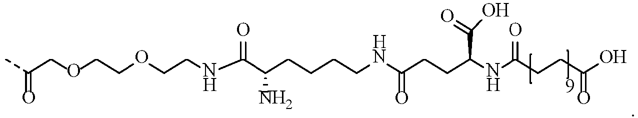

17. The polypeptide or a pharmaceutically acceptable salt thereof according to claim 1, wherein the structural unit 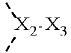 is selected from the group consisting of 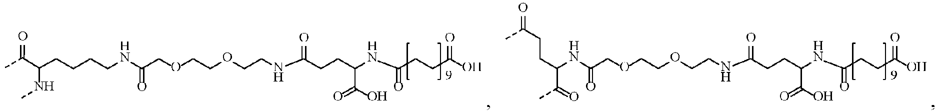 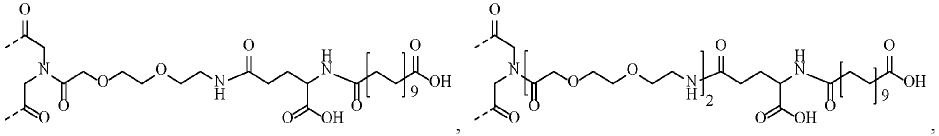 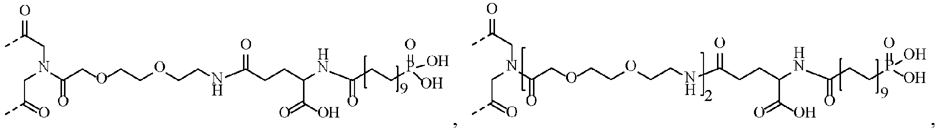 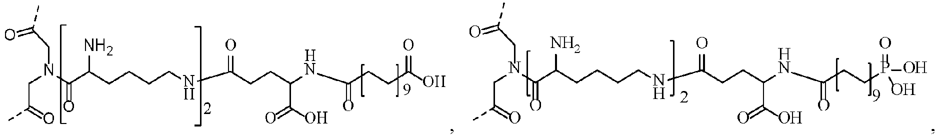 and 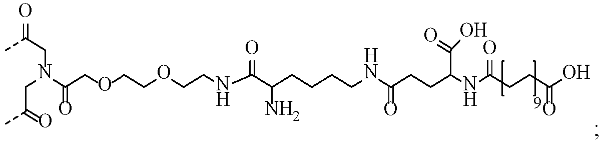 further preferably, the structural unit 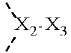 is selected from the group consisting of 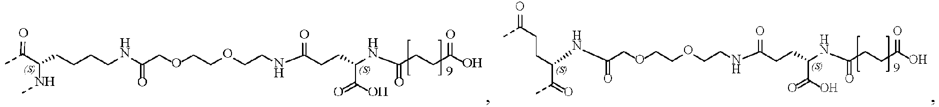 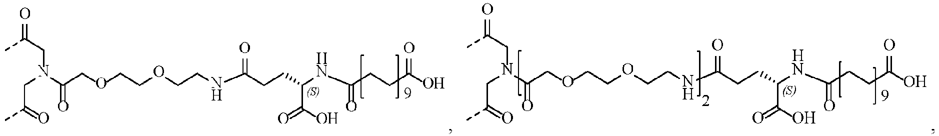 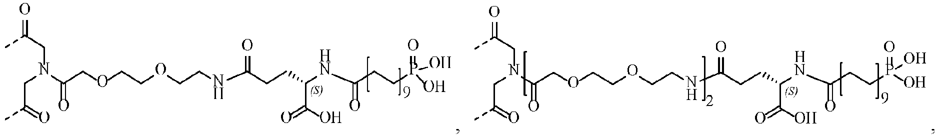 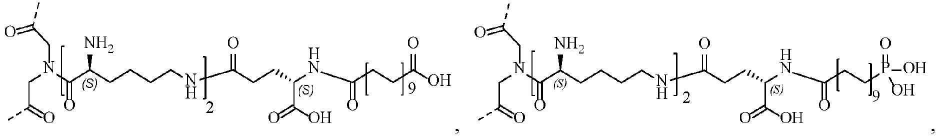 and

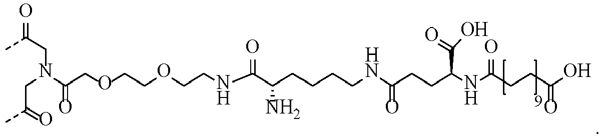

18. The polypeptide or a pharmaceutically acceptable salt thereof according to claim 1, wherein a structure of the stapled body is selected from the group consisting of 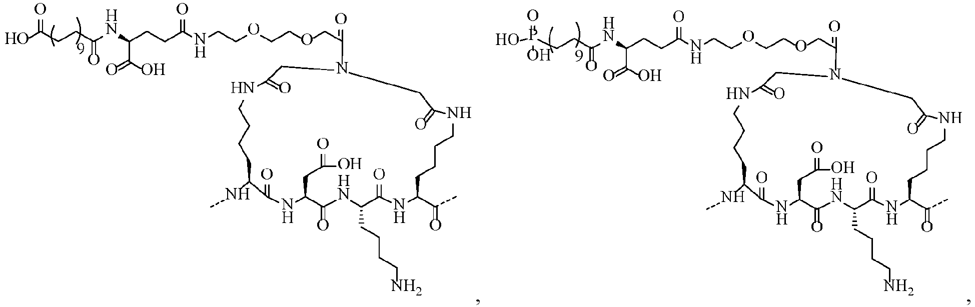 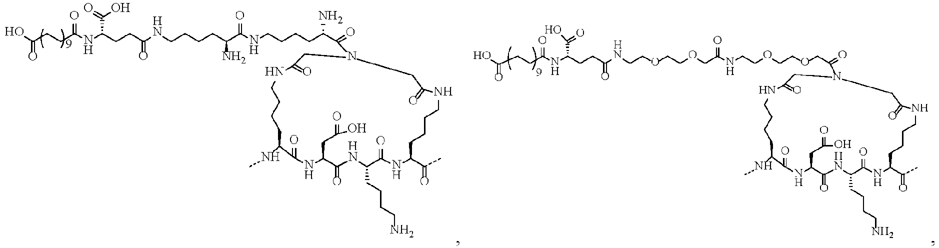 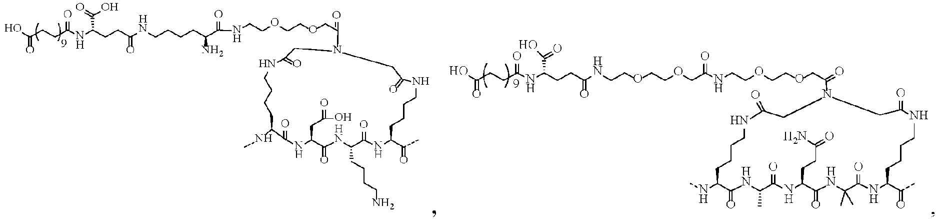 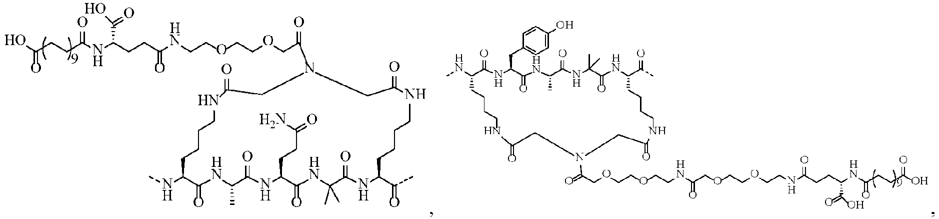 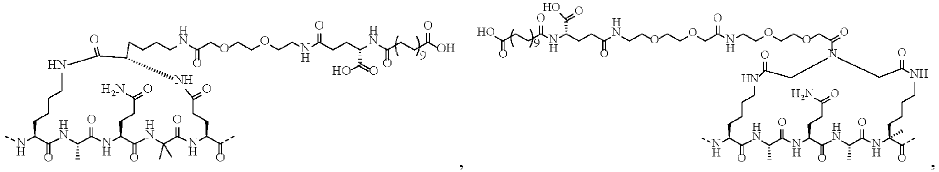 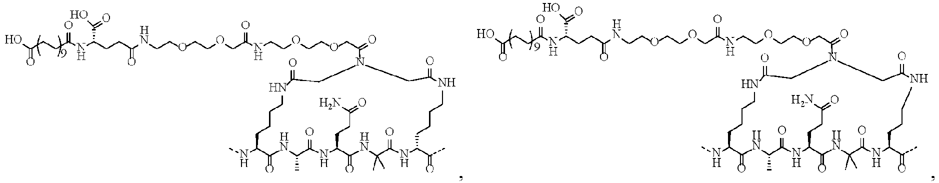 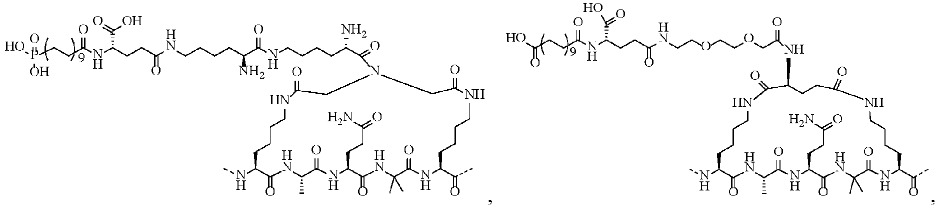 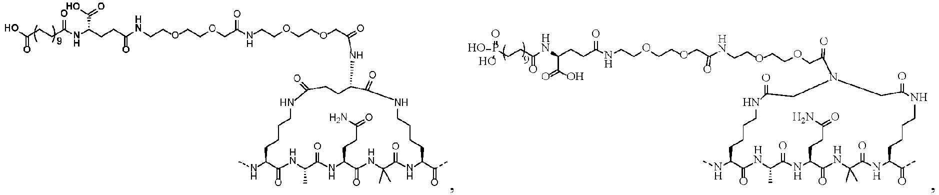 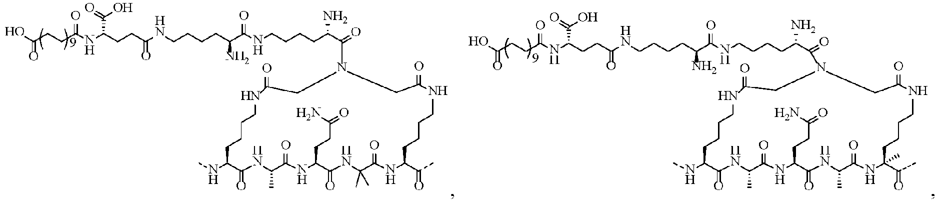 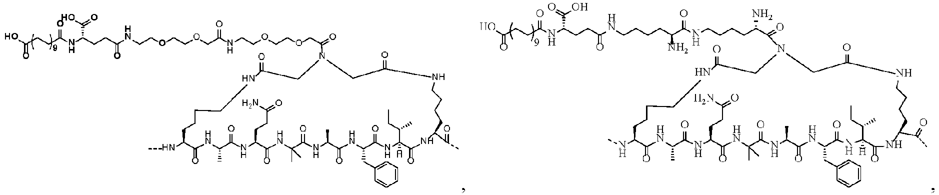 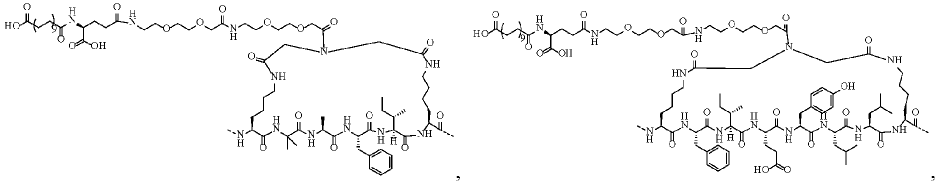 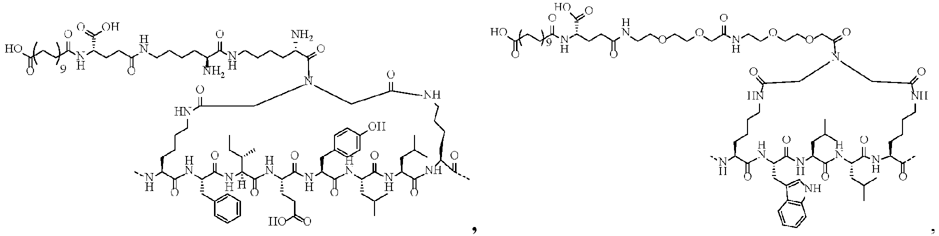 and

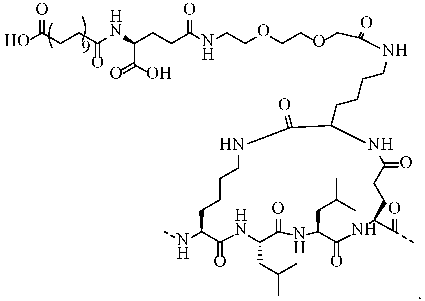

19. A polypeptide compound of a formula selected from the group consisting of 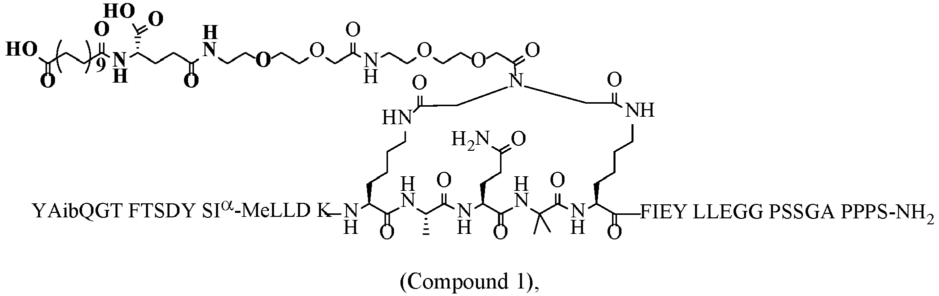 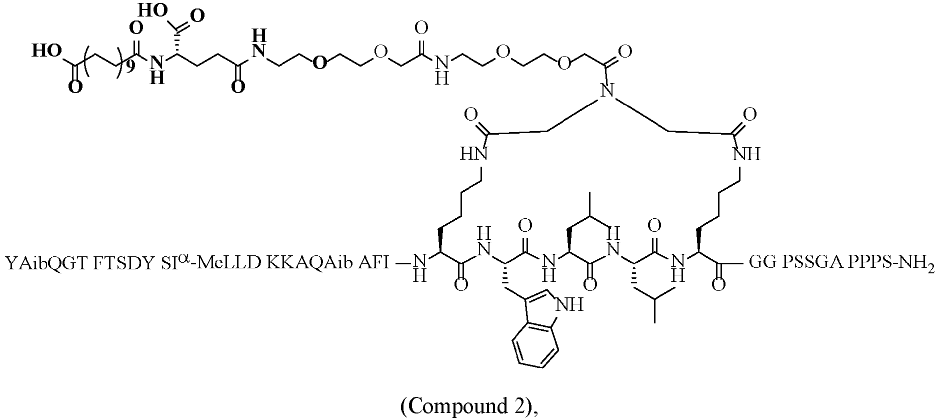 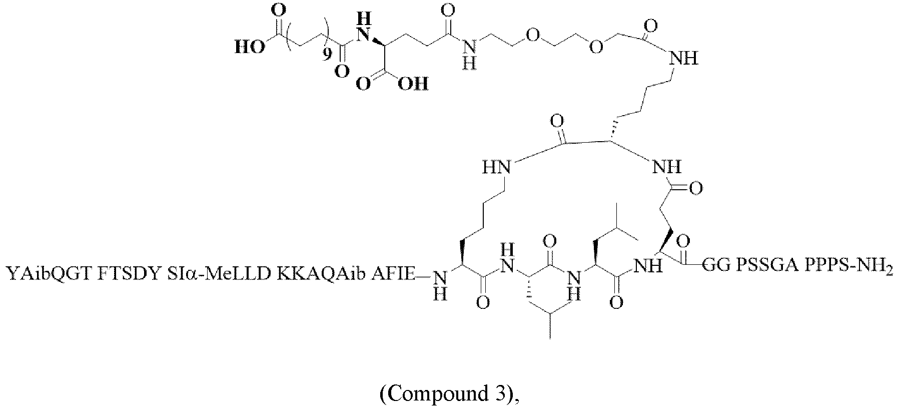 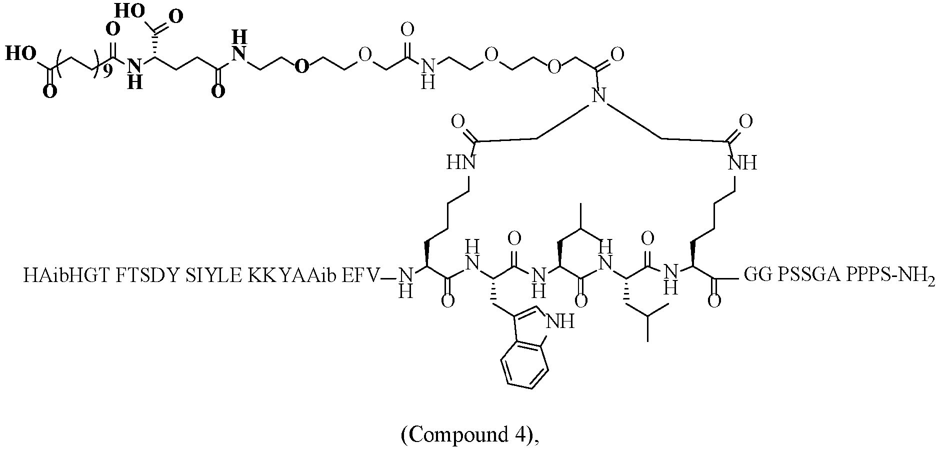 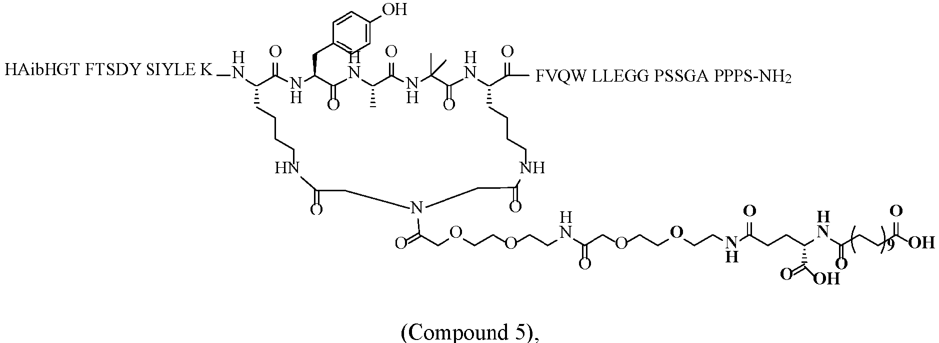 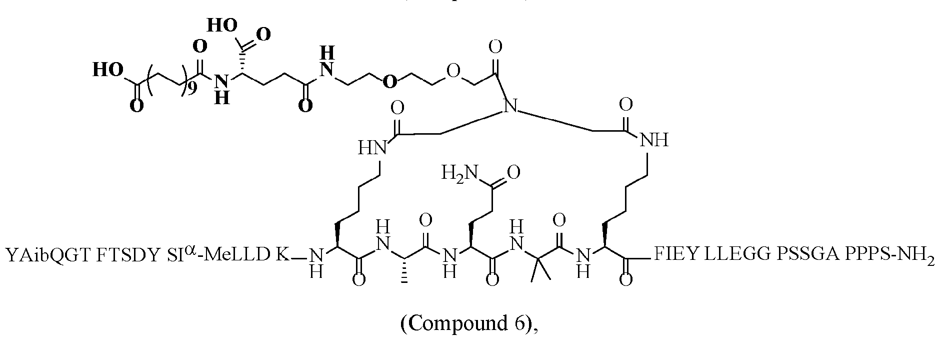 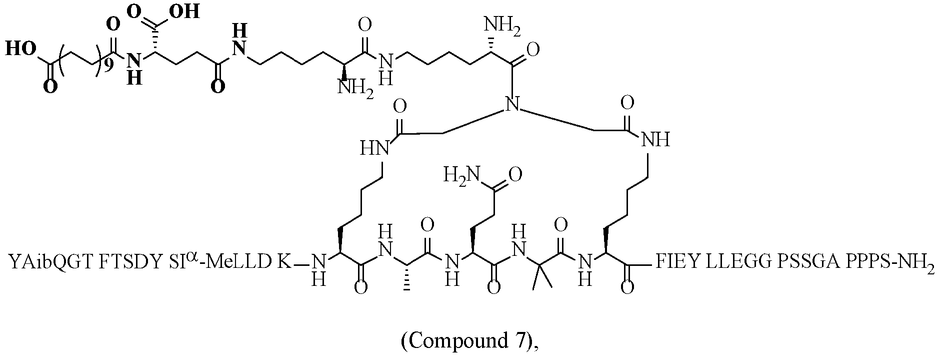 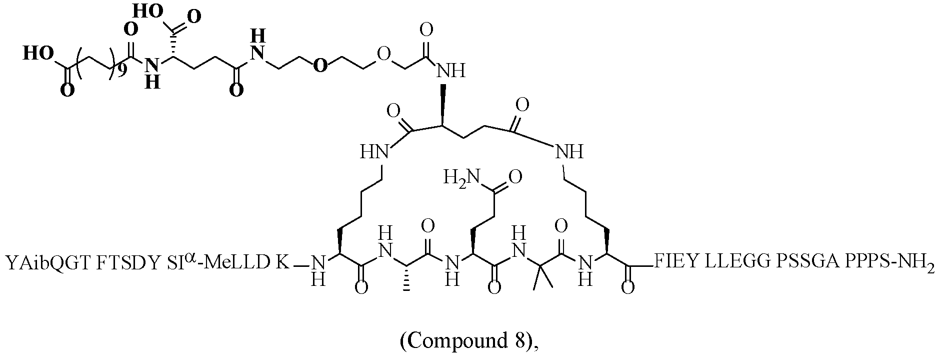 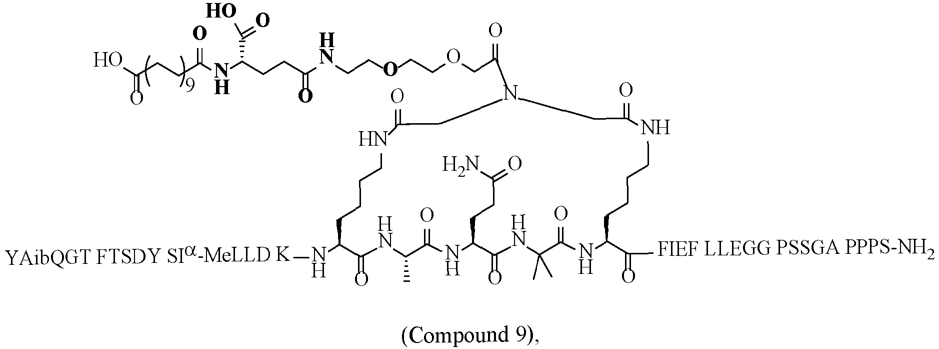 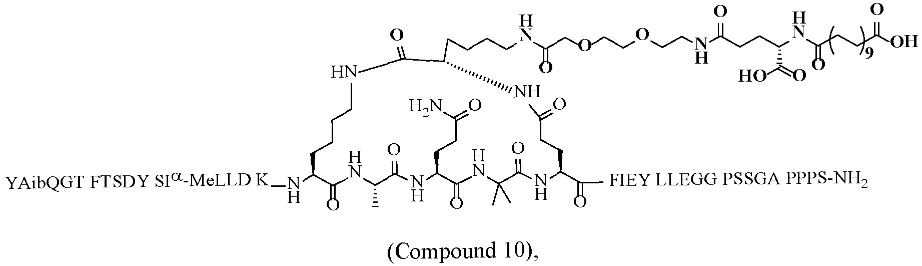 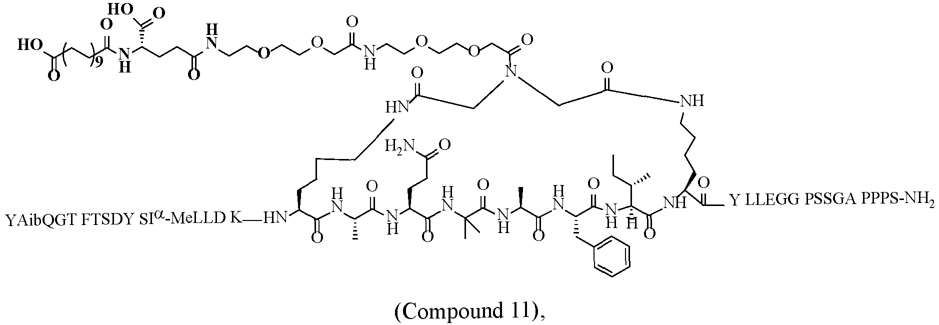 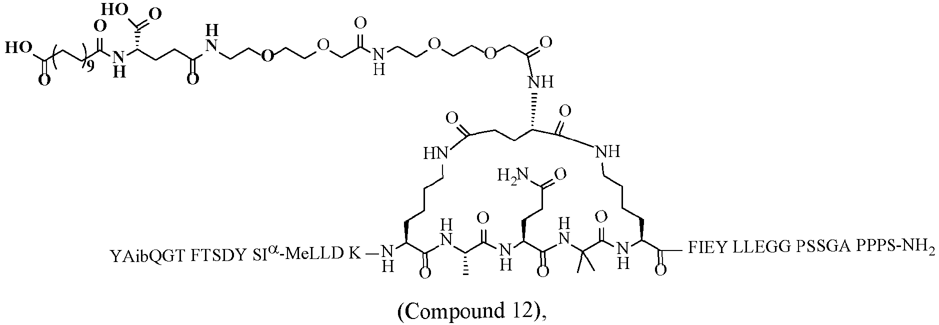 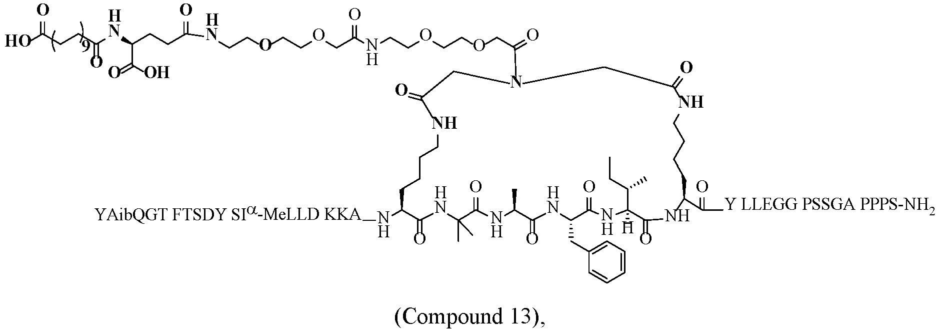 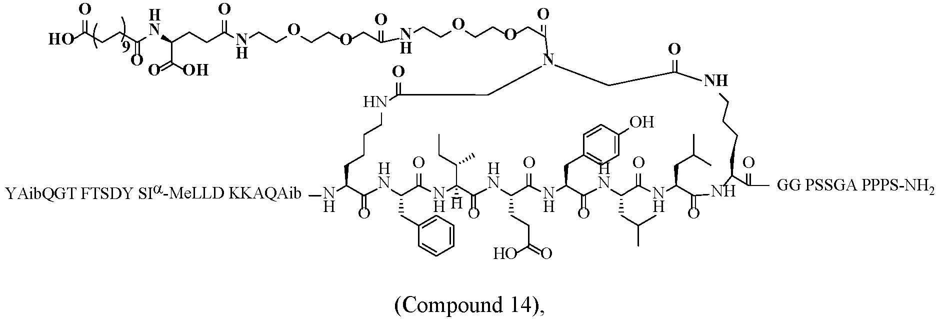 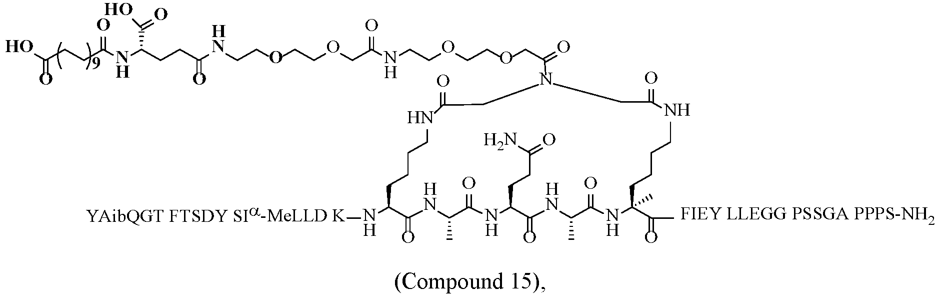 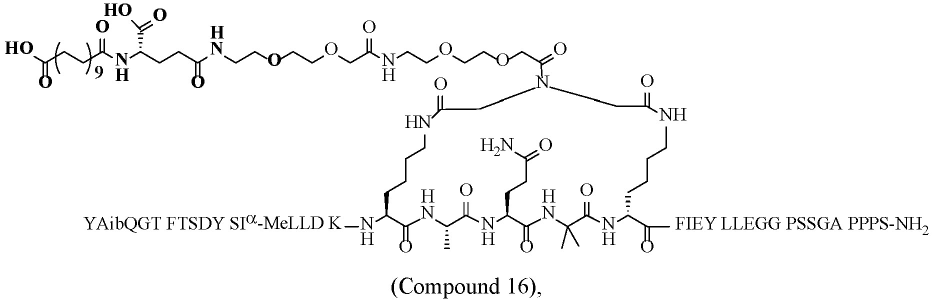 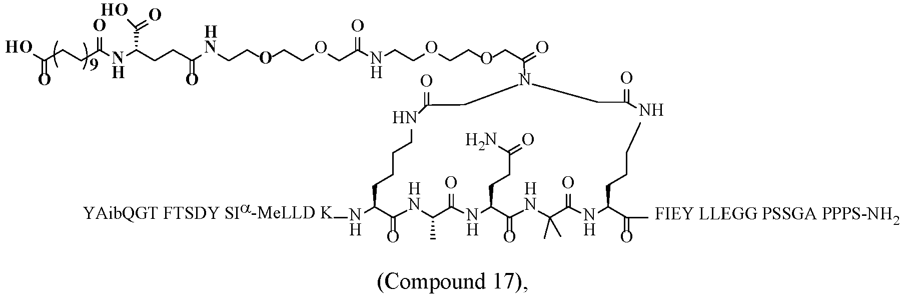 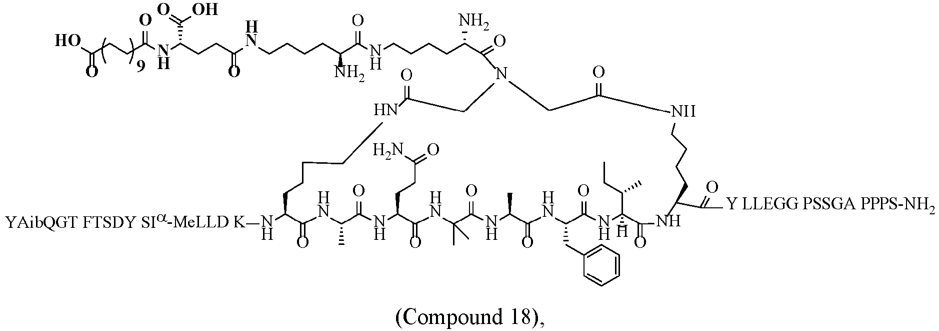 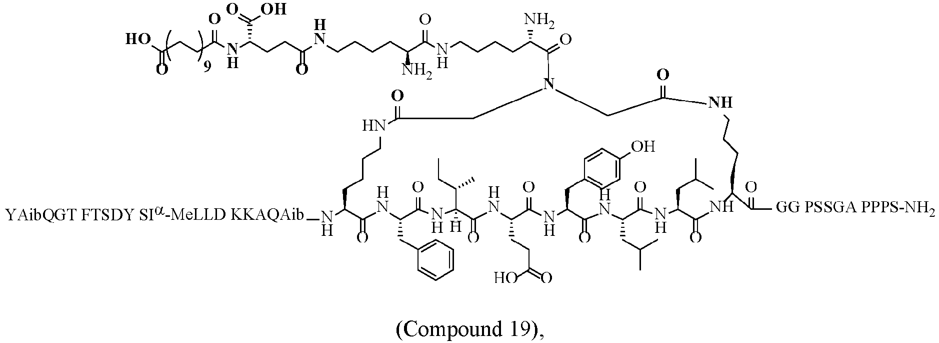 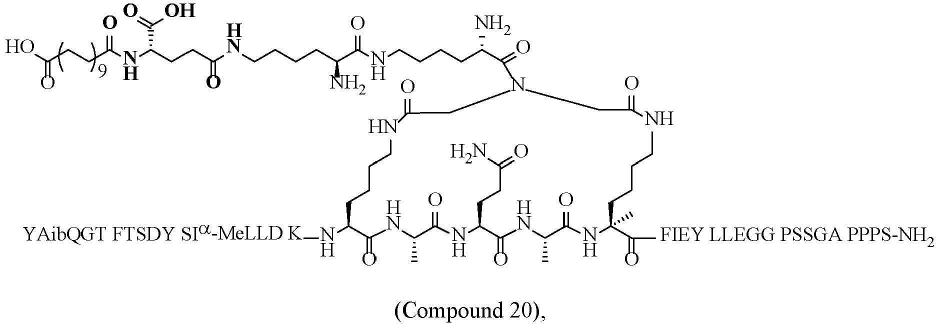 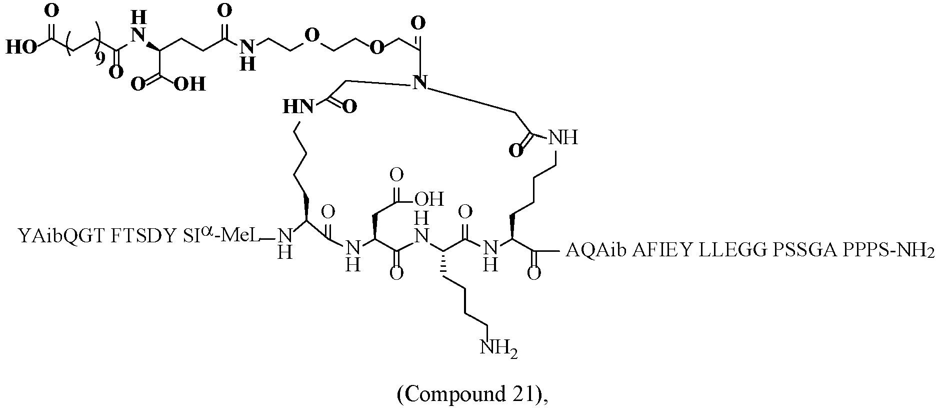 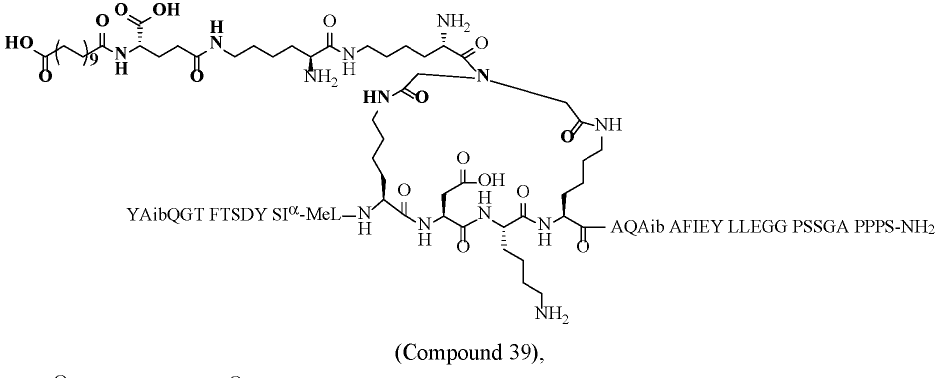 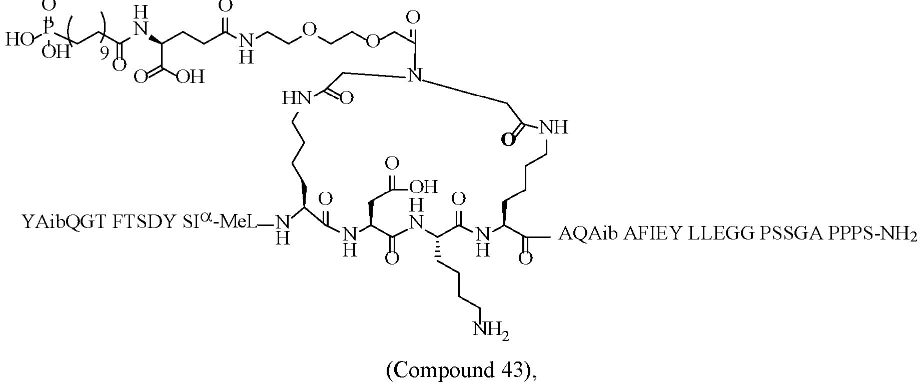 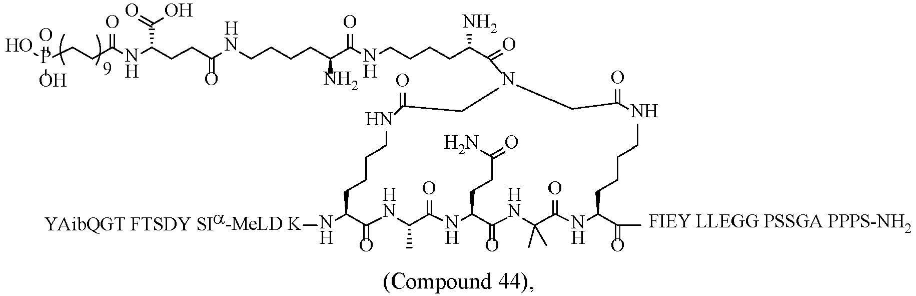 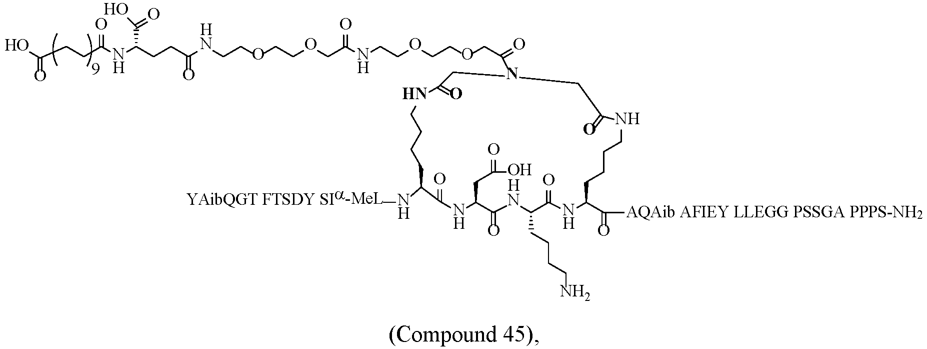 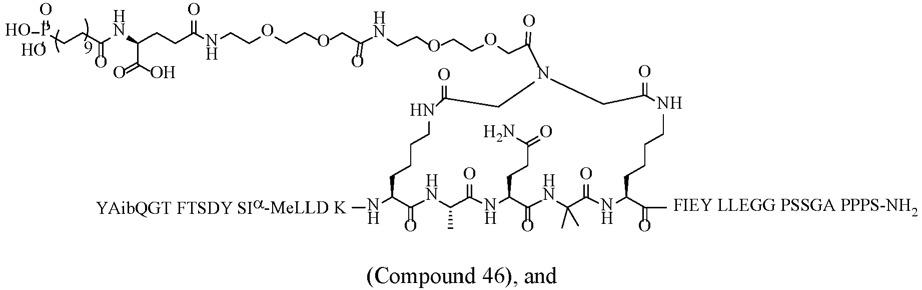 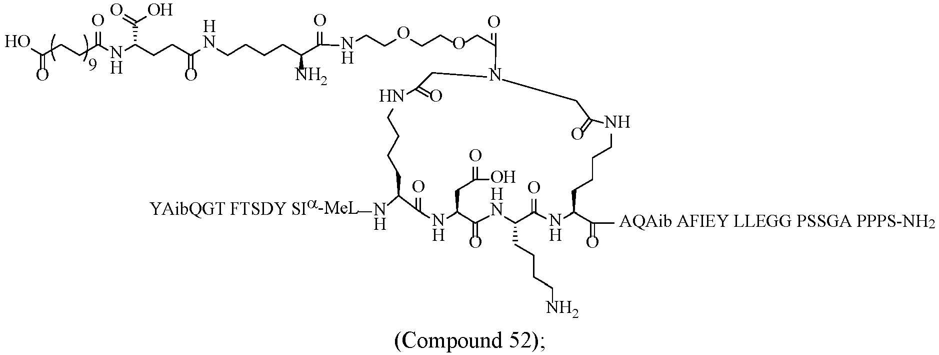 or a pharmaceutically acceptable salt thereof.

20. The polypeptide or a pharmaceutically acceptable salt thereof according to any one of claims 1-20, wherein the salt is a salt formed by the polypeptide compound with any one of: inorganic acids, comprising hydrochloric acid, hydrobromic acid, nitric acid, carbonic acid, bicarbonate, phosphoric acid, monohydrogen phosphate, dihydrogen phosphate, sulfuric acid, hydrosulfate, hydroiodic acid, and phosphorous acid; and organic acids, comprising acetic acid, trifluoroacetic acid, propionic acid, isobutyric acid, maleic acid, malonic acid, benzoic acid, succinic acid, octanedioic acid, fumaric acid, lactic acid, mandelic acid, phthalic acid, benzenesulfonic acid, p-toluenesulfonic acid, citric acid, tartaric acid, methanesulfonic acid, pamoic acid, arginine, and glucuronic acid.

21. A pharmaceutical composition, wherein the pharmaceutical composition comprises the polypeptide or a pharmaceutically acceptable salt thereof according to any one of claims 1-20.

22. Use of the polypeptide or a pharmaceutically acceptable salt thereof according to any one of claims 1-20 and the pharmaceutical composition according to claim 21 in the preparation of a drug for treating metabolic diseases and related diseases thereof.

23. The use according to claim 22, wherein the metabolic diseases comprise diabetes, obesity and non-alcoholic steatohepatitis and related diseases thereof.
